# EUROPEAN PATENT SPECIFICATION

(11) **EP 0 854 722 B1**
(45) Date of publication and mention of the grant of the patent: **02.11.2006**
(21) Application number: 96937660.7
(22) Date of filing: 04.10.1996
(51) Int. Cl.: A61K 31/505, A61K 31/40, A61P 31/12, C12N 15/81, C12N 15/79, A61K 48/00, C12N 9/00, C12N 15/11, C12Q 1/48, C07K 14/39, A61K 38/16

(54) **PROTEINS INVOLVED IN TARGETING OF PEPTIDYL TRANSFER CENTER, AND CORRESPONDING THERAPEUTIC AGENTS AND METHODS**
PROTEINE, DIE MIT mRNA-EXPRESSION UND -STABILITÄT EINBEGRIFFEN SIND UND TESTS FÜR AGENTIEN, DIE IHRE FUNKTION BEEINFLUSSEN.
PROTEINES IMPLIQUEES DANS LE CIBLAGE DU CENTRE DE TRANSFERT DE PEPTIDYLE, ET PROCEDES ET AGENTS THERAPEUTIQUES CORRESPONDANTS

(30) Priority: 06.10.1995 US 605041
(43) Date of publication of application: 29.07.1998
(73) Proprietor: UNIVERSITY OF MEDICINE AND DENTISTRY OF NEW JERSEY, Piscataway, NJ 08855-6810 (US)
(72) Inventor: PELTZ, Stuart, W., Piscataway, NJ 08854 (US); DINMAN, Jonathan, D., North Brunswick, NJ 08902 (US); CUI, Ying, Plainsboro, NJ 08536 (US)
(74) Representative: Harrison Goddard Foote
(86) International application number: PCT/US1996/016011
(87) International publication number: WO 1997/012617

(56) References cited:
- GENETICS, vol. 141, no. 1, September 1995, pages 95-105, XP000616457 DINMAN J D ET AL: "5 S RRNA IS INVOLVED IN FIDELITY OF TRANSLATIONAL READING FRAME"
- GENETICS, vol. 136, no. 1, January 1994, pages 75-86, XP000616352 DINMAN J D ET AL: "TRANSLATIONAL MAINTENANCE OF FRAME: MUTANTS OF SACCHAROMYCES CEREVISIAE WITH ALTERED - 1 RIBOSOMAL FRAMESHIFTING EFFICIENCES" cited in the application
- PROGRESS IN NUCLEIC ACID RESEARCH AND MOLECULAR BIOLOGY, vol. 47, 1994, pages 271-298, XP000617057 PELTZ S W ET AL: "NONSENSE-MEDIATED MRNA DECAY IN YEAST" cited in the application
- INTERSCIENCE CONFERENCE ON ANTIMICROBIAL AGENTS & CHEMOTHERAPY, vol. 1/2, 1970, pages 473-481, XP000617152 MONRO R E ET AL: "ANTIBIOTICS ACTING ON THE PEPTIDYL TRANSFERASE CENTER OF RIBOSOMES"
- J. MED. CHEM., vol. 32, no. 8, August 1989, pages 2002-2015, XP000644499 VAN DEN BROEK ET AL.: "Lipophilic analogues of sparsomycin as strong inhibitors of protein synthesis and tumor growth : a structure-activity relationship"
- BIOCHIM. BIOPHYS. ACTA, vol. 312, 1973, pages 368-376, XP000617035 CARRASCO ET AL.: "The trichodermin group of antibiotics, inhibitors of peptide bond formation by eukaryotic ribosomes"
- EUR. J. BIOCHEM., vol. 44, no. 2, 15 May 1974, pages 437-444, XP000617042 BARBACID ET AL.: "Binding of [acetyl-14C] trichodermin to the peptidyl transferase centre of eukaryotic ribosomes"
- NATURE, vol. 277, 11 January 1979, pages 148-150, XP002026926 PALMER ET AL.: "Phenotypic suppression of nonsense mutants in yeast by aminoglycoside antibiotics"
- PROC. NATL ACAD. SCI., vol. 92, July 1995, pages 6587-6591, XP002026927 LEE ET AL.: "A genetic screen identifies cellular factors involved in retroviral -1 frameshifting"
- MOL. GEN. GENET., vol. 217, 1989, pages 362-369, XP002026928 WEISS-BRUMMER ET AL.: "The paromomycin resistance mutation (parR-454) in the 15S rRNA gene of the yeast Saccharomyces cerevisiae is involved in the ribosomal frameshifting"
- MOL. GEN. GENET., vol. 248, no. 2, 28 July 1995, pages 207-216, XP002026929 WEISS-BRUMMER ET AL.: "Mutation of a highly conserved base in the yeast mitochondrial 21S rRNA restricts ribosomal frameshifting"
- JOURNAL OF MOLECULAR BIOLOGY, vol. 254, no. 5, 15 December 1995, pages 838-847, XP000617136 O'CONNOR M ET AL: "THE INVOLVEMENT OF TWO DISTINCT REGIONS OF 23 S RIBOSOMAL RNA IN TRNA SELECTION"
- MICROBIOL. REV., vol. 60, no. 1, 6 March 1996, pages 250-265, XP000617036 WICKNER: "Double-stranded RNA viruses of Saccharomyces cerevisiae"
- GENES AND DEVELOPMENT, vol. 5, 1991, pages 2303-2314, XP000616363 LEEDS P ET AL: "THE PRODUCT OF THE YEAST UPF1 GENE IS REQUIRED FOR RAPID TURNOVER OF MRNAS CONTAINING A PREMATURE TRANSLATIONAL TERMINATION CODON" cited in the application
- PROC. NATL ACAD. SCI., vol. 90, August 1993, pages 7034-7038, XP002026930 HE ET AL.: "Stabilization and ribosome association of unspliced pre-mRNAs in a yeast upf1- mutant"
- JOURNAL OF VIROLOGY, vol. 66, no. 6, June 1992, pages 3669-3676, XP000616354 DINMAN J D ET AL: "RIBOSOMAL FRAMESHIFTING EFFICIENCY AND GAG/GAG-POL RATIO ARE CRITICAL FOR YEAST M1 DOUBLE-STRANDED RNA VIRUS PROPAGATION" cited in the application
- EMBO JOURNAL, vol. 1, no. 5, 1982, pages 609-613, XP000617142 SCHULZE H ET AL: "MINIMAL SET OF RIBOSOMAL COMPONENTS FOR RECONSTITUTION OF THE PEPTIDYLTRANSFERASE ACTIVITY" cited in the application
- MOL. CELL. BIOL., vol. 5, no. 1, January 1985, pages 99-108, XP000616553 FRIED ET AL.: "Characterization of yeast strains with conditionally expressed variants of ribosomal protein genes tcm1 and cyh2"
- BIOCHEMISTRY, vol. 30, 1991, pages 5421-5428, XP002026931 MURALIKRISHNA ET AL.: "A photolabile oligodeoxyribonucleotide probe of the peptidyltransferase center : identification of neighboring ribosomal components"
- NUCLEIC ACIDS RES., vol. 18, no. 19, 1990, pages 5823-5828, XP002026932 MAICAS ET AL.: "Translation of the Saccharomyces cerevisiae tcm1 gene in the absence of a 5'-untranslated leader"
- J. BACTERIOL., vol. 155, 1983, pages 8-13, XP000617038 SCHULTZ ET AL.: "Nucleotide sequence o fthe tcm1 gene (ribosomal protein L3) of Saccharomyces cerevisiae" cited in the application

## Description

### FIELD OF THE TNVENTTON

The present invention relates to the identification or proteins that are involved in mRNA frameshifting and the nonsense mRNA decay pathway, as well as recombinant genes encoding RNAs with increased *in vivo* stability. Identification of these proteins and stabilized RNAs provides *in vitro* assay systems for identifying agents that affect the functional activity of mRNAs by altering frameshift frequency. Such agents will be useful antiviral or antimicrobial drugs. The present assay systems further provide for identification of agents that affect RNA stability. Such agents will be useful for treating diseases associated with nonsense mutations. Also provided are stabilized antisense RNAs, and stabilized mRNAs encoding peptides for use in two-hybrid ligand assay systems.

### BACKGROUND OF THE INVENTION

### RNA Turnover and Ribosomal Frameshifting

Many studies have indicated that the processes of mRNA turnover and translation are directly linked [Peltz *et al.. Prog. Nucl. Acid Res. & Mol. Biol.* **47**:211-298 (1994)]. One clear example of the relationship between translation mRNA turnover is the observation that nonsense mutations in a gene can decrease the stability of nonsense-containing transcripts [Peltz *et al. Prog. Nucl. Acid Res. & Mol. Biol.* **47**:271-298(1994)]. The yeast *Saccharomyces cerevisiae* has been used to identity and characterize the transacting factors involved in the nonsense-mediated mRNA decay pathway. Mutations in the *UPF1, UPF2 and UPF3* genes elevate the concentration of nonsense-containing mRNAs in cells by increasing their half-lives without affecting the decay of most wildtype transcripts [Leeds *et al., Genes & Dev.* **5**:2303-2314 (1991). Leeds *et al., Mol. Cell. Biol.* **12**:2165-2177 (1992): Peltz *et al., Genes & Dev*. **7**:1737-1754 (1993). Hagan *et al., Mol. Cell. Biol.*15:809-823 (1995): Cui et *al., Genes & Dev.* **9**:437-454 (1995): He *et al., Genes & Dev.* **9**:437-454 (1995)]. The Upf1p not only functions in the nonsense-mediated mRNA decay pathway, but also has separable functions involved in modulating nonsense and frameshift suppression, suggesting that these processes are tightly linked and may share similar components.

In addition to the translational apparatus being able to stabilize nonsense transcripts and suppress nonsense and frameshift mutations, mechanisms have evolved that regulate gene expression by inducing elongating ribosomes to shift reading frame in response to specific ribosomal frameshifting signals [Chandler *et al., mol. microbiol.* **7**:497-503 (1993); Farabaugh *et al., J. Biol. Chem.* **270**:10361-10364 (1995); Hayashi *et al., Biochem. J.* **306**:1-10 (1995)]. Frameshifting events produce fusion proteins, in which the N- and C-terminal domains are encoded by two distinct, overlapping open reading frames. Ribosomal frameshifting is different from frameshift suppression in that these events are directed by specific mRNA sequences and structures, rather than being a consequence of mutations in the host gene products.

### Viral Frameshifting

Most examples of ribosomal frameshifting have been identified in viruses, all of which use their RNA (+) strands as: 1) mRNAs encoding multiple protein products, 2) the species of RNA that is packaged into nascent viral particles and 3) the template for replication of the viral genetic material. Production of multiple protein products could be achieved by mRNA splicing or editing. These mechanisms might, however, pose the consequence of producing altered RNA (+) strands, resulting in the production of mutant viral genomes, unless splicing or editing removed an RNA site required for packaging or replication of the genomic RNA. Perhaps for this reason, (+) ssRNA and dsRNA viruses are not known to use splicing or mRNA editing, and retroviruses remove their packaging site (Ψ) when they splice their RNAs [Mann *et al., Cell* **33**:153-159, (1983); Wantanabe and Temin, *Proc. Natl. Acad. Sci. USA* **79**:5986-5990 (1982)]. In using ribosomal frameshifting and/or readthrough of termination codons to make fusion proteins, RNA (+) strand templates are not altered, and so production and packaging of mutant viral genomes is prevented [Icho and Wickner, *J. Biol Chem.* **264**:6716-6723, (1989)].

For example, the killer virus system in yeast consists of the L-A and M₁ double-stranded RNA viruses and utilizes programmed -1 ribosomal frameshifting for appropriate gene expression [Wickner, R.B. *J. Biol. Chem.,* **268**:3797-3800 (1993)]. The dsRNA I-A virus has two open reading frames. The 5' *gag* encodes the Gag protein and the 3' *pol* gene encodes a multifunctional protein domain required for viral RNA packaging [Wickner, R.B. *J. Biol. Chem.,* **268:**3797-3800 (1993)]. A -1 ribosomal frameshift event is responsible for the production of the Gag-Pol fusion protein. M₁, a satellite dsRNA virus of L-A which encodes a secreted killer toxin [Wickner, R.B. *J. Biol. Chem.,* **268**:3797-3800 (1993)], is encapsulated and replicated using the gene products synthesized by the L-A virus. Previous results have demonstrated that mutations that alter the efficiency of -1 ribosomal frameshifting of the L-A virus changes the ration of Gag to Gag-Pol synthesized and causes the loss of the M₁ satellite virus [Dinman *et al., J. Virol.* **66:**3669-3676 (1992); Dinman *et al., Genetics,* **136:**75-86 (1994)].

### Frameshifting Mechanisms

Ribosomal frameshifting in the -1 direction in retroviruses, (+) ssRNA viruses and dsRNA viruses requires a special sequence, X XXY YYZ (the 0-frame is indicated by spaces) called the "slippery site" [Jacks and Varmus, *Science* **230**:1237-1242 (1985)]. The simultaneous slippage of ribosome-bound A- and P-site tRNAs by one base in the 5' direction still leaves their non-wobble bases correctly paired in the new reading frame. A second promoting element [Jacks *et al., Cell* **55:**447-458 (1988)], usually an RNA pseudoknot, is located immediately 3' to the slippery site [Brow and Geiduschek, *J. Biol. Chem.* **262:**13953-13958 (1987); Dinman *et al., Proc. Natl. Acad. Sci. USA* **88:**174-178 (1991); TenDam *et al., Biochemistry* **31:**11665-11676 (1992)]. The mRNA pseudoknot makes the ribosome pause over the slippery site, and is thought to increase the probability of 5' ribosomal movement [Somogyi *et al., Mol. Cell. Biol.* **13**:6931-6940 (1993); Tu *et al., Proc. Natl. Acad. Sci. USA* **89**:8636-8640 (1992)]. The efficiency of -1 ribosomal frameshifting can be affected by the ability of the ribosome bound tRNAs to un-pair from the 0-frame, the ability of these tRNAs to re-pair to the -1 frame, the relative position of the RNA pseudoknot from the slippery site and its thermodynamic stability [Brierly *et al., J. Mol. Biol.* **227**:463-479 (1992); Brierly *et al., J. Mol. Biol.* **227**:463-479 (1991); Brow et al., (1987) *supra;* Dinman *et al.,* (1991) *supra;* Dinman and Wickner, *J. Virology* **66**:3669-3676 (1992); Jacks *et al.,* (1988) *supra;* Morikawa and Bishop, *Virology* **186**:389-397 (1992)]. Site directed *in vitro* mutagenesis has been used to examine the slippery site and the mRNA pseudoknot. The mRNA pseudoknot structure is required for efficient -1 ribosomal frameshifting in the L-A virus of yeast [Dinman *et. al.,* (1991) *supra*; Dinman *et al.,* (1992) *supra*].

A screen for mutations that increased the programmed -1 ribosomal frameshift efficiencies in cells identified nine chromosomal mutants that were called *mof* (for ***M*aintenance *O*f** *F*rame [Dinman *et al., J. Virol.* **66**:3669-3676 (1992); Dinman *et al., Genetics,* **136:**75-86 (1994)]. The screen originally used to identify the *mof* mutants utilized a construct in which the *lacZ* gene was inserted downstream of the L-A -1 ribosomal frameshift signal and in the -1 reading frame relative to a translational start site. The assay for *mof* mutants relied upon identifying cells with higher β-galactosidase activities as a consequence of increased of -1 ribosomal frameshifting efficiency. The reporter of mRNA used in this screen has a short (approximately 100 nt) protein coding region 5' of the frameshift site, followed by approximately 3.1 kb *lacZ* mRNA that is out of frame with the 5' open reading frame. Thus, it is conceivable that the translation apparatus may see the reported transcript as an aberrant nonsense-containing mRNA. Seen in this light, the increased β-galactosidase activity observed in *mof* strains may result from mutations that stabilize nonsense-containing transcripts. Thus, the experiments presented here survey the nonsense-mediated mRNA decay phenotypes of the *mof* mutants and relate this phenotype to the ability to maintain the killer phenotype.

The citation of any reference herein should not be construed as an admission that such reference is available as "Prior Art" to the instant application.

### SUMMARY OF THE INVENTION

In its first aspect, the present invention relates to the use of a compound selected from anisomycin, sparsomycin or poromycin in the manufacture of a medicament for the treatment of viral infections caused by viruses using -1 ribosomal frame shifting. Preferably the viral infection is an HIV infection.

Exemplified in the Examples appended hereto, the *mof4-1* allele has been identified as *UPF1* of *Saccharomyces cerevisiae,* and the protein UPF1 has been found to complement the *mof4-1* mutation. Thus, UPF1 is identified as a mRNA frameshift-associated protein. The *mof4-1* mutation is associated with an increase in efficiency of -1 ribosomal frameshifting of viral mRNA.

In addition, *mof2-1* has been identified as an allele of the gene *sui1,* which encodes the protein SUI1 (Castilho-Valavicius et al., 1990, Genetics 124:483-495: Yoon and Donahue. 1992. Mol. Cell. Biol. 12:248-260). This protein has a human homologue (Fields and Adams, 1994, Biochem. Biophys. Research Commun. 198:288-291; Kasperaitis et al.. 1995. FEBS Lett. **365**:47-50). It has been found that the human homologue of SUI1 can complement the *mof2-1* mutation in *S. cerevisiae,* thus confirming a role for both the yeast and human proteins in ribosomal frameshifting of viral mRNA.

There is also described the identification of *mof5-1* as an allele of the *PRP19* gene (also known as *CDC40*) (Jones et al., 1995. Proc. Natl. Acad. Sci. USA: Vaisman et al.. Mol. Gen. Genetics. **247**:123-136; Vijayraghavan et al., 1989, Genes & Dev. **3**:1206-1216).

Identification of these genes provides for selection and testing of agents that affect the efficiency of ribosomal frameshifting. Agents that interfere with ATPase activity, helicase activity, or zinc finger motif configuration may be selected for testing. Such agents may be useful drugs for treating viral infections, since many retroviruses, notably HIV, coronaviruses, and other RNA viruses that are associated with medical and veterinary pathologies. By providing the identity of proteins that modulate frameshifting events, an initial screen for agents may include a binding assay to such proteins. The ability of a binding agent to affect frameshifting efficiency can be measured *in vitro. e.g.,* using a killer assay as described in the Example appended hereto (Dinman and Wickner. 1992, J. Virol. **66**:3669-3676). This assay may be employed for testing the effectiveness of agents on the activity of frameshift associated proteins from human as well as yeast or other non-human source, including but not limited to animals.

Agents that either increase or decrease the efficiency of frameshifting alter the ratio of Gag to Gag-pol proteins expressed by viral genes. An increase of this ratio outside of a narrow range interferes with the assembly process of viral particles, because too much Gag-pol is available. A decrease in this ratio results in suppression of viral replication because the level of pol expression is too low. In either event, the end result is interference in the production of viral particles. In a specific embodiment, antibiotics that increase or decrease frameshifting are effective against HIV.

*UPF1* is found to be involved in mRNA decay process, and the allele of *UPF1* found in *mof4-1* stabilized nonsense mRNA.

There is disclosed a screening assay for the identification or agents that affect the function of proteins involved in the nonsense mRNA decay pathway. Such agents can be tested for the ability to stabilize nonsense or short mRNA transcripts. Identification of a protein involved in this pathway allows for rational selection of such agents. Many assays for nonsense-mediated decay are known in the art (Zhang et al., 1995, Mol. Cell. Biol. **15**:2231-2244; Hagan et al.. 1995. Mol. Cell. Biol. **15**:809-823: Peltz et al., 1994, in *Progress in Nucleic Acid Research and Molecular Biology* **47**:271-298). Agents that interfere with ATPase activity, helicase activity, or zinc finger motif configuration may be selected for testing.

A further discovery of the present invention is that recombinant genes for expression of mRNA. particularly for frameshift analysis, may yield artifactual results because the reporter transcript 5' of the frameshift site may be too short, and thus recognized as an aberrant transcript by the degradation machinery. In this instance, increased activity of the gene in the frameshift site may be the result of mutations that stabilize the nonsense-containing transcripts rather than that increase frameshift efficiency. The present invention has advantageously identified this problem for the first time, and thus provides strategies for overcoming rapid degradation of short transcripts that are recognized by the translation apparatus as aberrant nonsense-containing mRNA.

This latter result has critical implications for a number of different technologies. For example, identification of agents that inhibit the decay pathway or stabilize nonsense transcripts may be critical for the success of antisense RNA technology. Antisense RNAs are small, diffusible, untranslated and highly structured transcripts that pair to specific target RNAs at regions of complementarity, thereby controlling target RNA function or expression. However, attempts to apply antisense RNA technology have met with limited success. The limiting factor appears to be in achieving sufficient concentrations of the antisense RNA in a cell to inhibit or reduce the expression of the target gene. It is likely that one impediment to achieving sufficient concentration is the nonsense decay pathway, since the short antisense RNA transcripts, which are not meant to encode a gene product, will likely lead to rapid translation termination if translation occurs, and consequently to rapid degradation and low abundance of the antisense RNA in the cell. Thus, the agents that stabilize aberrant mRNA transcripts may also stabilize antisense RNAs.

The ability to stabilize nonsense mRNA has important implications for treating diseases associated with nonsense mutations, such as thalassemia. As with any biological system, there will be a small amount of suppression of a nonsense mutation, resulting in expression of a full length protein (which may or may not include an amino acid substitution or deletion). In the natural state, such low quantities of full length protein are produced that pathology results. However, by stabilizing the nonsense mRNA, the likelihood of "read-through" transcripts is dramatically increased, and may allow for enough expression of the protein to overcome the pathological phenotype.

The ability to stabilize short RNA transcripts will also aid in developing two-hybrid systems to genetically identify proteins that interact. For example, various peptides that interact with protein targets can be assayed if mRNAs encoding such peptides can be stabilized to allow for translation. RNAs that encode small peptide libraries will be unstable and be degraded by nonsense-mediated mRNA decay pathway in the absence of some agent to stabilize them.

Alternatively, strains that inactivate the decay pathway and stabilize aberrant RNA may be useful screening systems for tow-hybrid systems, since the natural tendency of such strains is to stabilize mRNAs.

### BRIEF DESCRIPTION OF THE DRAWINGS

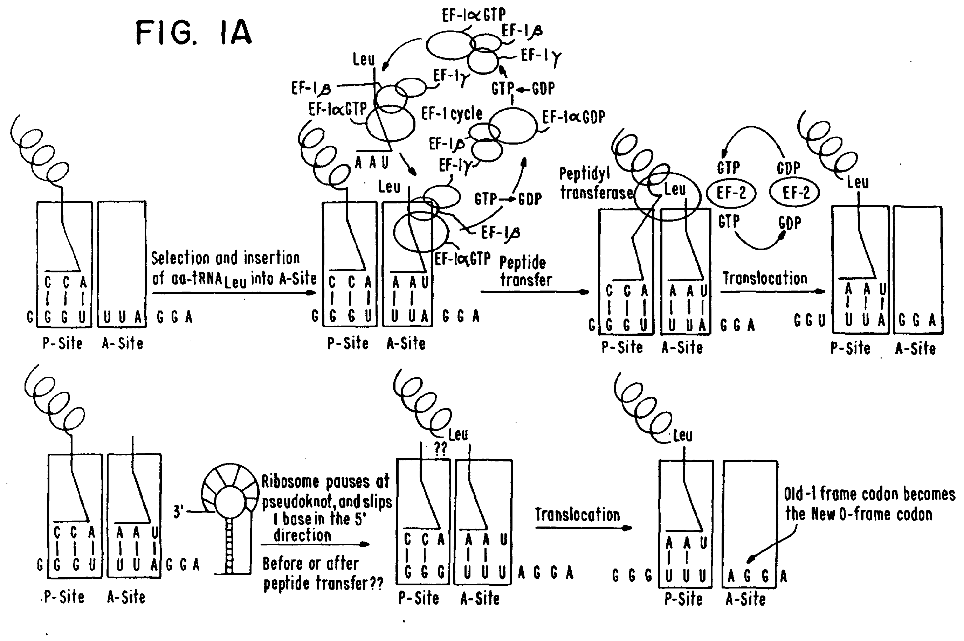

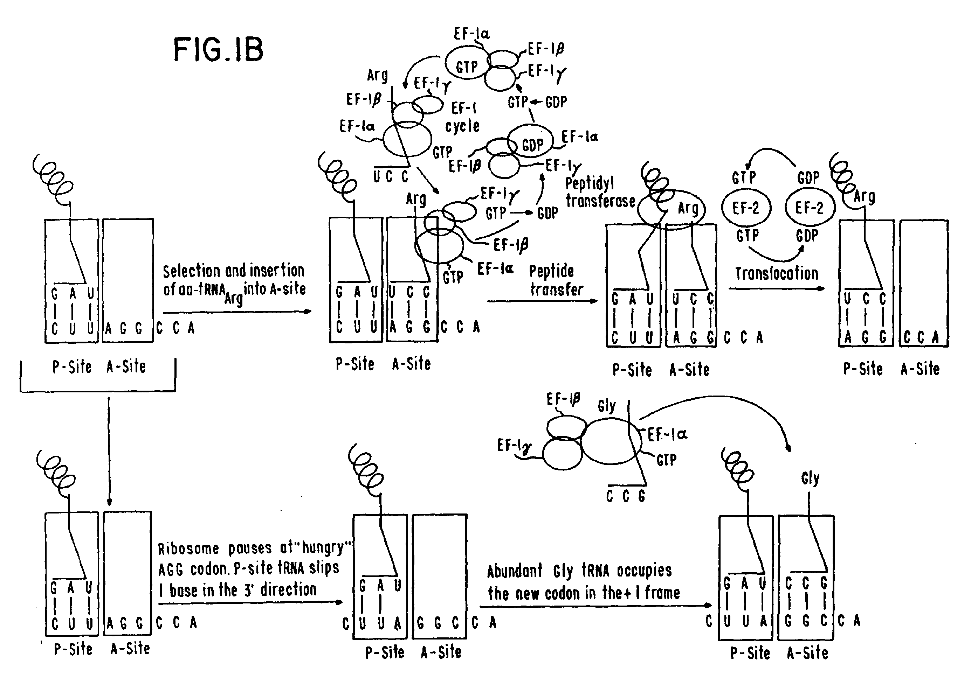

**FIGURE 1.** The "integrated model" of -1 ribosomal frameshifting. (A) depicts how -1 ribosomal frameshifting is related to the stages of translation elongation. (B) depicts how Ty1 mediated +1 ribosomal frameshifting is related to translational elongation.

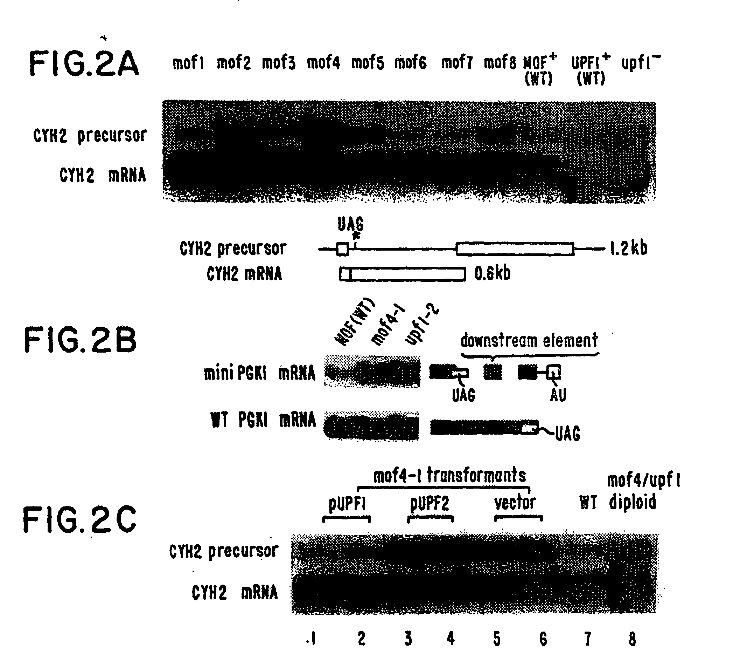

**FIGURE 2**. Characterization of the mRNA abundance of nonsense-containing mRNAs in *mof* strains. A) The mRNA abundance for the CYH2 precursor and CYH2 mRNA were determined by RNA blot analysis of RNAs from strains harboring the *MOF*⁺ (WT), *UPF1*⁺ (WT), *upf1*⁻ and the eight different *mof* alleles. The RNA blot containing 20µg of RNA per lane was hybridized with a radiolabeled CYH2 probe. A schematic representation of the CYH2 precursor and its spliced products is shown below the autoradiogram. B) The mRNA abundance for the wildtype PGK1 transcript and nonsense-containing mini-PGK1 mRNA was determined by RNA blot analysis of RNAs from strains harboring the *MOF*⁺ (WT), *mof4-1,* and *upf1-2.* The RNA blot was hybridized with a radiolabeled DNA fragment of the *PGK1* gene. Schematic representations of the nonsense-containing mini-*PGK1* allele and the wildtype *PGK1* gene are shown to the right of the autoradiogram. C) The *mof4-1* strain was transformed with centromere plasmids containing just the vector, harboring either of the UPF1 or the UPF2 gene and the diploid cells of mof4-1 crossed with a *upf1*Δ strain. The mRNA abundance of the CYH2 precursor was determined by RNA blot analysis as described in (A).

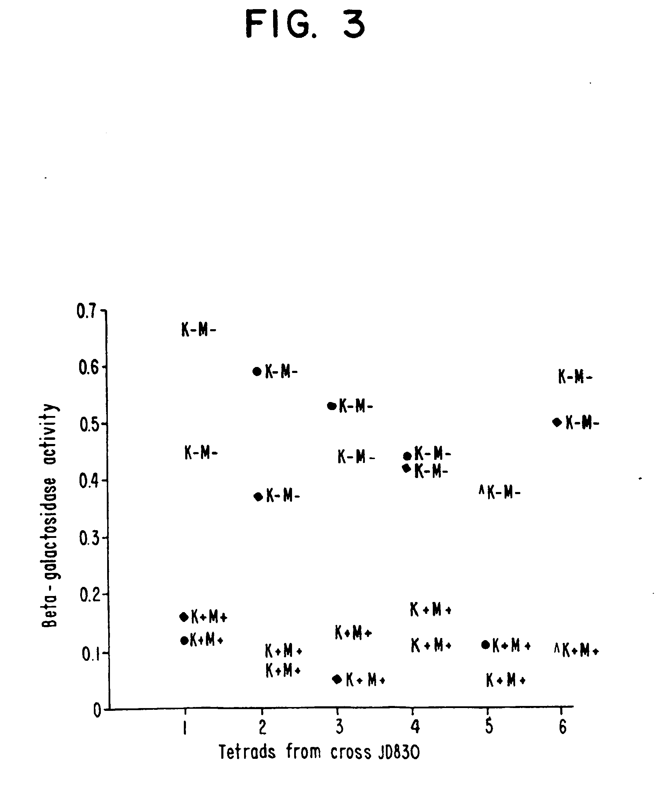

**FIGURE 3.** The killer phenotype and maintenance of M₁ co-segregate with *mof4-1.* Tetrad analysis was performed from a cross (cross JD830) between a *mof4-1* strain (JD474-3D) that does not maintain either the killer phenotype (K⁻) or double-stranded M₁ RNA (M₁₋) with a wildtype strain (JD742-2D; M +K+). Both parental strains contained the chromosomally integrated LacZ gene frameshift construct (*leu2-1::pJD85;* [Dinman and Wickner, *Genetics,* **136**:75-86 (1994)]). The spores from each tetrad were assayed for their β-galactosidase activity, their killer phenotype and their ability to propagate the double-stranded M₁ RNA as described in the Materials and Methods. The β-galactosidase activity (Y axis) for each set of tetrads (X axis) is shown as well as the ability of each of the spores to maintain either the killer phenotype K+/K⁻) or the double-stranded M₁ RNA (M₁+/M₁⁻).

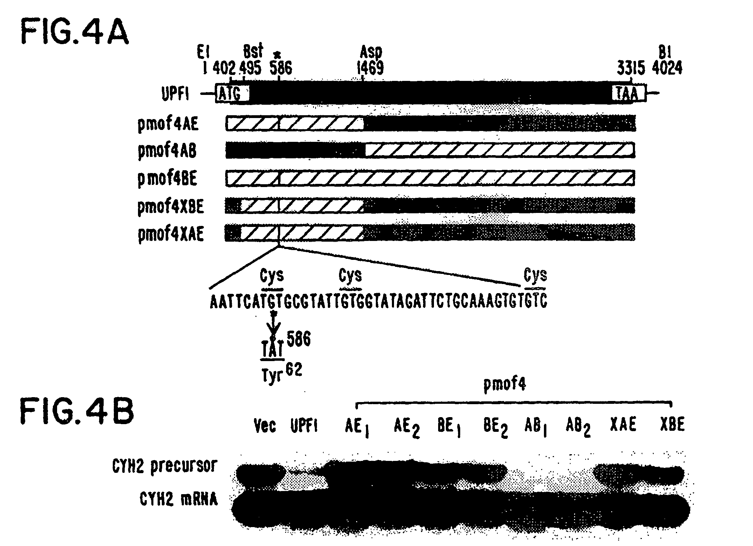

**FIGURE 4.** Identification and characterization of the lesion in the *mof4-1* allele. Hybrid genes between the wildtype *UPF1* and the *mof4-1* alleles schematically represented in panel A were constructed, transformed into a *upf1*Δ strain and CYH2 precursor abundance was determined by RNA blotting analysis as described in Figure 1. An autoradiogram of this analysis is shown in panel B. The black rectangle in panel A represents sequences from the wildtype *UPF1* gene while the hatched rectangle represents sequences from the *mof4-1* allele. The cysteine-rich region of the UPF1 gene is represented by gray rectangle in the wild type UPF1 gene. The dark vertical line represents the location of the mutation within the *mof4-1* allele. The *mof4-1* allele was sequenced and the sequence change is shown. For each hybrid allele shown in Panel A two identical constructs were prepared from different PCR reactions and are designated with the subscript 1 or 2 in panel B. The restriction endonucleases represented in panel A: E1(EcorR1), Bst(BstX1), Asp(Asp718), B1(BamH1).

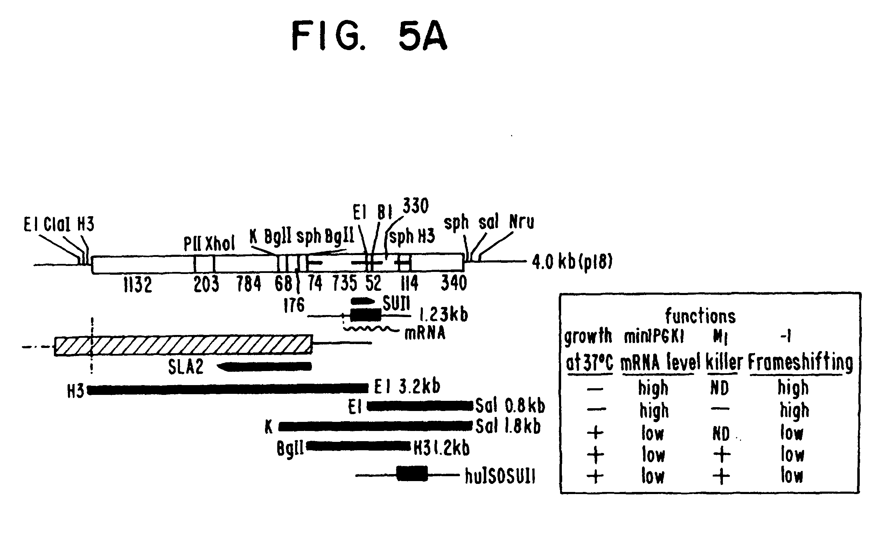

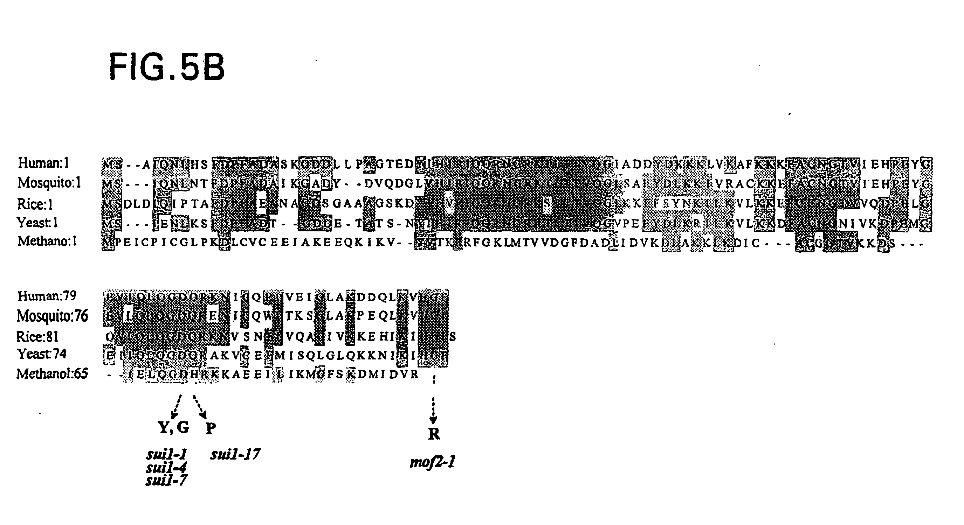

**FIGURE 5.** A) Cloning strategy for cloning *mof2-1.* B) Sequence analysis to identify the mutation in the *mof2-1* allele.

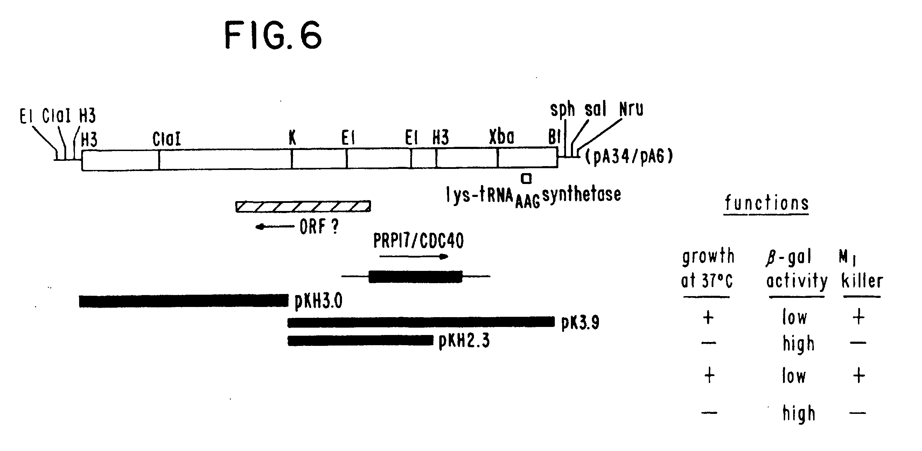

**FIGURE 6.** Cloning strategy for cloning *mof5-1.*

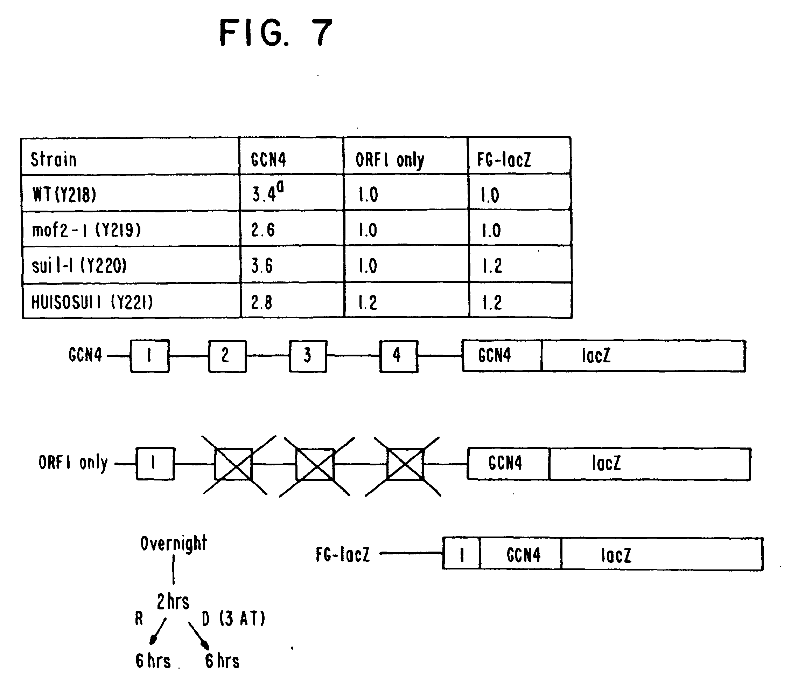

**FIGURE 7.** *Neither mof2-1 nor sui1-1 repress GCN4 expression.* Isogenic *sui1*Δ strains transformed with pSUI1, pmof2-1, psui1-1, or PHUISOSUI1 were co-transformed with the following reporter plasmids. pGCN4 = Contains the PCN4 upstream controlling region plus the first 10 amino codons of the GCN4 structural gene fused with the *lac*Z gene. pORF1 only contains only the first GCN4 upstream ORF (uORF), and the AUG's of the other uORFs have been deleted. In pFG-lacZ, uORF1 is fused in-frame with the GCN4-lacZ fusion. Cells were grown overnight in selective media, diluted to mid-log phase and grown for an additional 2 hours. Cultures were split, and 3-aminotriozole was added, derepressing GCN4 expression. After 6 hours, β-galactosidase activities were determined. The ratios of β-galactosidase activities of derepressed to repressed cells are shown. There are no significant differences in these ratios between wild-type and *mof*2-1, *sui*1-1 or HUISOSUI1 cells.

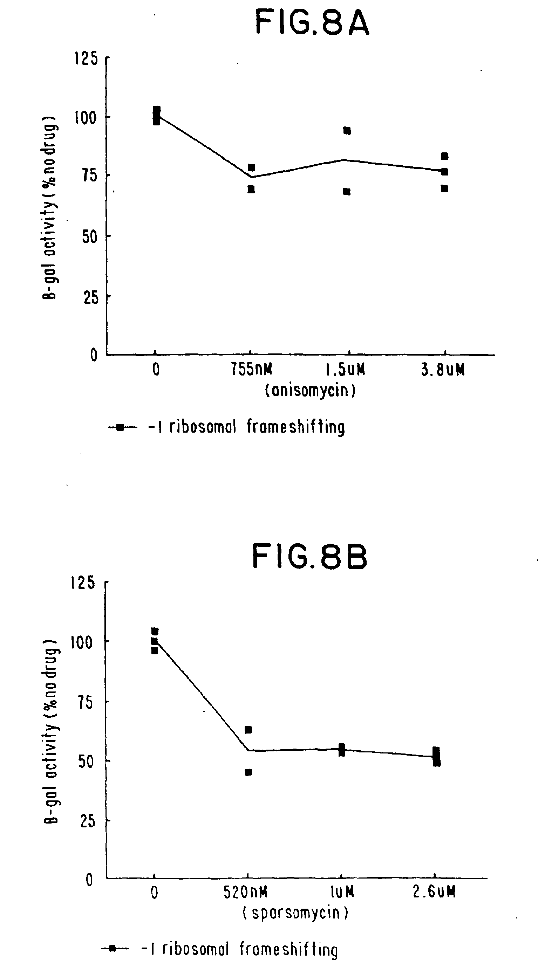

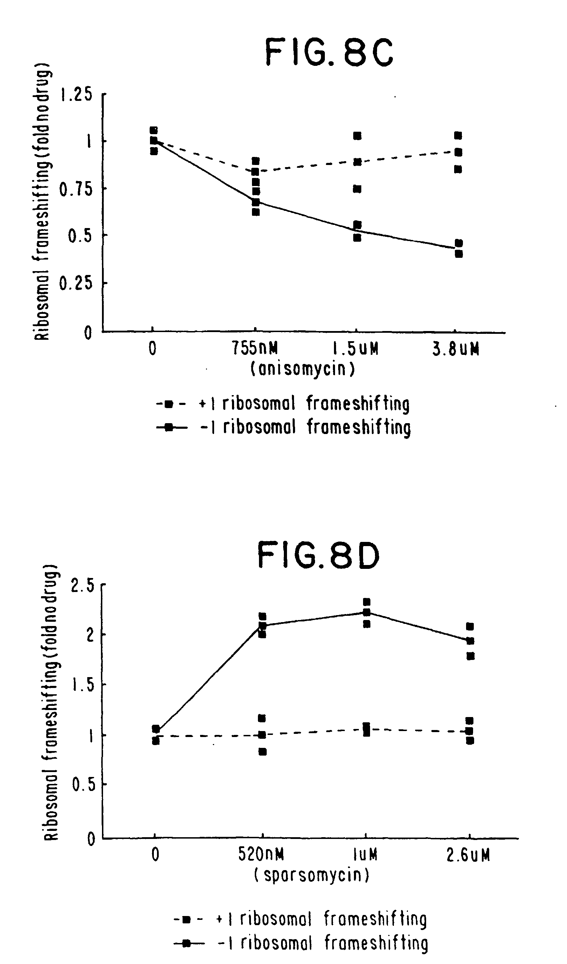

**FIGURE 8**. Selective medium (H-trp) containing the indicated concentrations of anisomycin (A, C) or sparsomycin (B, D) was inoculated to an O.D.₅₅₀ of 0.200 JD88 cells harboring the plasmids pTI25 (0-frame control), pF8 (-1 L-A derived ribosomal frameshift tester) [Jimenez et al., *Biochem. Biophys. Acta* 383:427 (1975)] or pJD104 (+1 Ty*1* derived ribosomal frameshift tester) [Balasundaram et al., *Proc. NAtl. Acad. Sci. USA* **91**:172 (1994)], and incubated at 30°C for 5 hrs, after which _-galactosidase (_-gal) activities were determined. (A, B) The _-gal activities produced from pTI25 as a percentage of the no-drug controls. ((C, D) The fold changes in in -1 or + 1 ribosomal efficiencies as compared to the no-drug controls (-1 = 1.8%; + 1 = 5.5%).

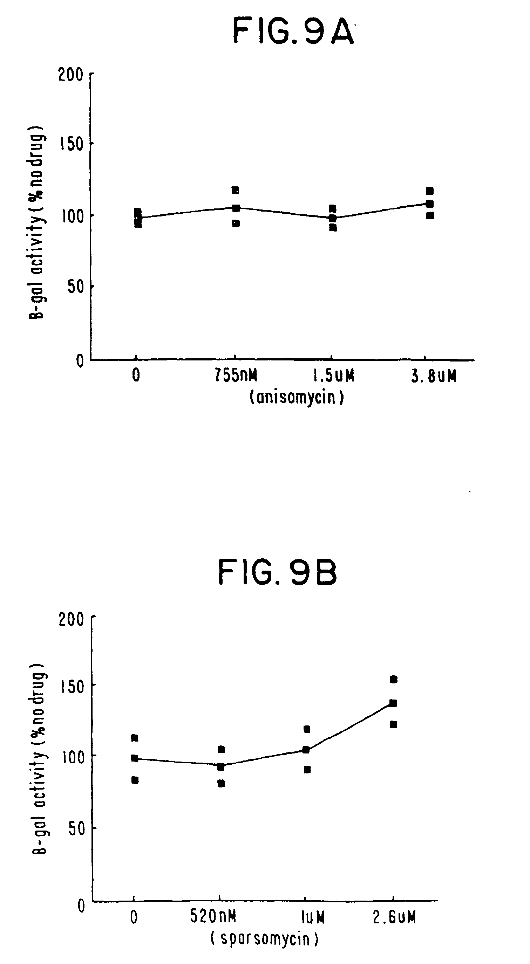

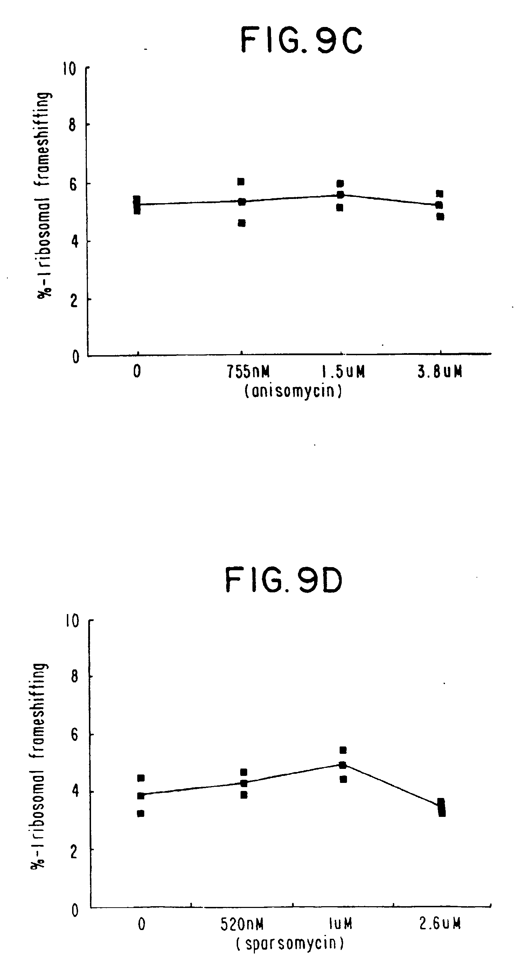

**FIGURE 9.** 1906 cells (*MATa leu2 mak8-2 K*⁻ *MKT*⁺) harboring either pJD136.0 or pJD136.-1 (*LEU2 CEN* vectors into which the *Hind* III fragments from either pTI25 or pF8 containing either the 0-frame control or -1 ribosomal frameshift indicator fragments were cloned) were inoculated to an O.D.₅₅₀ of 0.2 in H-Leu medium containing the indicated concentrations of anisomycin or sparsomycin and incubated at 30°C for 5 hrs, after which _-galactosidase (_-gal) activities were determined. (A, B) As described above for Figure 8A and 8B. (C, D) The ordinate depicts actual -1 ribosomal frameshifting efficiencies.

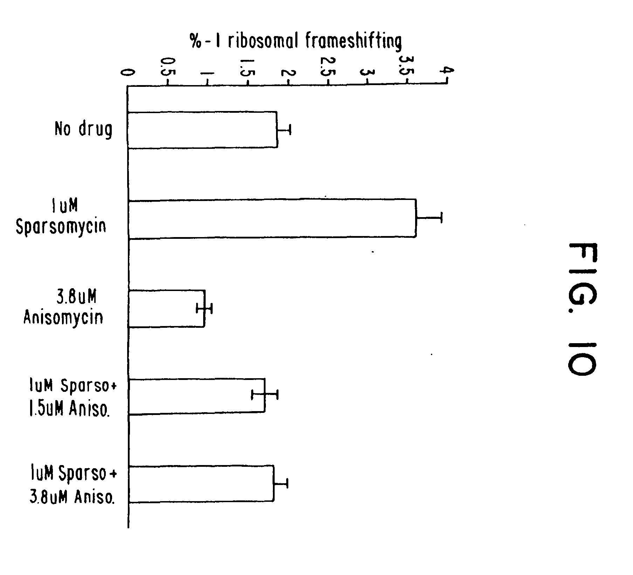

**FIGURE 10.** As described in Figure 8, JD88 cells were used in H-trp medium containing the indicated concentrations of drugs and the efficiencies of -1 ribosomal frameshifting were determined after 5 hours incubation at 30°C.

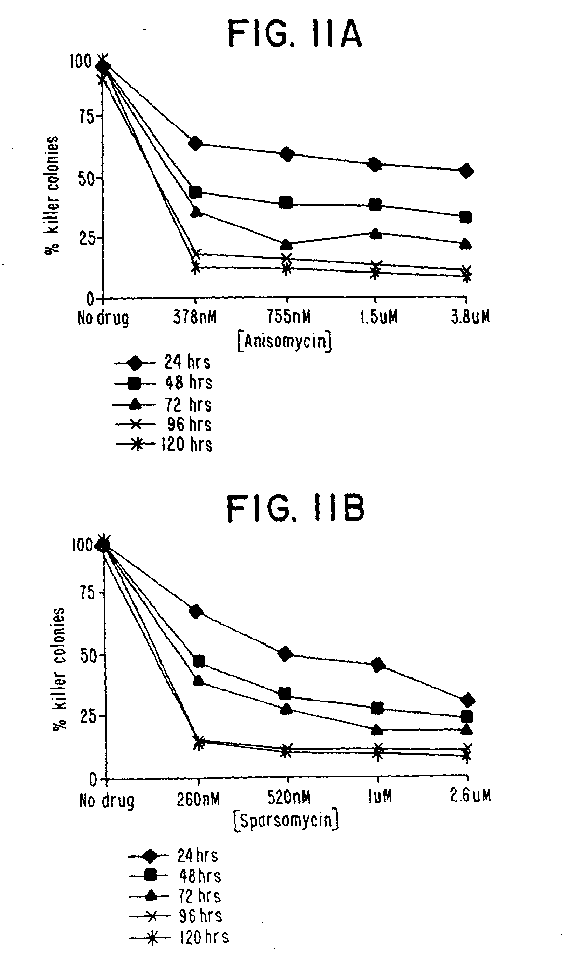

**FIGURE 11**. JD88 cells were cultured in rich medium containing the indicated concentrations of either anisomycin (A) or sparsomycin (B). After 24, 48, 72, 96, or 120 hrs., aliquots of cells were removed, washed twice with sterile water, streaked onto rich medium, and grown to single colonies at 30°C. These were then replica plated onto indicator plates, and scored for their killer phenotypes. Loss of the killer phenotype was measured by dividing the number of non-killer (K⁻) colonies by the total number of colonies. Each data set corresponds to > 100 total colonies.

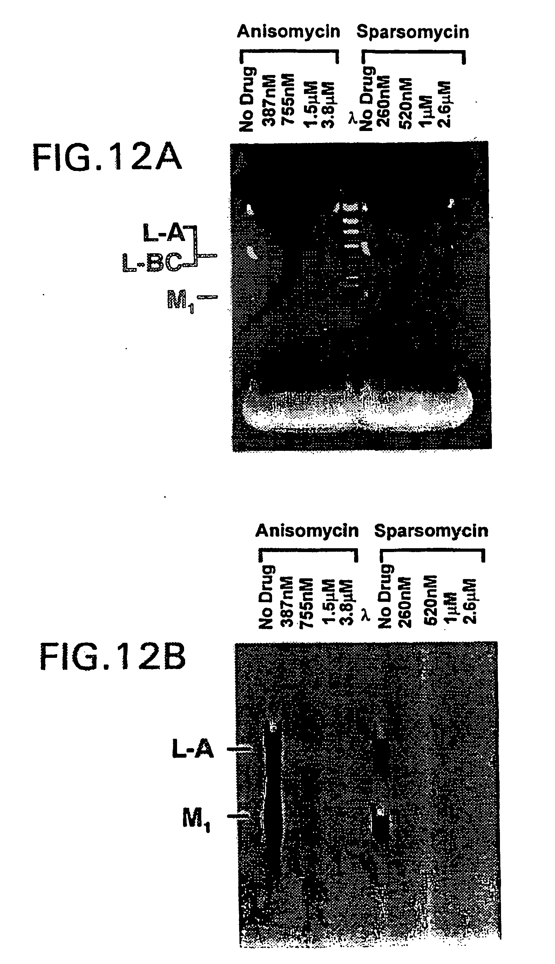

**FIGURE 12.** (A) A single non-killer (K⁻) colony from each drug concentration in the 72 hr data set was picked at random and total nucleic acids (TNA) were extracted [Dinman and Wickner, *Virology* **66**:3669 (1992)]. Approximately equal amounts of TNA were separated through a 1% non-denaturing TAE-agarose gel and stained with ethidium bromide. The 4.6 kb L-A and L-BC, and the 1.8 kb M₁ dsRNA bands are indicated. (B) RNA was denatured in the gel shown in (A), transferred to nitrocellulose and hybridized with [³²P]CTP labeled L-A and M₁ (+) strand RNA probes as described in [Dinman and Wickner, *Genetics* **136**:75 (1994)].

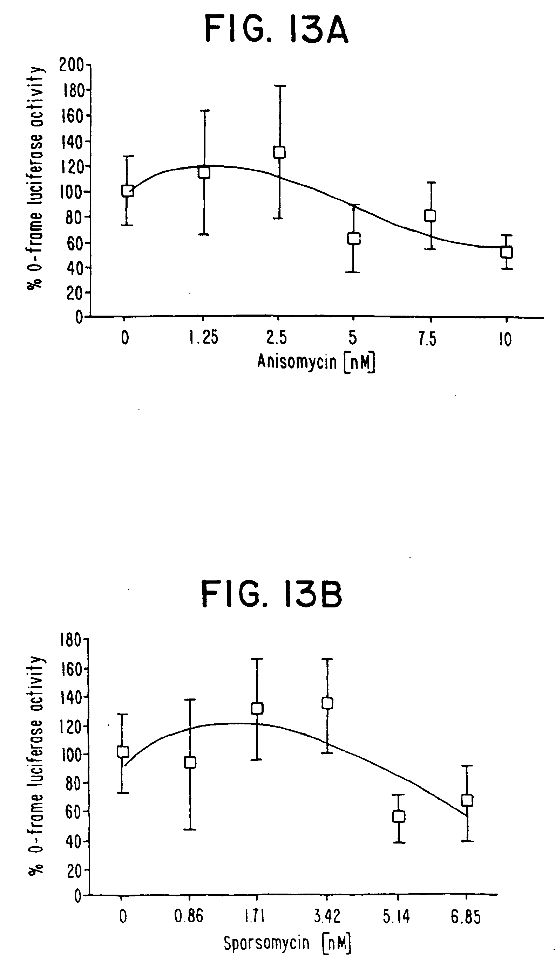

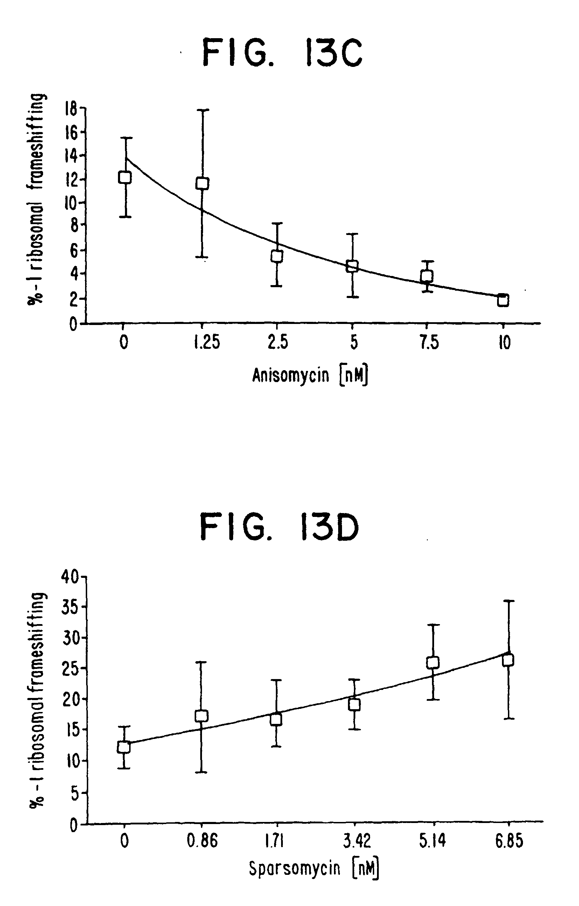

**FIGURE 13.** -1 ribosomal frameshifting *in vitro.* A luciferase based reporter system was constructed based on pT7--LUC minus 3'UTR-A₅₀ [Gallie et al., *Mol. Gen. Genet.* **288**:258 (1991)] (referred to here a pLUC0). The -1 ribosomal frameshift test plasmid construct (pJD120, referred to here as pLUC-1) contains (5' to 3') an AUG initiation codon, an L-A -1 ribosomal frameshift signal (from pF8), followed by the luciferase cDNA which is in the -1 reading frame with respect to the initiation codon. Methyl ⁷pppG capped, polyadenylated mRNA transcripts were made using pLUC0 and pLUC-1 linearized with *Dra I,* T7 RNA polymerase, and a mMessage mMachine *in vitro* transcription kit (Ambion). Translation competent yeast extracts were made from yeast strain JD696 (*MAT_ura3-52* [L-A-o M-o L-BC-o]) as described in [Iizuka et al., *Mol. Cell. Biol.* **14:**7322 (1994)]. 20 ng of LUC0 or LUC-1 mRNA, and the indicated concentrations of anisomycin or sparsomycin were incubated at 24°C for 1 hr with 15 _1 of the yeast extracts in triplicate and luciferase activities of each sample were determined using a luminometer (Turner Designs Model 20/20). (A, B) Luciferase activities generated using the LUC0 reporter mRNAs as the percentage of no drug controls. (C, D) Efficiency of -1 ribosomal frameshifting which was calculated by dividing the luciferase activity from the LUC-1 reporter mRNA by that generated from the LUC0 control mRNA.

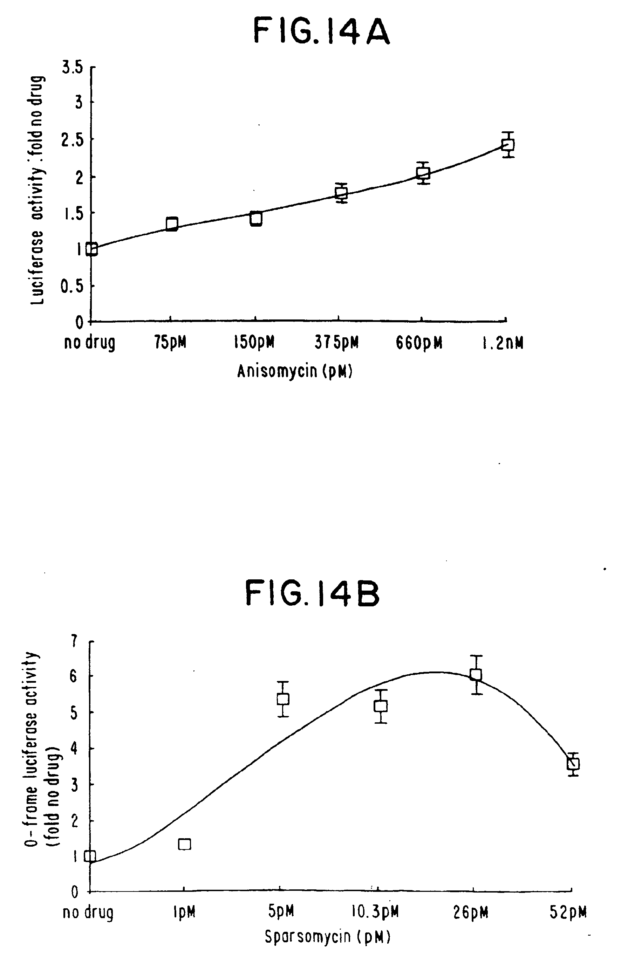

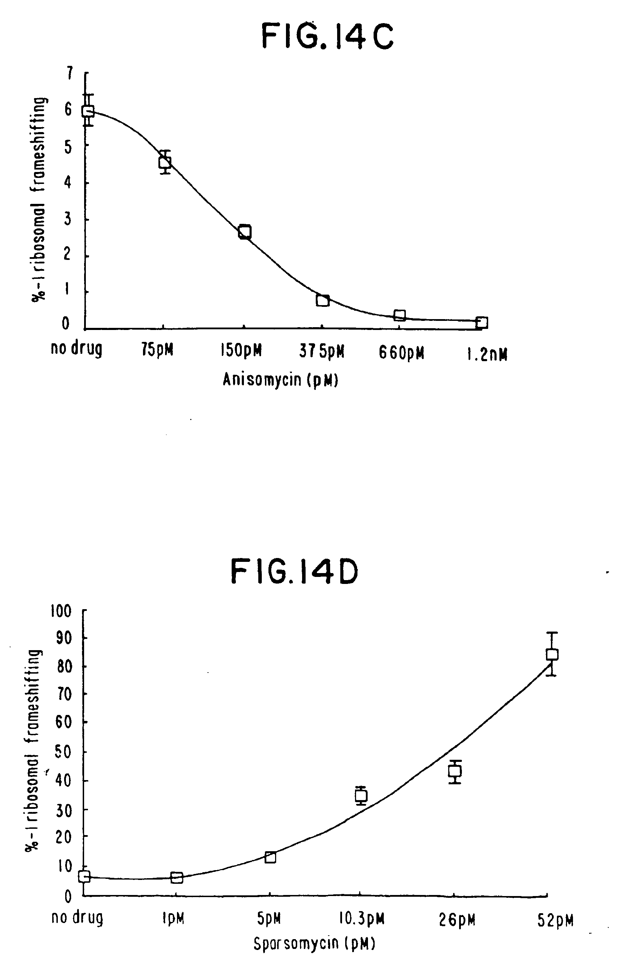

**FIGURE 14.** The LUC0 and LUC-1 mRNAs were used to measure the effects of anisomycin and sparsomycin on translation and -1 ribosomal frameshifting in an *in vitro* rabbit reticulocyte translation system (Retic Lysated IVT kit, Ambion). As in Figure 13, 20ng of either LUC0 or LUC-1 mRNAs were used for these assays.

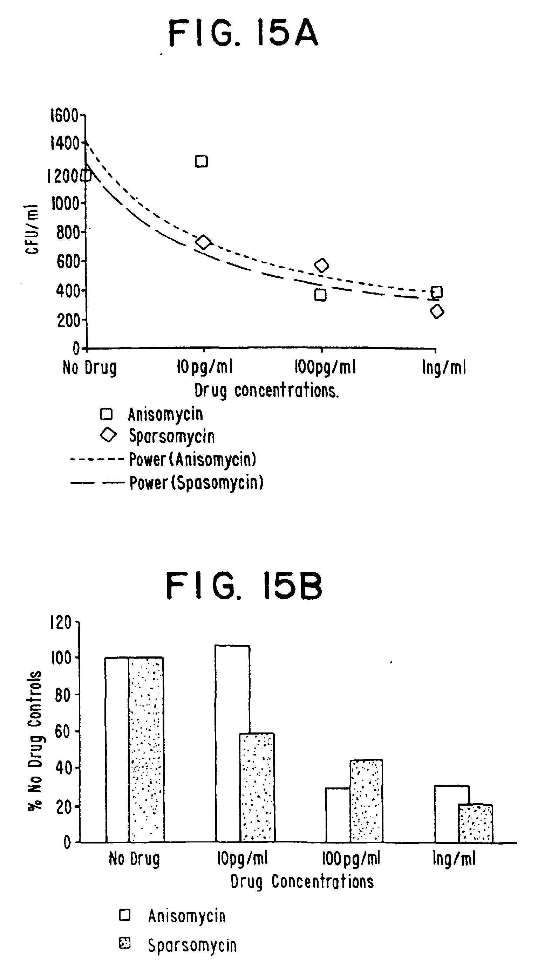

**FIGURE 15**. Asinomycin and sparsomycin decrease titers of HIV. Virus producer cells (#69 HIVgpt) were incubated in DMEM containing 10% fetal calf serum with the indicated concentrations of either anisomycin or sparsomycin and incubate at 37_C. After 4 days, virus containing supernatants were harvested, a series of dilutions were made, and virus reporter cells (HELA T4) were incubated with 0.3 ml of diluted supernatant solutions for 3 hours, after which they were aspirated and replaced with 3 ml of DMEM/10% fetal calf serum. Each dilution was tested in duplicate. After 24 hours, the growth medium was replaced with DMEM/10% fetal calf serum containing 7 ug/ml gpt, to select against uninfected cells. Medium was subsequently changed every 3 days. After 14 days, colonies were counted for each dilution, and the total number of colony forming units was determined by multiplying the dilution factor times 3.33. (A) HIV titers of human cells treated with anisomycin (open squares) or sparsomycin (open diamonds). (B) Effects of asinimycin (open bars) and sparsomycin (solid bars) in HIV titers as a percentage of untreated infected cells.

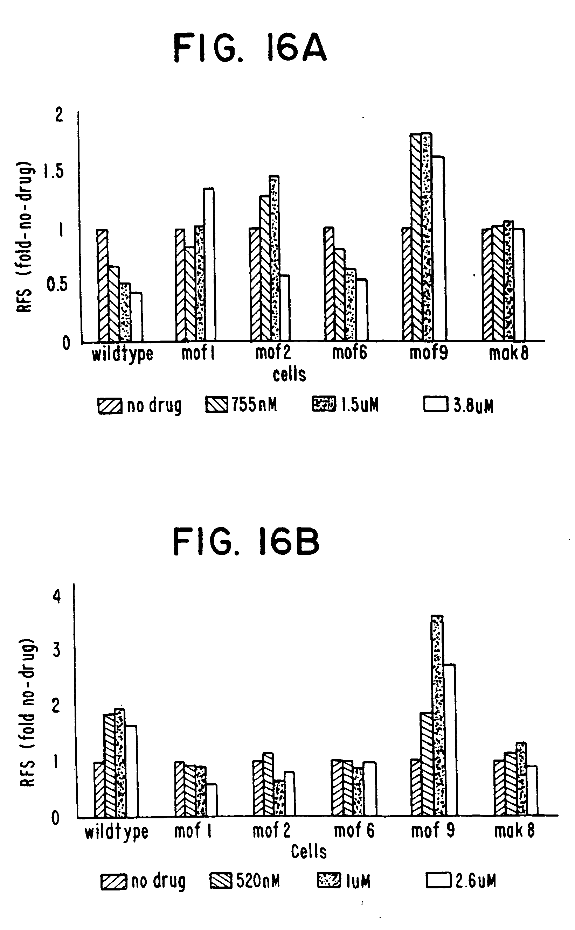

**FIGURE 16.** (A) Effects of anisomycin on -1 ribosomal frameshifting. (B) Effects of sparsomycin on -1 ribosomal frameshifting. Wildtype of *mof* mutant cells were treated with the indicated amounts of each drug, and the relative ribosomal frameshifting was evaluated and normalized to cells without drug treatment.

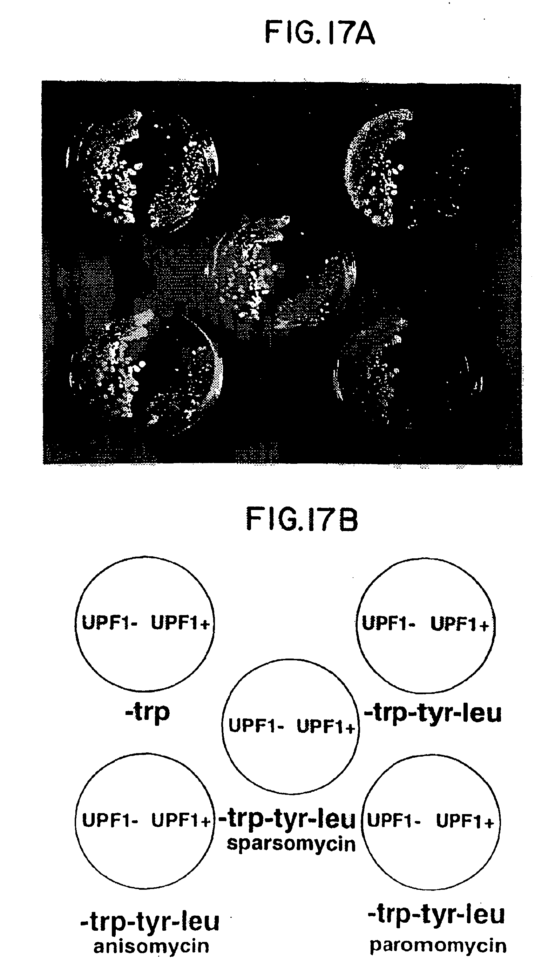

**FIGURE 17.** Effects of drugs at a concentration of 5 µg/ml on nonsense supression in UPF+ and UPF- strains. (A) Strain growth. (B) Labels.

### DETAILED DESCRIPTION OF THE INVENTION

Ribosomal frameshifting is a critical aspect of gene expression in many retroviruses and RNA viruses. In addition, mRNA degradation is an important control point in the regulation of gene expression and has been shown to be linked to the process of translation. One clear example of this linkage is the observation that nonsense mutations accelerate the degradation of mRNAs. The present invention represents the first identification of proteins that mediate either of these activities.

Understanding how ribosomes maintain translational reading frame is a major challenge for the fields of translational control and virology. In the past ten years, it has been shown that a number of eukaryotic viruses induce ribosomes to shift reading frame in order to regulate the expression of gene products having enzymatic functions. Historically, many of the definitive experiments in the fields of molecular biology and virology have exploited genetically malleable host cell/virus systems. The present invention is based, in part, on studies on ribosomal frameshifting in one such system, the L-A virus of the yeast *Saccharomyces cerevisiae.* These studies have demonstrated that 1) viral mRNA sequences and secondary structures can modulate the efficiency of -1 ribosomal frameshifting; 2) the efficiency of -1 ribosomal frameshifting is critical for the propagation of the M₁ satellite virus of L-A, and 3) mutants of yeast host chromosomal gene products involved in *M*aintenance *O*f translational reading *F*rame (*mof*) can be obtained, many of which have interesting secondary phenotypes.

Figure 1A shows a model integrating the translation elongation cycle with the current model describing -1 ribosomal frameshifting. Examination of this integrated model reveals that, since -1 ribosomal frameshifting occurs during translational elongation where both ribosomal A- and P-sites are occupied, this event must occur after the delivery of aminoacyl-tRNA by EF-1α to the A-site, and before EF-2 mediated translocation: peptide bond formation and peptidyl transfer occur within these parameters. Overexpression of a fragment of the yeast ribosomal protein L3, which is involved in peptidyl transferase center formation [Fried and Warner, *Proc. Natl. Acad. Sci. USA* **78**:238 (1981); Schultz and Friesen, *J. Bacteriol.* **155**:8 (1983); Schultze and Nierhaus, *EMBO J.* **5**:609 (1982)] increases the efficiency of -1 ribosomal frameshifting and promotes loss of M₁ in wild-type cells. Thus, pharmacological agents that affect the peptidyl transferase center affect the efficiency of -1 ribosomal frameshifting and interfering with viral propagation.

Figure 1B shows that, in contrast, since Ty*1* directed +1 ribosomal frameshifting requires slippage of a specific P-site tRNA, this process should not be affected by such a class of drugs. Rather, conditions that would serve to change translational parameters during the time that only the P-site is occupied by peptidyl-tRNA would be predicted to affect the efficiency of + 1 ribosomal frameshifting.

Current models describing -1 and + 1 ribosomal frameshifting have been integrated within the context of the translational elongation cycle. The resulting "integrated model" has tremendous predictive value with regard to identifying agents which may change the efficiencies of ribosomal frameshifting in either direction, which in turn are predicted to cripple the ability of cells to propagate a wide range of viruses that rely on ribosomal frameshifting strategies to ensure correct morphogenesis. Among human pathogens, these would include most of the retroviruses including HIV, as well as a number of dsRNA and (+) stranded ssRNA viral pathogens. This strategy also has applications with regard to viral diseases of veterinary and agricultural importance.

The model predicts that agents which change translational parameters after insertion and selection of aa-tRNA by EF-1, through the peptidyl-transfer step, and before EF-2 mediated translocation, should alter the efficiency of -1 ribosomal frameshifting. Therefore, although we have tested it using only two antibiotics which specifically target the peptidyl transferase function of eukaryotic ribosomes, the model predicts that other antibiotics acting at the same step (*e.g*., the sesquiterpene antibiotics of the trichodermin group) should yield similar results.

In particular, the present invention relates, in part, to the discovery that a subset of *mof* alleles in yeast (Dinman and Wickner, 1994, Genetics 136:75-86) which were isolated as chromosomal mutations that increased the frameshifting efficiency at the L-A virus frameshift site and caused loss of the L-A satellite virus M₁, also affect the nonsense-mediated mRNA decay pathway. The levels of nonsense-containing mRNAs were elevated in cell harboring the *mof4-1* allele, and to a lesser extent in cells containing *mof2-1, mof5-1,* and *mof8-1* alleles.

The invention further relates to the discovery that *mof4-1* is allelic to *UPF1,* which has been demonstrated to be involved in the nonsense-mediated mRNA decay pathway. In addition, *mof2-1* has been discovered to be allelic to *SUI1,* which has a human homologue, and *mof5-1* has been discovered to be allelic to *PRP17*/*CDC40.* Cloning of *mof4-1* is described in the Example, *infra.* Example 2 presents the cloning of *mof2-1,* and identifies the mutation of this allele; Figure 6 presents the cloning of *mof5-1.*

A genetic and biochemical study of the *UPF1* gene was undertaken in order to understand the mechanism of Upf1p function in the nonsense-mediated mRNA decay pathway. Our analysis suggests that Upf1p is a multifunctional protein with separable activities that can affect mRNA turnover and nonsense suppression. Mutations have been identified in the conserved helicase motifs of the Upf1p that inactivate its mRNA decay function while not allowing suppression of *leu2-2* and *tyr7-1* nonsense alleles. In particular, one mutation located in the ATP binding and hydrolysis motif of Upf1p that changed the aspartic and glutamic acid to alanine residues (DE572AA) lacked ATPase and helicase activity and formed a Upf1p:RNA complex in the absence of ATP. Surprisingly, however, the Upf1p:RNA complex dissociated as a consequence of ATP binding. This result suggests that ATP binding, independent of its hydrolysis, can modulate Upf1p:RNA complex formation for this mutant protein. In addition, mutations in the amino terminal cysteine/histidine-rich region of the Upf1p have been identified and biochemicaly characterized that have normal nonsense-mediated mRNA decay activities but are able to suppress *leu2-2* and *tyr7-1* nonsense alleles. Biochemical characterization of these mutant proteins demonstrated that they have altered RNA binding properties. Furthermore, using the two-hybrid system, we characterize Upf1p-Upf2p and demonstrate the Upf2p-Upf3p interactions. Mutations in the cysteine/histidine-rich region of the Upf1p abolish Upf1p-Upf2p interaction. Based on these results, the role of the Upf complex in nonsense-mediated mRNA decay and nonsense suppression appears to be mediated by separate domains on the protein. This has obvious implications for drug targeting, in that one or the other domain can be targeted for drug developement, *e.g*., using the combinatorial library techniques or rational drug design techniques.

In a further aspect, a clone has been obtained, as described herein, which demonstrates that one such mutant, *mof2-1,* is a unique allele of the yeast *SUI1* gene. Although *sui1-1* mutants have somewhat elevated efficiencies of -1 ribosomal frameshifting, *mof2-1* is unique in that is the only known allele of *SUI1* that promotes loss of the M₁ dsRNA satellite virus. Furthermore, -1 ribosomal frameshifting is specifically affected by these mutants, in that they have no effects on ribosomal frameshifting in the + 1 direction. The *mof2-1* mutation also affects the nonsense-mediated mRNA decay pathway. In this respect, the present application shows that the *mof2-1* mutation has an intermediate nonsense-mediated mRNA decay phenotype, in that it requires a minimum of 2 downstream elements in order to activate this pathway. The ability to suppress the *his4*^{*uag*} mutation demonstrates that *mof2-1* mutants also have a Sui⁻ phenotype. However, unlike *sui2* and *SUI3* mutants, *mof2-1* mutants are not able to repress expression of the *GCN4* gene. Expression of the human homologue of this gene can correct the mutant phenotypes in yeast.

Based upon these new data, the present invention teaches that the Sui1 protein (Sui1p) plays a role in monitoring translational fidelity throughout all stages of translation. Moreover, addressing the role of the Sui1/Mof2 protein in translation, the present invention indicates that this protein aids in the proofreading function of ribosomes at all stages of translation, including initiation, elongation and termination. Recently, reversion analysis of *sui1* mutants has identified 5 suppressor loci called *ssu* (suppressors of *sui1*), that improve growth of a *sui1* mutant at restrictive temperatures. *Ssu1* encodes ribosomal protein S4 *RPS4* and *ssu4* encodes *RPS26. RPS4* corresponds to *E. coli* S5, a known *ram* (*r*ibosomal *am*biguity) mutant. Ram proteins have been implicated in ribosomal P-site editing during translational elongation. Although not intending to be limited thereby, the present invention is further based on the hypothesis is that, in interacting with yeast equivalent of bacterial Ram proteins, *mof2-1* acts as a yeast *ram* mutant. The yeast equivalent of bacterial *ram* proteins are: bacterial S5 = yeast RPS4 = *sup44,* and bacterial S4 = yeast RPS13A = *sup46.*

The invention further permits examination of 1) whether *SUP44* and/or *SUP46* are synthetically lethal with *mof2-1*; 2) whether the overexpression of wild-type *sup44* and *sup46* is able to suppress the *mof2-1* phenotypes, and 3) determine whether the wild-type Sui1p is ribosome associated, and whether there are qualitative differences between the different forms of the Sui1p (Sui1-1p, Mof2-1p and the human Sui1p) to bind ribosomes.

The present invention further hypothesizes that the Mof2 protein (Mof2p) is a general regulator of translational fidelity, and acts as a stimulatory factor upon nucleotide triphosphate (NTP) hydrolysis. Sui1p physically interacts with eIF5, stimulating GTP hydrolysis during the initiation step. Further, two of the *ssu* mutants, *ssu2* and *ssu3* encode eIF5 and eIF2γ respectively. The suppressor mutations in both of these G-proteins map to the regions containing homology to G-proteins, suggesting that these suppressor mutants alter the fidelity of translation by changing GTP hydrolysis activity in these translation initiation factors. Accordingly, the present invention proposes to: 1) examine the effects of purified wild-type, Mof2-1p, Sui1-1p and hISOSUI1p on GTP hydrolysis with purified G-proteins known to be involved in elongation phase of translation, *i.e*., EF-1α, and EF-2; 2) examine the effects of these forms of Sui1p on ATP hydrolysis by Upf1p and mutants thereof; 3) examine synthetic lethality of *mof2-1* with alleles of *TEF2* (encoding EF-1α), EF-2, and *UPF1.* Gene dosage experiments indicate whether overexpression of EF-1α, EF-2, or Upf1p can suppress either the *mof2-1,* or the *sui1-1* mutations.

The invention further relates to the discovery that *ifs1* and *ifs2* alleles, which were previously identified as mutations that enhance frameshifting at the -1 ribosomal frameshift signal from mouse mammary tumor virus, are allelic to the *UPF2* and *UPF1* genes, respectively, although both *ifs* strains maintained M₁.

In addition, expression of the N-terminal 100 amino acids of the *TCM1*/*MAK8* gene also increases the efficiency of -1 ribosomal frameshifting and interferes with replication of the M₁ dsRNA virus of L-A. Additionally, other non-antibiotic agents which affect elongation within the specific window should also affect the efficiency of -1 ribosomal frameshifting.

To determine a suitable range of drug concentrations the effects of anisomycin and sparsomycin on cell growth were assayed. We determined that anisomycin concentrations ranging from 0.76 -3.8 µM, and sparsomycin concentrations ranging from 0.52 - 2.6 µM inhibited overall cellular growth rates by less than 30% (data not shown). These ranges of drug concentration were selected for further *in vivo* investigations.

### Ribosomal Frameshifting and Nonsense in RNA Decay

The strategy employed in the identification of the *mof* mutants relied upon finding cells expressing increased amounts of β-gal. Increased efficiencies of -1 ribosomal frameshifting would result in the increased expression of β-gal when the *lacZ* gene is downstream of a L-A -1 ribosomal frameshift signal and in the -1 reading frame with regard to the translational start site. The same result could, however, be observed as a consequence of chromosomal mutations other than those affecting -1 ribosomal frameshifting efficiencies. For example, mutations increasing the stability of the *lacZ* gene product, or mutations increasing its transcriptional rate could also yield the desired result. The most interesting possibility would be mutations which increase the half-life of the *lacZ* reporter mRNA.

All of the *mof* mutants were tested for their Upf⁻ specific mRNA decay phenotypes (11). Since the half-life of the endogenous CYH2 precursor mRNA is known to be increased in *upf* mutants, the abundance of the CYH2 precursor was determined in wild-type and *mof* mutants. The abundance of the CYH2 precursor RNA was slightly elevated in the *mof2-1, mof5-1* and *mof8-1* mutants and that it was greatly increased in *mof4-1* cells. Mutant nonsense-mediated mRNA decay phenotypes of *mof2-1, mof4-1, mof5-1, mof7-1* and *mof8-1* are enhanced using a mini-PGK1 reporter construct which contains only one DSE. Increasing the number of DSEs decreases this mutant phenotype, especially in *mof2-1* mutants.

Complementation testing revealed that *mof2-1, mof5-1* and *mof8-1* do not correspond to any known mutations in the nonsense-mediated mRNA decay pathway. *mof4-1* has been identified as an allele of the *UPF1* gene. Diploid cells resulting from a cross of *mof4-1* and *upf1-2* cells had β-gal activities indistinguishable from the *mof4-1* partner, and the abundance of the CYH2 precursor in these cells remained elevated. Introduction of a single copy centromere based plasmid containing the *UPF1* gene into *mof4-1* cells was able to correct both mutant phenotypes, whereas introduction of a similar plasmid containing the *UPF2* gene, or the vector alone did not have any affect upon the mutant phenotypes of the *mof4-1* cells.

Although the half lives of the -1 ribosomal frameshifting reporter mRNAs are increased in *upf* mutants, the ribosomes translating them would continue to frameshift with the same efficiency. Thus, although *upf* mutants should be indistinguishable from *mof* mutants by the β-gal assay, *upf* mutants should be able to maintain the M₁ virus because the ratio of Gag to Gag-pol would remain unaffected. True *mof* mutants, by virtue of their affect upon -1 ribosomal frameshifting efficiency should not be capable of propagating M₁ however. Three of the four *mof* mutants that also have the Upf⁻ phenotype, i.e. *mof2-1, mof4-1, mof5-1* are incapable of propagating M₁, and are thus true *mof* mutants. Only *mof8-1,* which has a weak Upf⁻ nonsense mRNA decay mutant phenotype is capable of maintaining M₁.

The cloning and characterization of *mof4-1* is a model for an understanding of translational elongation processes, and of how we may apply our insights toward the rational development of antiviral agents that specifically target ribosomal frameshifting. The *mof4-1* allele of *UPF1* is interesting in that this is the only known allele of *UPF1* that is incapable of maintaining the M₁ satellite virus. With its increased efficiency of -1 ribosomal frameshifting and high-abundance of nonsense-mRNAs, the *mof4-1* allele of *UPF1* defines a new class of mutant. The *mof4-1* allele was sequenced and determined that it consists of a cystine (Cys) to tyrosine missense mutation at amino acid 62, the first Cys residue in the putative zinc finger. Our data demonstrate that there is a connection between the phenomena defined by the *mof* and *upf* mutants, illuminating the continuity in the translational process, from mRNA stability through the synthesis of the complete protein product. *mof4-1* is also sensitive to the translational inhibitor paromomycin, and the efficiency of -1 ribosomal frameshifting can be increased with increasing concentrations of paromomycin. This represents the first demonstration that the efficiency of -1 ribosomal frameshifting can be modulated by a specific drug, and as such has broad pharmacological implications.

In addition to examining the effects of the *mof* mutants upon the accumulation of the CYH2 precursor mRNA, the abundance of other nonsense-containing mRNAs were also determined. These mRNAs are encoded by mini-*PGK1* alleles harboring different stop codons (UUA, UAG, UGA), the *HIS4* gene with a stop codon inserted into the NheI site (*HIS4*-UGA(Nhe)) and the full length *PGK1* gene containing nonsense codons at different positions within the coding region (*PGK1*-n-UAG-AU) ((25,57) and see fig 2A -2D for constructs). The stabilities of these mRNAs were previously demonstrated to be dependent on the Upf gene products.

The mRNA levels from the mini-PGK1 and HIS4-UAG(Nhe) alleles in the *mof2-1, mof5-1* and *mof8-1* cells were almost as high as in *mof4-1* and Upf cells and were not dependent on the type of stop codon. Interestingly, the location of a stop codon within the *PGK1* gene affects the nonsense-mediated mRNA decay phenotype, especially in the case of *mof2-1.* In the H2(3) mutation, the UAG terminator occurs before all of the downstream elements, and there are only 2 (known) downstream elements 5' of the H2(2) mutation. The levels of the PGK-n-UAG-AU mRNAs (where the nonsense codons occur after the downstream elements have been translated) in *mof2-1* cells match those seen in wild-type cells. This is an interesting finding and testable hypotheses for these different effects will be described below.

In view of the foregoing, it becomes apparent that the present invention provides a number of routes for affecting ribosomal frameshifting, which has important implications for antiviral therapy and for suppression of pathological nonsense mutations. More importantly, two antibiotics and an approximately 100-amino acid N-terminal segment of a ribosome binding protein, L3, disrupt the normal frameshift and nonsense decay pathways. Thus, the present invention provides drugs for use as antiviral compounds or to alter ribosomal decay.

The term "drugs" is used herein to refer to a compound, such as an antibiotic or protein, that can affect function of the peptidyl transferase center. Such compounds can increase or decrease -1 frameshift efficiency; in either event, the result is disruption of protein expression that has antiviral consequences, or can suppress nonsense mutations.

### Genes Encoding Frameshift or mRNA Decay Proteins

In accordance with the present invention there may be employed conventional molecular biology, microbiology, and recombinant DNA techniques within the skill of the art. Such techniques are explained fully in the literature. See, *e.g.,* Sambrook, Fritsch & Maniatis, *Molecular Cloning: A Laboratory Manual,* Second Edition (1989) Cold Spring Harbor Laboratory Press, Cold Spring Harbor, New York (herein "Sambrook et al., 1989"); *DNA Cloning: A Practical Approach,* Volumes I and II (D.N. Glover ed. 1985); *Oligonucleotide Synthesis* (M.J. Gait ed. 1984); *Nucleic Acid Hybridization* [B.D. Hames & S.J. Higgins eds. (1985)]; *Transcription And Translation* [B.D. Hames & S.J. Higgins, eds. (1984)]; *Animal Cell Culture* [R.I. Freshney, ed. (1986)]; *Immobilized Cells And Enzymes* [IRL Press, (1986)]; B. Perbal, *A Practical Guide To Molecular Cloning* (1984); F.M. Ausubel et al. (eds.), *Current Protocols in Molecular Biology,* John Wiley & Sons, Inc. (1994).

Therefore, if appearing herein, the following terms shall have the definitions set out below.

A "vector" is a replicon, such as plasmid, phage or cosmid, to which another DNA segment may be attached so as to bring about the replication of the attached segment. A "replicon" is any genetic element (e.g., plasmid, chromosome, virus) that functions as an autonomous unit of DNA replication *in vivo, i.e.,* capable of replication under its own control.

A "cassette" refers to a segment of DNA that can be inserted into a vector at specific restriction sites. The segment of DNA encodes a polypeptide of interest, and the cassette and restriction sites are designed to ensure insertion of the cassette in the proper reading frame for transcription and translation.

A cell has been "transfected" by exogenous or heterologous DNA when such DNA has been introduced inside the cell. A cell has been "transformed" by exogenous or heterologous DNA when the transfected DNA effects a phenotypic change. Preferably, the transforming DNA should be integrated (covalently linked) into chromosomal DNA making up the genome of the cell.

"Heterologous" DNA refers to DNA not naturally located in the cell, or in a chromosomal site of the cell. Preferably, the heterologous DNA includes a gene foreign to the cell.

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester anologs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, *inter alia,* in linear or circular DNA molecules (*e.g*., restriction fragments), plasmids, and chromosomes. In discussing the structure of particular double-stranded DNA molecules, sequences may be described herein according to the normal convention of giving only the sequence in the 5' to 3' direction along the nontranscribed strand of DNA (*i.e.,* the strand having a sequence homologous to the mRNA). A "recombinant DNA molecule" is a DNA molecule that has undergone a molecular biological manipulation.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (*see* Sambrook et al., *supra*). The conditions of temperature and ionic strength determine the "stringency" of the hybridization. For preliminary screening for homologous nucleic acids, low stringency hybridization conditions, corresponding to a Tₘ of 55°, can be used, *e.g.,* 5x SSC, 0.1% SDS, 0.25% milk, and no formamide; or 30% formamide, 5x SSC, 0.5% SDS). Moderate stringency hybridization conditions correspond to a higher Tₘ, *e.g.*, 40% formamide, with 5x or 6x SCC. High stringency hybridization conditions correspond to the highest Tₘ, *e.g.*, 50% formamide, 5x or 6x SCC. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tₘ for hybrids of nucleic acids having those sequences. The relative stability (corresponding to higher Tₘ) of nucleic acid hybridizations decreases in the following order: RNA:RNA, DNA:RNA, DNA:DNA. For hybrids of greater than 100 nucleotides in length, equations for calculating Tₘ have been derived (*see* Sambrook et al., *supra,* 9.50-0.51). For hybridization with shorter nucleic acids, *i.e.,* oligonucleotides, the position of mismatches becomes more important, and the length of the oligonucleotide determines its specificity (*see* Sambrook et al., *supra,* 11.7-11.8). Preferably a minimum length for a hybridizable nucleic acid is at least about 10 nucleotides; preferably at least about 15 nucleotides; and more preferably the length is at least about 20 nucleotides.

In a specific embodiment, the term "standard hybridization conditions" refers to a Tₘ of 55°C, and utilizes conditions as set forth above. In a preferred embodiment, the Tₘ is 60°C; in a more preferred embodiment, the Tₘ is 65°C.

"Homologous recombination" refers to the insertion of a foreign DNA sequence of a vector in a chromosome. Preferably, the vector targets a specific chromosomal site for homologous recombination. For specific homologous recombination, the vector will contain sufficiently long regions of homology to sequences of the chromosome to allow complementary binding and incorporation of the vector into the chromosome. Longer regions of homology, and greater degrees of sequence similarity, may increase the efficiency of homologous recombination.

A DNA "coding sequence" is a double-stranded DNA sequence which is transcribed and translated into a polypeptide in a cell *in vitro* or *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (*e.g.,* mammalian) DNA, and even synthetic DNA sequences. If the coding sequence is intended for expression in a eukaryotic cell, a polyadenylation signal and transcription termination sequence will usually be located 3' to the coding sequence.

Transcriptional and translational control sequences are DNA regulatory sequences, such as promoters, enhancers, terminators, and the like, that provide for the expression of a coding sequence in a host cell. In eukaryotic cells, polyadenylation signals are control sequences.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase.

A coding sequence is "under the control" of transcriptional and translational control sequences in a cell when RNA polymerase transcribes the coding sequence into mRNA, which is then trans-RNA spliced and translated into the protein encoded by the coding sequence.

As used herein, the term "homologous" in all its grammatical forms and spelling variations refers to the relationship between proteins that possess a "common evolutionary origin," including proteins from superfamilies (*e.g.*, the immunoglobulin superfamily) and homologous proteins from different species (*e.g.,* myosin light chain, etc.) (Reeck et al., 1987, Cell 50:667). Such proteins (and their encoding genes) have sequence homology, as reflected by their high degree of sequence similarity.

Accordingly, the term "sequence similarity" in all its grammatical forms refers to the degree of identity or correspondence between nucleic acid or amino acid sequences of proteins that may or may not share a common evolutionary origin (*see* Reeck et al., *supra*). However, in common usage and in the instant application, the term "homologous," when modified with an adverb such as "highly," may refer to sequence similarity and not a common evolutionary origin.

In a specific embodiment, two DNA sequences are "substantially homologous" or "substantially similar" when at least about 50% (preferably at least about 75%, and most preferably at least about 90 or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Maniatis et al., *supra;* DNA Cloning, Vols. I & II, *supra;* Nucleic Acid Hybridization, *supra.*

Similarly, in a particular embodiment, two amino acid sequences are "substantially homologous" or "substantially similar" when greater than 30% of the amino acids are identical, or greater than about 60% are similar (functionally identical). Preferably, the similar or homologous sequences are identified by alignment using, for example, the GCG (Genetics Computer Group, Program Manual for the GCG Package, *Version* 7, Madison, Wisconsin) pileup program.

The term "corresponding to" is used herein to refer similar or homologous sequences, whether the exact position is identical or different from the molecule to which the similarity or homology is measured. A nucleic acid or amino acid sequence alignment may include spaces. Thus, the term "corresponding to" refers to the sequence similarity, and not the numbering of the amino acid residues or nucleotide bases.

The present invention contemplates isolation of a gene encoding a frameshift or mRNA decay protein of the invention, including a full length, or naturally occurring form of frameshift or mRNA decay protein, from any eukaryotic, such as yeast, but including animal, particularly mammalian or avian, and more particularly human, or plant source. As used herein, the term "gene" refers to an assembly of nucleotides that encode a polypeptide, and includes cDNA and genomic DNA nucleic acids.

A gene encoding a frameshift or mRNA decay protein, whether genomic DNA or cDNA, can be isolated from any source, particularly from a human cDNA or genomic library. Methods for obtaining such genes are well known in the art, as described above (*see, e.g.,* Sambrook et al., 1989, *supra*). A specific example of isolation of such a gene is shown in the Example appended hereto.

Accordingly, any eukaryotic cell potentially can serve as the nucleic acid source for the molecular cloning of a gene encoding a frameshift or mRNA decay protein. The DNA may be obtained by standard procedures known in the art from cloned DNA (*e.g.,* a DNA "library"), by chemical synthesis, by cDNA cloning, or by the cloning of genomic DNA, or fragments thereof, purified from the desired cell (See, for example, Sambrook et al., 1989, *supra;* Glover, D.M. (ed.), 1985, DNA Cloning: A Practical Approach, MRL Press, Ltd., Oxford, U.K. Vol. I, II). Clones derived from genomic DNA may contain regulatory and intron DNA regions in addition to coding regions; clones derived from cDNA will not contain intron sequences. Whatever the source, the gene should be molecularly cloned into a suitable vector for propagation of the gene.

In the molecular cloning of the gene from genomic DNA, DNA fragments are generated, some of which will encode the desired gene. The DNA may be cleaved at specific sites using various restriction enzymes. Alternatively, one may use DNAse in the presence of manganese to fragment the DNA, or the DNA can be physically sheared, as for example, by sonication. The linear DNA fragments can then be separated according to size by standard techniques, including but not limited to, agarose and polyacrylamide gel electrophoresis and column chromatography.

Once the DNA fragments are generated, identification of the specific DNA fragment containing the desired gene may be accomplished in a number of ways. For example, if an amount of a portion of the gene or its specific RNA, or a fragment thereof, is available and can be purified and labeled, the generated DNA fragments may be screened by nucleic acid hybridization to the labeled probe (Benton and Davis, 1977, Science 196:180; Grunstein and Hogness, 1975, Proc. Natl. Acad. Sci. U.S.A. 72:3961). For example, a set of oligonucleotides corresponding to the partial amino acid sequence information obtained for the frameshift or mRNA decay protein can be prepared and used as probes for DNA encoding frameshift or mRNA decay protein. Preferably, a fragment is selected that is highly unique to frameshift or mRNA decay protein of the invention. Those DNA fragments with substantial homology to the probe will hybridize. As noted above, the greater the degree of homology, the more stringent hybridization conditions can be used. Moreover, because the frameshift and mRNA decay proteins are fundamental to translation, they are highly conserved, *e.g.*, from yeast to human. Thus, identification of such a protein in yeast or another eukaryotic cell readily leads to obtaining such a protein from human or other animal cDNA libraries.

Further selection can be carried out on the basis of the properties of the gene, *e.g*., if the gene encodes a protein product having the isoelectric, electrophoretic, amino acid composition, or partial amino acid sequence of frameshift or mRNA decay protein as disclosed herein. Thus, the presence of the gene may be detected by assays based on the physical, chemical, or immunological properties of its expressed product. For example, cDNA clones, or DNA clones which hybrid-select the proper mRNAs, can be selected which produce a protein that. *e.g.,* has similar or identical electrophoretic migration, isoelectric focusing or non-equilibrium pH gel electrophoresis is behavior, proteolytic digestion maps, or antigenic properties as known for frameshift or mRNA decay protein.

A gene can also be identified by mRNA selection. *i.e.,* by nucleic acid hybridization followed by *in vitro* translation. In this procedure, nucleotide fragments are used to isolate complementary mRNAs by hybridization. Such DNA fragments may represent available, purified DNA, including DNA from another species, or may be synthetic oligonucleotides designed from the partial amino acid sequence information. Immunoprecipitation analysis or functional assays of the *in vitro* translation products of the products of the isolated mRNAs identifies the mRNA and, therefore, the complementary DNA fragments, that contain the desired sequences. In addition, specific mRNAs may be selected by adsorption of polysomes isolated from cells to immobilized antibodies specifically directed against frameshift or mRNA decay protein.

A radiolabeled cDNA can be synthesized using the selected mRNA (from the adsorbed polysomes) as a template. The radiolabeled mRNA or cDNA may then be used as a probe to identify homologous DNA fragments from among other genomic DNA fragments.

The present application also describes cloning vectors containing genes encoding analogs and derivatives of frameshift or mRNA decay protein of the invention, that have the same or homologous functional activity as frameshift or mRNA decay protein, and homologs thereof from other species. The production and use of derivatives and analogs related to frameshift or mRNA decay protein are within the scope of the present invention. In a specific embodiment, the derivative or analog is functionally active, *i.e.,* capable of exhibiting one or more functional activities associated with a full-length, wild-type frameshift or mRNA decay protein of the invention.

Frameshift or mRNA decay protein derivatives can be made by altering encoding nucleic acid sequences by substitutions, additions or deletions that provide for functionally equivalent molecules. Preferably, derivatives are made that have enhanced or increased functional activity relative to native frameshift or mRNA decay protein.

Due to the degeneracy of nucleotide coding sequences, other DNA sequences which encode substantially the same amino acid sequence as a frameshift of mRNA decay gene may be used in the practice of the present invention. These include but are not limited to allelic genes, homologous genes from other species, and nucleotide sequences comprising all or portions of such genes which are altered by the substitution of different codons that encode the same amino acid residue within the sequence, thus producing a silent change. Likewise, the frameshift or mRNA decay protein derivatives of the invention include, but are not limited to, those containing, as a primary amino acid sequence, all or part of the amino acid sequence of a frameshift or mRNA decay protein, including altered sequences in which functionally equivalent amino acid residues are substituted for residues within the sequence resulting in a conservative amino acid substitution. For example, one or more amino acid residues within the sequence can be substituted by another amino acid of a similar polarity, which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Such alterations will not be expected to affect apparent molecular weight as determined by polyacrylamide gel electrophoresis, or isoelectric point.

The genes encoding frameshift or mRNA decay protein derivatives and analogs can be produced by various methods known in the art. The manipulations which result in their production can occur at the gene or protein level. For example, the cloned frameshift or mRNA decay protein gene sequence can be modified by any of numerous strategies known in the art (Sambrook et al., 1989, *supra*). The sequence can be cleaved at appropriate sites with restriction endonuclease(s), followed by further enzymatic modification if desired, isolated, and ligated *in vitro.* In the production of the gene encoding a derivative or analog of frameshift or mRNA decay protein, care should be taken to ensure that the modified gene remains within the same translational reading frame as the frameshift or mRNA decay protein gene, uninterrupted by translational stop signals, in the gene region where the desired activity is encoded.

Additionally, the frameshift or mRNA decay protein-encoding nucleic acid sequence can be mutated *in vitro* or *in vivo,* to create and/or destroy translation, initiation, and/or termination sequences, or to create variations in coding regions and/or form new restriction endonuclease sites or destroy preexisting ones, to facilitate further *in vitro* modification. Preferably, such mutations enhance the functional activity of the mutated frameshift or mRNA decay protein gene product. Any technique for mutagenesis known in the art can be used, including but not limited to, *in vitro* site-directed mutagenesis (Hutchinson, C., et al., 1978, J. Biol. Chem. 253:6551; Zoller and Smith, 1984, DNA 3:479-488; Oliphant et al., 1986, Gene 44:177; Hutchinson et al., 1986, Proc. Natl. Acad. Sci. U.S.A. 83:710), use of TAB^{®} linkers (Pharmacia), etc. PCR techniques are preferred for site directed mutagenesis (see Higuchi, 1989, "Using PCR to Engineer DNA", in *PCR Technology: Principles and Applications for DNA Amplification, H.* Erlich, ed., Stockton Press, Chapter 6, pp. 61-70).

The identified and isolated gene can then be inserted into an appropriate cloning vector. A large number of vector-host systems known in the art may be used. Possible vectors include, but are not limited to, plasmids or modified viruses, but the vector system must be compatible with the host cell used. Examples of vectors include, but are not limited to, *E. coli,* bacteriophages such as lambda derivatives, or plasmids such as pBR322 derivatives or pUC plasmid derivatives, *e.g.,* pGEX vectors, pmal-c, pFLAG, etc. The insertion into a cloning vector can, for example, be accomplished by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. Recombinant molecules can be introduced into host cells via transformation, transfection, infection, electroporation, etc., so that many copies of the gene are generated. Preferably, the cloned gene is contained on a shuttle vector plasmid, which provides for expansion in a cloning cell, *e.g., E. coli,* and facile purification for subsequent insertion into an appropriate expression cell line, if such is desired. For example, a shuttle vector, which is a vector that can replicate in more than one type of organism, can be prepared for replication in both *E*. *coli* and *Saccharomyces cerevisiae* by linking sequences from an *E. coli* plasmid with sequences form the yeast 2µ plasmid.

In an alternative method, the desired gene may be identified and isolated after insertion into a suitable cloning vector in a "shot gun" approach. Enrichment for the desired gene, for example, by size fractionation, can be done before insertion into the cloning vector.

### Expression of Frameshift or mRNA Decay Proteins

The nucleotide sequence coding for frameshift or mRNA decay protein, or a functionally active derivative, including a chimeric protein, thereof, can be inserted into an appropriate expression vector, *i.e.,* a vector which contains the necessary elements for the transcription and translation of the inserted protein-coding sequence. Such elements are termed herein a "promoter." Thus, the nucleic acid encoding frameshift or mRNA decay protein of the invention is operationally associated with a promoter in an expression vector of the invention. Both cDNA and genomic sequences can be cloned and expressed under control of such regulatory sequences. An expression vector also preferably includes a replication origin.

The necessary transcriptional and translational signals can be provided on a recombinant expression vector, or they may be supplied by the native gene encoding frameshift or mRNA decay protein and/or its flanking regions.

Potential host-vector systems include but are not limited to mammalian cell systems infected with virus (*e.g.,* vaccinia virus, adenovirus, etc.); insect cell systems infected with virus (*e.g.,* baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

A recombinant frameshift or mRNA decay protein, or functional fragment, derivative, chimeric construct, or analog thereof, may be expressed chromosomally, after integration of the coding sequence by recombination. In this regard. any of a number of amplification systems may be used to achieve high levels of stable gene expression (*See* Sambrook et al., 1989, *supra*).

The cell into which the recombinant vector comprising the nucleic acid encoding frameshift or mRNA decay protein is cultured in an appropriate cell culture medium under conditions that provide for expression of frameshift or mRNA decay protein by the cell.

Any of the methods previously described for the insertion of DNA fragments into a cloning vector may be used to construct expression vectors containing a gene consisting of appropriate transcriptional/translational control signals and the protein coding sequences. These methods may include *in vitro* recombinant DNA and synthetic techniques and *in vivo* recombination (genetic recombination).

Expression of frameshift or mRNA decay protein may be controlled by any promoter/enhancer element known in the art, but these regulatory elements must be functional in the host selected for expression. Promoters which may be used to control frameshift or mRNA decay protein gene expression include, but are not limited to, the SV40 early promoter region (Benoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); prokaryotic expression vectors such as the β-lactamase promoter (Villa-Kamaroff, et al., 1978, Proc. Natl. Acad. Sci. U.S.A. 75:3727-3731), or the *tac* promoter (DeBoer, et al., 1983, Proc. Natl. Acad. Sci. U.S.A. 80:21-25); see also "Useful proteins from recombinant bacteria" in Scientific American, 1980, 242:74-94; promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al. , 1984, Cell 38:647-658; Adames et al., 1985. Nature 318:533-538: Alexander et al., 1987, Mol. Cell. Biol. 7:1436-1444), mouse mammary rumor virus control region which is active in testicular, breast, lymphoid and mast cells (Leder et al., 1986. Cell 45:485-495), albumin gene control region which is active in liver (Pinkert et al., 1987. Genes and Devel. 1:268-276), alpha-fetoprotein gene control region which is active in liver (Krumlauf et al., 1985. Mol. Cell. Biol. 5:1639-1648: Hammer et al., 1987, Science 235:53-58), alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94), myelin basic protein gene control region which is active in oligodendrocyte cells in the brain (Readhead et al., 1987. Cell 48:703-712), myosin light chain-2 gene control region which is active in skeletal muscle (Sani. 1985. Nature 314:283-286), and gonadotropic releasing hormone gene control region which is active in the hypothalamus (Mason et al., 1986. Science 234:1372-1378).

Expression vectors containing a nucleic acid encoding a frameshift or mRNA decay protein can be identified by four general approaches: (a) PCR amplification of the desired plasmid DNA or specific mRNA. (b) nucleic acid hybridization, (c) presence or absence of selection marker gene functions, and (d) expression of inserted sequences. In the first approach, the nucleic acids can be amplified by PCR to provide for detection of the amplified product. In the second approach, the presence of a foreign gene inserted in an expression vector can be detected by nucleic acid hybridization using probes comprising sequences that are homologous to an inserted marker gene. In the third approach, the recombinant vector/host system can be identified and selected based upon the presence or absence of certain "selection marker" gene functions (*e.g.,* β-galactosidase activity, thymidine kinase activity, resistance to antibiotics, transformation phenotype, occlusion body formation in baculovirus, etc.) caused by the insertion of foreign genes in the vector. In another example, if the nucleic acid encoding frameshift or mRNA decay protein is inserted within the "selection marker" gene sequence of the vector, recombinants containing the frameshift or mRNA decay protein insert can be identified by the absence of the frameshift or mRNA decay protein gene function. In the fourth approach, recombinant expression vectors can be identified by assaying for the activity, biochemical, or immunological characteristics of the gene product expressed by the recombinant, provided that the expressed protein assumes a functionally active conformation.

Once a particular recombinant DNA molecule is identified and isolated, several methods known in the art may be used to propagate it. Once a suitable host system and growth conditions are established, recombinant expression vectors can be propagated and prepared in quantity. As previously explained, the expression vectors which can be used include, but are not limited to, the following vectors or their derivatives: human or animal viruses such as vaccinia virus or adenovirus; insect viruses such as baculovirus; yeast vectors; bacteriophage vectors (*e.g.,* lambda), and plasmid and cosmid DNA vectors, to name but a few.

In a preferred embodiment, the frameshift protein is expressed in a cell that is virally infected, and the ability of an agent to inhibit or eliminate viral killing can be evaluated.

In another preferred embodiment, the mRNA decay protein is co-expressed in a cell with an aberrant mRNA transcript, such as a nonsense transcript, and antisense transcript, or a short transcript, and the ability of an agent to increase stability of the aberrant transcript can be evaluated.

Vectors are introduced into the desired host cells by methods known in the art, *e.g.,* transfection, electroporation, microinjection, transduction, cell fusion, DEAE dextran, calcium phosphate precipitation, lipofection (lysosome fusion), use of a gene gun, or a DNA vector transporter (see, *e.g.,* Wu et al., 1992, J. Biol. Chem. 267:963-967; Wu and Wu, 1988, J. Biol. Chem. 263:14621-14624; Hartmut et al., Canadian Patent Application No. 2,012,311, filed March 15, 1990).

### Screening for Agents that Affect Protein Activity

Any screening technique known in the art can be used to screen for agents that affect the function of a frameshift protein or a mRNA decay protein. The present invention contemplates screens for small molecule ligands.

Knowledge of the primary sequence of a frameshift or mRNA decay protein, and the similarity of that sequence with proteins of known function, can provide an initial clue as to agents that are likely to affect protein activity. Identification and screening of such agents is further facilitated by determining structural features of the protein, *e.g.*, using X-ray crystallography, neutron diffraction, nuclear magnetic resonance spectrometry, and other techniques for structure determination. These techniques provide for the rational design or identification of agonists and antagonists.

In another aspect, synthetic libraries (Needels et al., 1993. "Generation and screening of an oligonucleotide encoded synthetic peptide library." Proc. Natl. Acad. Sci. USA 90:10700-4: Lam et al.. U.S. Patent No. 5,382.513. issued January 17. 1995: Lam et al., International Patent Publication No. WO 92/00252; and Ohlmeyer et al.. 1993, Proc. Natl. Acad. Sci. USA 90:10922-10926, and the like can be used to screen for agents according to the present invention.

The screening can be performed with recombinant cells that express the frameshift protein or mRNA decay protein, or alternatively, with the purified protein. For example, the ability of labeled protein to bind to a molecule in a combinatorial library can be used as a screening assay, as described in the foregoing references.

### Antisense RNA and Ribozymes

Antisense nucleotides and ribozymes that incorporate the design strategies put forth herein, or that take advantage of the discovery that the aberrant mRNA decay pathway may shorten the half-life of antisense RNA or ribozymes so as to render them ineffective for the desired purpose. *i.e.,* to interfere with the expression of a gene at the translational level. This approach utilizes antisense nucleic acid and ribozymes to block translation of a specific mRNA. either by masking that mRNA with an antisense nucleic acid or cleaving it with a ribozyme.

Antisense nucleic acids are DNA or RNA molecules that are complementary to at least a portion of a specific mRNA molecule (*see* Marcus-Sekura, 1988. Anal. Biochem. 172:298). In the cell, they hybridize to that mRNA, forming a double stranded molecule. The cell does not translate an mRNA in this double-stranded form. Therefore, antisense nucleic acids interfere with the expression of mRNA into protein. Oligomers of about fifteen nucleotides and molecules that hybridize to the AUG initiation codon will be particularly efficient, since they are easy to synthesize and are likely to pose fewer problems than larger molecules when introducing them into organ cells. Antisense methods have been used to inhibit the expression of many genes *in vitro* (Marcus-Sekura, 1988, *supra*; Hambor et al., 1988, J. Exp. Med. 168:1237).

Ribozymes are RNA molecules possessing the ability to specifically cleave other single stranded RNA molecules in a manner somewhat analogous to DNA restriction endonucleases. Ribozymes were discovered from the observation that certain mRNAs have the ability to excise their own introns. By modifying the nucleotide sequence of these RNAs, researchers have been able to engineer molecules that recognize specific nucleotide sequences in an RNA molecule and cleave it (Cech, 1988, J. Am. Med. Assoc. 260:3030). Because they are sequence-specific, only mRNAs with particular sequences are inactivated.

Investigators have identified two types of ribozymes, *Tetrahymena*-type and "hammerhead"-type. *Tetrahymena*-type ribozymes recognize four-base sequences, while "hammerhead "-type recognize eleven- to eighteen-base sequences. The longer the recognition sequence, the more likely it is to occur exclusively in the target MRNA species. Therefore, hammerhead-type ribozymes are preferable to *Tetrahymena*-type ribozymes for inactivating a specific mRNA species, and eighteen base recognition sequences are preferable to shorter recognition sequences.

### Antiviral Therapy

In yet a further embodiment, the present invention provides the means to treat viral infections by providing agents that modulate frameshift efficiency, and thus directly affect viral replication or assembly of viral particles. The efficiency of -1 ribosomal frameshifting in the naturally occurring L-A slippery site is 1.8% - 2.0% (16). Changing the efficiency of -1 ribosomal frameshifting using the drugs or molecular biological reagents of the present invention changes the ratio of Gag to Gag-pol that is synthesized. This in turn affects viral particle assembly and RNA packaging, affecting the ability of the cell to propagate the virus. In particular, changing the slippery site sequence affects the efficiency of -1 ribosomal frameshifting. The efficiency of -1 ribosomal frameshifting can also be affected by introducing mutated cellular gene products that interact with the translational apparatus, particularly the peptidyl transfer center. Both molecular biological and genetic methods have been used to demonstrate that the 1.9% efficiency of ribosomal frameshifting yields the optimum ratio of structural to enzymatic proteins. Changing frameshifting efficiencies more than 2-3 fold results in loss of the M₁ satellite virus, whether the virus is supported by L-A cDNA clones containing altered slippery sites, or by the wild-type L-A virus in mutant host cells. Even slight changes in -1 ribosomal frameshifting efficiencies significantly lower M₁ copy numbers.

The present invention advantageously provides drugs and methods to identify drugs for use in antiviral (or nonsense suppression) therapy of viruses that use the basic -1 ribosomal frameshifting mechanism, which includes four large families of animal viruses and three large families of plant viruses.

For example, almost all retroviruses use -1 ribosomal frameshifting, including lentiviruses (immunodeficiency viruses) such as HIV-1 and HIV-2, SIV, FIV, BIV, Visna virus, Arthritis-encephalitis virus, and equine infectious anemia virus; spumaviruses (the foamy viruses), such as human foamy virus and other mammalian foamy viruses; the T cell lymphotrophic viruses, such as HTLV-I, HTLV-II, STLVs, and BLV; avian leukosis viruses, such as leukemia and sarcoma viruses of many birds, including commercial poultry; type B retroviruses, including mouse mammary tumor virus; and type D retroviruses, such as Mason-Pfizer monkey virus and ovine pulmonary adenocarcinoma virus. In addition, many coronaviruses use the -1 frameshifting, including human coronaviruses, such as 229-E, OC43; animal coronaviruses, such as calf coronavirus, transmissible gastroenteritis virus of swine, hemagglutinating encephalomyelitis virus of swine, and porcine epidemic diarrhea virus; canine coronavirus; feline infectious peritonitis virus and feline enteric coronavirus; infectious bronchitis virus of fowl and turkey bluecomb virus; mouse hepatitis virus, rat coronavirus, and rabbit coronavirus. Similarly, torovirus (a type of coronavirus) is implicated, such as human toroviruses associated with enteric and respiratory diseases; breda virus of calves and bovine respiratory virus; berne virus of horses; porcine torovirus; feline torovirus. Another coronavirus is the arterivirus, which includes simian hemorrhagic fever virus, equine arteritis virus, Lelystad virus (swine), VR2332 virus (swine), and lactate dehydrogenase-elevating virus (rodents). Other animal viruses are paramyxoviruses, such as human -1 ribosomal frameshifting reported in measles, and astroviruses, such as human astroviruses 1-5, and bovine, ovine, porcine, canine, and duck astroviruses.

The plant viruses that involve a -1 frameshifting mechanism include tetraviruses, such as sobemoviruses (*e.g.,* southern bean mosaic virus, cocksfoot mettle virus), leuteoviruses (*e.g*., barley yellowswarf virus, beet western yellows virus, and potato leaf roll virus), enamoviruses (*e.g.*, pea mosaic virus), and umbraviruses (*e.g*., carrot mottle virus); tombusviruses, such as tombusvirus (*e.g*., tomato bushy stunt virus), carmovirus (*e.g.,* carnation mottle virus), necrovirus (*e.g.,* tobacco necrosis virus); dianthoviruses (*e.g.,* red clover necrotic mosaic virus), and machiomovirus (*e.g.,* maize chlorotic mottle virus).

In addition, totiviruses, such as L-A and L-BC (yeast) and other fungal viruses, giradia lamblia virus (intestinal parasite), triconella vaginell virus (human parasite), leishmania brasiliensis virus (human parasite), and other protozoan viruses are -1 frameshift viruses.

### Suppression of Pathological Nonsense Mutations

The nonsense-mediated mRNA decay pathway regulates decay of transcripts that have acquired nonsense codons through a mutagenic event. As such, this pathway is clearly implicated in regulation of gene expression. More importantly, modulating this pathway can overcome lack of gene expression due to a nonsense mutation. Also, antisense RNAs may be substrates for the nonsense-mediated mRNA decay pathway, leading to a decrease in their cellular concentration and a reduction of their ability to inhibit gene expression. Mutations, or drugs, that inactivate this pathway may increase the abundance of "nonsense" RNAs, resulting in enhanced efficiency with which antisense RNAs inhibit gene expression.

Modulation of the mRNA decay pathway also has significant potential for treating certain conditions. Nonsense mutations are found in many genes that results in diseases. A list of such disease (and the nonsense containing allele) follows: nonspherocytic hemolytic anemia (*TPI*), β-thalassemia (β-*GLOBIN*), hypercholesterolemia (*LDL RECEPTOR Lebanese allele*), pulmonary emphysema (α*-1 ANTITRYPSIN*), adrenal hyperplasia (*STEROID-21 HYDROLASE (CYP21)*), apolipoprotein C-II deficiency (*APOLIPOPROTEIN C-II*_{*padova*}), hemophilia B (*FACTOR IX*_{*PORTLAND*}), Bernard-Soulier syndrome (*GLYCOPROTEIN 1B*α), fructose intolerance (*ALDOLASE B*), insulin resistance (*INSULIN RECEPTOR*), maple syrup urine disease (α-*KETOACID DEHYDROGENASE*), thrombosis (*PROTEIN S*), goiter and hypothyroidism (*THYROGLOBULIN*), chronic granulomatous (*CYTOCHROME B558*), Sandhoff disease (*HEBX*), vonWillebrand disease type III (*von WILLEBRAND FACTOR),* gyrate atrophy (*ORNITHINE AMINOTRANSFERASE*). 1.25-dihydroxyvitamin D3 resistant rickets (*VITAMIN D RECEPTOR*), spherocytosis (*ERYTHROCYTE BAND 3*), cystic fibrosis (*CFTR*), and spherocytosis (*ERYTHROID ANKYRIN*).

### Gene Therapy and Transgenic Vectors

A gene encoding a mutant ribosomal frameshifting or mRNA decay protein or polypeptide domain fragment thereof, or a gene encoding an antisense or ribozyme specific for a wildtype ribosomal frameshifting or mRNA decay protein, is introduced *in vivo* in a viral vector. Such vectors include an attenuated or defective DNA virus, such as but not limited to herpes simplex virus (HSV), papillomavirus. Epstein Barr virus (EBV), adenovirus, adeno-associated virus (AAV), and the like. Defective viruses, which entirely or almost entirely lack viral genes, are preferred. Defective virus is not infective after introduction into a cell. Use of defective viral vectors allows for administration to cells in a specific, localized area, without concern that the vector can infect other cells. Thus, adipose tissue can be specifically targeted. Examples of particular vectors include, but are not limited to, a defective herpes virus 1 (HSV1) vector [Kaplitt et al.. *Molec. Cell. Neurosci.* **2**:320-330 (1991)], an attenuated adenovirus vector, such as the vector described by Strarford-Perricaudet et al. [*J. Clin. Invest.* **90**:626-630 (1992)], and a defective adeno-associted virus vector [Samulski et al.. *J. Virol.* **61**:3096-3101 (1987): Samulski et al., *J. Virol.* **63**:3822-3828 (1989)].

Preferably, for *in vivo* administration, an appropriate immunosuppressive treatment is employed in conjunction with the viral vector, *e.g.,* adenovirus vector, to avoid immuno-deactivation of the viral vector and transfected cells. For example, immunosuppressive cytokines, such as interleukin-12 (IL-12), interferon-γ (IFN-γ), or anti-CD4 antibody, can be administered to block humoral or cellular immune responses to the viral vectors [*see, e.g.,* Wilson. *Nature Medicine* (1995)]. In addition, it is advantageous to employ a viral vector that is engineered to express a minimal number of antigens.

In another embodiment the gene can be introduced in a retroviral vector, *e.g.,* as described in Anderson et al., U.S. Patent No. 5,399,346; Mann et al., 1983. Cell 33:153; Temin et al., U.S. Patent No. 4,650,764; Temin et al., U.S. Patent No. 4,980,289; Markowitz et al., 1988. J. Virol. 62:1120: Temin et al., U.S. Patent No. 5.124.263: International Patent Publication No. WO 95/07358. published March 16, 1995. by Dougherty et al.; and Kuo et al., 1993, Blood 82:845.

Targeted gene delivery is described in International Patent Publication WO 95/28494, published October 1995.

Alternatively, the vector can be introduced *in vivo* by lipofection. For the past decade, there has been increasing use of liposomes for encapsulation and transfection of nucleic acids *in vitro.* Synthetic cationic lipids designed to limit the difficulties and dangers encountered with liposome mediated transfection can be used to prepare liposomes for *in vivo* transfection of a gene encoding a marker [Felgner, et. al., *Proc. Natl. Acad Sci. U.S.A.* **84:**7413-7417 (1987); *see* Mackey, et al.. *Proc. Natl. Acad. Sci. U.S.A.* **85:**8027-8031 (1988)]. The use of cationic lipids may promote encapsulation of negatively charged nucleic acids, and also promote fusion with negatively charged cell membranes [Felgner and Ringold. *Science* **337:**387-388 (1989)]. The use of lipofection to introduce exogenous genes into the specific organs *in vivo* has certain practical advantages. Molecular targeting of liposomes to specific cells represents one area of benefit. It is clear that directing transfection to particular cell types would be particularly advantageous in a tissue with cellular heterogeneity, such as pancrease, liver, kidney, and the-brain. Lipids may be chemically coupled to other molecules for the purpose of targeting [*see* Mackey, et. al., *supra*]. Targeted peptides, *e.g.,* hormones or neurotransmitters, and proteins such as antibodies, or non-peptide molecules could be coupled to liposomes chemically.

It is also possible to introduce the vector *in vivo* as a naked DNA plasmid. Naked DNA vectors for gene therapy can be introduced into the desired host cells by methods known in the art, *e.g.,* transfection, electroporation, microinjection, transduction, cell fusion. DEAE dextran, calcium phosphate precipitation, use of a gene gun, or use of a DNA vector transporter [*see. e.g.,* Wu et al., *J. Biol. Chem.* **267**:963-967 (1992); Wu and Wu, *J. Biol. Chem.* **263**:14621-14624 (1988); Hartmut et al., Canadian Patent Application No. 2,012,311, filed March 15, 1990].

Moreover, it is possible to co-express a gene product that modulates activity at the peptidyl transferase center and a therapeutic heterologous antisense or ribozyme gene under control of the specific DNA recognition sequence by providing a gene therapy expression vector comprising both a gene coding for a modulator of a peptidyl transferase center (including but not limited to a gene for a mutant frameshift or mRNA decay protein, or an antisense RNA or ribozyme specific for mRNA encoding such a protein) with a gene for an unrelated antisense nucleic acid or ribozyme under coordinated expression control. In one example, these elements are provided on separate vectors; alternatively these elements may be provided in a single expression vector.

The present invention may be better understood by reference to the following non-limiting Examples, which are provided as exemplary of the invention.

### EXAMPLE 1: mof4-1. an Allele of the UPF1/IFS2 Gene, Affects mRNA Turnover and -1 Ribosomal Frameshifting Efficiency

mRNA degradation is an important control point in the regulation of gene expression and has been shown to be linked to the process of translation. One clear example of this linkage is the observation that nonsense mutations accelerate the degradation of mRNAs. This Example demonstrates that a subset of the *mof* alleles (maintenance of *f*rame) in yeast, which were isolated as chromosomal mutations that increased the frameshifting efficiency at the L-A virus frameshift site and caused loss of the L-A satellite virus M₁, also affect the nonsense-mediated mRNA decay pathway. The levels of nonsense-containing mRNAs were elevated in cells harboring the *mof4-1* alleles. Furthermore, *mof4-1* is allelic to *UPF1,* which has been demonstrated to be involved in the nonsense-mediated mRNA decay pathway. Although cells harboring the *mof4-1* allele lose the M₁ virus, the other f alleles (i.e., *upf1, upf2* and *upf3*) involved in nonsense-mediated mRNA decay maintain M₁. The *ifs1* and *ifs2* alleles previously identified as mutations that enhance frameshifting at the -1 ribosomal frameshift signal from the mouse mannary tumor virus were shown to be allelic to the *UPF2* and *UPF1* genes, respectively, and both *ifs* strains maintained M₁. The *mof4-1* strain is more sensitive to the aminoglycoside paromomycin than a *upf1Δ* strain, and frameshifting efficiency increases in a *mof4-1* strain grown in the presence of paromomycin. These results indicate that the Upf1p has a dual function in both translation and mRNA turnover.

### Materials and Methods

*Strain and media:* The strains of *Saccharomyces cerevisiae* used are listed in Table 1. YPAD, YPG, SD, synthetic complete medium and 4.7 MB plates for testing the killer phenotype were as previously reported [Dinman *et al., Genetics* **136:**75-86 (1994)].

**Table 1. Strains used in this study**

| Cells | Genotype | reference |
|---|---|---|
| Y52- | MATα rpb1-1 his4-519 ura3-52 UPFl::hisG | a |
| YGC106 | MATa ade2,3 ura3 leu2 his7 can1 sap3 UPF1::hisG | this study |
| PLY36 | MATα his4-38 suf1-1 ura3-52 met14 upf1-2 | b |
| PLY136 | MATa his4-38 suf1-1 ura3-52 met14 trp1-1 upf1-2 | b |
| YGC112 | MATa his4-38 suf1-1 ura3-52 met14 upf1-2 trp1-1 leu2-3 UPF2::URA3 | a |
| PLY139 | MATa his4-38 suf1-1 ura3-52 upf3-1 | b |
| PLY133 | MATα his4-38 suf1-1 ura3-52 met14 upf4-2 | b |
| 2907 | MATa his3-Δ200 leu2 trp1-Δ901 ura3-52 ade2-10 K⁻ | c |
| JD61 | MATa his4-644 lsu2-1::pJD85 K⁻ | d |
| JD75-1A | MATa leu2-1::pJD85 his4-644 ura3 ade2 mof1-1 K⁻ | d |
| JD65-5C | MATα leu2-1::pJD85 his3,4-644 ura3 ade2 trp1-Δ901 mof3-1 K⁻ | d |
| JD474-2C | MATα leu2-1::pJD85 ura 3 trp1-Δ901 mof4-1 K⁻ | d |
| JD474-3D | MATa leu2-1::pJD85 ura3 his4 mof4-1 K⁻ | e |
| JD469-2C | MATα leu2-1::pJD85 ura3 trp1-Δ901 ade2 mof6-1 K⁻ | d |
| JD471-1A | MATα leu2-1::pJD85 ura3 mof7-1 K⁻ | d |
| JD472-1A | MATα leu2-1::pJD85 ura3 mof8-1 K⁻ | d |
| JD472-9B | MATa leu2-1::pJD85 ura3 his3,4 mof2-1 K⁻ | d |
| JD742-2D | MATα leu2-1::pJD85 ura 3 his3,4 L-AHN M₁ K⁺ | this study |
| cJD744-2C | MATα leu2-1::pJD85 ade2 his3,4 trp1 ura3 mof5-1 K⁻ | d |
| cross JD830 | JD742-2D x JD474-3D | this study |
| 1074 | MATa leu 1 L-AHN M₁ kar1-1 K⁺ | d |
| JD474-5A | MATa leu2-1::pJD85 ura3 his4 trp1 ade2 mof4-1 K⁻ | d |
| 3164 | MATa kar1-1 arg1 L-AHN M₁ K⁺ | d |
| 3165 | MATa karl-1 arg1 thr(1,x) L-AHN M₁ K⁺ | d |
| 5X47 | MATa/MATα his1/ + trp1/ + ura3/ + K⁻R⁻ | e |
| L5*a* | MATα cup1Δ::ura3 ura3-52 his3-Δ200 ade2 lys2 trp1 leu2 K⁻ | f |
| ifs1 | MATα cup1Δ::ura3 ura3-52 his3-Δ200 ade2 lys2 trp1 leu2 ifs1-2 K⁻ | f |
| ifs2 | MATα cup1Δ::ura3 ura3-52 his3-Δ200 ade2 lys2 trp1 leu2 ifs2-1 K⁻ | f |

| | | |
|---|---|---|
| a. Cui et al., *Genes & Dev.,* **9:**437-454 (1995). b. Leeds et al., *Genes & Dev.,* **5:**2303-14 (1991). c. Dinman et al., *Proc. Natl. Acad. Sci. USA,* **88:**174-178 (1991). d. Dinman and Wickner, *Genetics,* **136:**75-86 (1994). e. Dinman and Wickner, *J. Virol.,* **66:**3669-76 (1992). f. Lee et al., *Proc. Natl. Acad. Sci. USA,* **92:**6587 (1995). | | |

*Genetic methods and assays.* Transformation of yeast and *E. coli* were performed as described previously [Hagan *et al., Mol. Cell. Biol.,* **15:**809-823 (1995); Cui *et al., Genes & Dev.,* **7:**1737-1754 (1995); He and Jacobson, *Genes & Dev.,* **9:**437-454 (1995)]. Cells were cured of L-A virus by streaking for single colonies at 39°C and loss of L-A was confirmed by agarose gel analysis. Generation of rho° cells, cytoductions and the killer test were performed as previously describe [Dinman and Wickner, *J. Virol.* **66:**3669-3676 (1992)]. Genetic crosses, sporulation and tetrad analysis, β-galactosidase assays and the killer test were performed as described [Dinman and Wickner, *Genetics,* **136:**75-86 (1994)]. dsRNA was prepared as described [Fried and Fink, *Proc. Natl. Acad. Sci. USA,* **75**:4224-4228 (1978)] and was analyzed by electrophoresis through 1.2% agarose gels. Testing for paromomycin sensitivity of the various strains was performed as described [Cui *et al.,* (1995) *supra*].

*Plasmids construction.* The plasmids pJD107 and pJD108 used for β-galactosidase assay were derived from pF8 and pT125 respectively [Dinman *et al., Proc. Natl. Acad. Sci. USA,* **88**:174-178 (1991)]. In pJD107, the 4.9 kb. Hind III fragment from pF8 was ligated into Hind III digested pRS426 [Christianson *et al., Gene,* **110**:119-122 (1992)] and contains the PGK1 promoter, a translational start site, followed by a 218 bp cDNA fragment of L-A containing the -1 ribosomal frameshift signal. This is followed by the *lacZ* gene, which is in the -1 frame with respect to the start site. pJD108 contains the 4.7 kb Hind III fragment of pT125 cloned into the HingIII site of pRS426, and the *lacZ* gene is in the O-frame without any intervening sequence. pYCp33UPF1 and pYCp33UPF2 were constructed as described before [Cui *et al.,* (1995) *supra*]. The plasmids pmof4AE, pmof4AB, and pmof4BE used to clone the *mof4-1* allele were constructed as follows: the 1.47 kb Asp718- EcoRI fragment or the 2.6 kb Asp718-BamHI fragment from pYCp33UPF1, containing the *UPF1* gene, was deleted and replaced with the corresponding fragments of the *mof4-1* allele that were isolated by PCR (see below). pmof4BE was cloned by inserting the 4.2kb EcoRI-BamHI DNA fragment from *mof4-1* into pyCplac33. Since the pYCp33UPF1 contains more than one BstXI site, pmof4XAE and pmof4XBE were constructed by two steps. A 978 bp BstXI- Asp718 DNA fragment from pPUC-UPF1 was replaced with a BstXI-Asp718 DNA fragment from pmo4AE and pmof4BE, respectively, forming pPUCmof4XAE and pPUCmof4XBE. The 4.2kb BamHI-EcoRI fragments from these two plasmids were cloned into pYCplac33.

*Identification of the mof4-1 mutation.* A PCR strategy was used to identify the *mof4-1* allele. The primers used for PCR DNA fragments from the *UPF1* gene were: Primer-1, 5'-CCGGAATTCATGAACGGGAAA-3' (SEQ ID NO:8); Primer-2, 5'-GACCGGCGTAACGGACGTTGTAATACAT-3' (SEQ ID NO:9); Primer-3, 5'-ATCCCCGCGGGAGTTGAAAGTTGCCATC-3' (SEQ ID NO:10); Primer-4, 5'-GACGGATCCAAAGTATATTGGAC-3' (SEQ ID NO:11). Genomic DNA (50-100ng) was prepared [Rose et al., *Methods in Yeast Genetics,* Cold Spring Harbor Press (1990)] from the *mof4-1* strain and used as the template in PCR. Primer pairs primer-1 and primer-2 were used to synthesize the DNA fragments to construct pmof4AE (Figure 4), primer-3 and primer 4 were used to synthesize the DNA fragment to construct pmof4AB (Figure 4) and primer 1 and primer 4 were used to construct pmof4BE (Figure 4), respectively. Two PCR products from two different reactions were used in the cloning reaction to minimize artifacts from PCR. The PCR conditions used were as follows: 95°C - 5 min, 94°C - 1 min, 45 or 50°C - 1 min, 72°C - 1.5 min, for 25 cycles. The DNA fragments from PCR were purified from 1% agarose gel and used for swapping the corresponding DNA fragment of the wild type UPF1 gene which was on a YCplac33 plasmid.

### Results

*Accumulation of CYH2 precursor and nonsense-containing PGK1 mRNA in a mof4-1 strain.* The eight *mof* mutants previously identified [Dinman and Wickner (1994) *supra*] were analyzed to determine whether they affected the activity of the nonsense-mediated mRNA decay pathway. The abundance of the inefficiently spliced CYH2 RNA precursor, which contains an intron near the 5' end, has been demonstrated to be a good monitor of the activity of this decay pathway [Hagan *et al.,* (1995) *supra*; Cui *et al.* (1995) *supra*; He *et al., Proc. Natl. Acad. Sci. USA,* **90**:7034-7038 (1993)]. The status of the nonsense-mediated mRNA decay activity in cells can be easily determined by comparing the ratio of the abundance of the CYH2 precursor to the CYH2 mRNA on an RNA blot.

The abundance of the CYH2 precursor and the CYH2 mRNA was monitored in cells harboring *upf1*⁻*, UPF1*⁺ (wildtype), and the various *mof* alleles by RNA blotting analysis. As shown in Figure 2A, the abundance of the CYH2 precursor was low in wild-type UPF1⁺ MOF⁺ cells but increased at least five-fold in the *upf1*⁻ strain. A survey of the CYH2 precursor abundance in the *mof* mutants demonstrated that the *mof4-1* allele had elevated CYH2 precursor level similar to the abundance previously observed in *upf* mutants (Figure 2A). The abundance of the CYH2 precursor was also elevated in cells harboring the *mof2-1, mof5-1* and *mof8-1* alleles, albeit not to the same extent as in *upf* or *mof4-1* strains (Figure 2A). The CYH2 mRNA abundance was not affected in these cells (Figure 2A).

The *mof4-1* allele was characterized further because it caused the greatest accumulation of the CYH2 precursor. To determine whether other nonsense-containing mRNAs were effected by the *mof4-1* allele, a nonsense-containing mini-PGK1 allele, whose abundance is sensitive to the *UPF1* status in the cell [Zhang *et al., Mol. Cell. Biol.* **15**:2231-2244 (1995)], was introduced into the *mof4-1* strain. The abundance of the wildtype and nonsense containing PGK1 mRNA was determined by RNA blotting and the results demonstrated that the nonsense-containing PGK1 transcript increased tenfold in a *mof4-1* strain as compared to its abundance in wild type cells, similar to the level in upf1-2 (Figure 2B). The abundance of the wildtype PGK1 mRNA did not change in any of the cells (Figure 2B).

*mof4-1 is allelic to the UPF1 gene.* We next determined whether *mof4-1* is allelic to any of the previously characterized *UPF* genes. A *mof4-1* strain was mated with *upf1Δ* or *upf2*Δ strains and the CYH2 precursor abundance was monitored in diploid cells. The CYH2 precursor abundance was low in the *mof4-1xupf2Δ* cells (data not shown), but was high in *mof4-1xupf1Δ* cells, with its abundance being equivalent to that observed in a haploid *upf1Δ* strain (Figure 2C). Furthermore, a strain harboring the *mof4-1* allele was transformed with centromere-based plasmids harboring either the *UPF1* gene, the *UPF2* gene, or vector alone and the abundance of CYH2 precursor was monitored. A mof4-1 strain transformed with a single copy of the UPF1 gene reduce the concentration of the CYH2 precursor to the same level as observed in wildtype UPF1⁺ cells (Figure 2C, Lanes 1-2 and 7). The plasmid harboring either *UPF2* gene or vector alone did not reduce the abundance of the CYH2 precursor in a *mof4-1* strain (Figure 2C, lanes 3-4 and 5-6). Furthermore, -1 ribosomal frameshifting efficiency as determined by pJD107 and pJD108 were elevated in both *mof4-1* and *upf1*⁻ cells, and the *UPF1* gene was able to reduce the frameshifting efficiency in a *mof4-1* strain to wildtype levels (data not shown). These results indicate that *MOF4* is allelic to *UPF1.*

Recently, Lee and colleagues [Lee *et al., Proc. Natl. Acad. Sci. USA,* **92**:6587(1995)] have identified two Increased Frame Shift (*ifs*) mutants in yeast. Using a construct containing the yeast *CUP1* gene downstream of a -1 ribosomal frameshift signal from the mouse mammary tumor virus *gag-pol* junction, *ifs* mutants were identified by loss of copper sensitivity in *CUP1Δ* cells. Both of these had -1 ribosomal frameshifting efficiencies approximately 2-fold greater than wild-type cells as measured by β-galactosidase activities. The *IFS1* gene was cloned and sequenced. Our comparison of *IFS1* and *UPF2* sequences demonstrated that they are identical [Cui *et al.,* (1995) *supra;* He and Jacobson, (1995) *supra*]. We have also determined that *ifs2* and *mof4-1* fall into the same complementation group by the β-galactosidase assay and that the Ifs phenotype of *ifs2* can be corrected by the *UPF1* gene (data not shown). Both *ifs1* and *ifs2* mutant strains were able to propagate M₁ (Table 2; see below), indicating that these mutations affected -1 ribosomal frameshifting efficiency by less than twofold.

**Table 2. Characterization of the Killer Phenotype and Drug Sensitivities**

| # | STRAIN | M₁ KILLER PHENOTYPE* | PAROMOMYCIN SENSITIVITY (cm)† |
|---|---|---|---|
| 1 | Wildtype *(MOF*⁺ *UPF*⁺*)* | + | 0.7 |
| 2 | *mof4-1* | - | 2.0 |
| 3 | *mof4-1*+pYCpUPF1 | + | 1.6 |
| 4 | *mof4-1* + vector | - | 2.4 |
| 5 | *mof4-1 (UPF1::URA3)* +vector | + | 1.7 |
| 6 | *mof4-1 (UPF1*::*URA3)* +pmof4XAB | + | 1.6 |
| 7 | *mof4-1 (UPF1*::*URA3)* +pmof4XAE | - | 2.3 |
| 8 | *upf1Δ* | + | ND |
| 9 | *upf2-1* | + | ND |
| 10 | *upf2Δ* | + | ND |
| 11 | *upf3-1* | + | ND |
| 12 | *upf4-1* | + | ND |
| 13 | *ifs1-1* | + | ND |
| 14 | *ifs2-1* | + | ND |

| | | | |
|---|---|---|---|
| * L-A and M₁ were introduced into cells by cytoduction. The killer phenotype was analyzed by the killer plate assay, and the ability to maintain the M₁ dsRNA virus was monitored by RNA blot analysis. † Strains #1 and #2 were grown in -Leu liquid media and subsequently plated on minimal media lacking leucine. A disc containing 1.0 mg of paromomycin was placed on the lawn of cells. The diameter of the zone of growth inhibition was determined after the plates were incubated at 30°C for 4 days. Strains #3, #4, #5, #6 and #7 are either *mof4-1* or *mof4-1* (*UPF1*::URA3 [deletion of the *mof4-1* allele from the chromosome) harboring the indicated plasmids. These cells were tested on media lacking uracel and leucine. | | | |

*Unlike upfΔ or upf1-2 alleles, the mof4-1 allele affects the maintenance of the M*_{*1*} *virus.* We next determined whether mutations that inactivate the nonsense-mediated mRNA decay pathway are able to maintain the M₁ dsRNA virus. L-A and M₁ were introduced by cytoduction into strains harboring the *mof4-1* allele of the *UPF1* gene, the *upf1Δ, upf2-1, upf2Δ, upf3-1, upf4-1, ifs1-1* and *ifs2-1* alleles, and these cells were replica plated onto a lawn of cells that are sensitive to the killer toxin. Cells that maintain M₁ secrete the killer toxin and a ring of growth inhibition is observed [Dinman and Wickner, (1992) *supra*]. The results from these experiments are summarized in Table 2 and demonstrate that only cells harboring the *mof4-1* allele were unable to maintain the killer phenotype. Consistent with the loss of the killer phenotype, the 1.8 kb M₁ dsRNA was not present in the *mof4-1* cells, confirming previous observation that *mof4-1* mutant cells also have a MAintenance of Killer phenotype (Mak⁻), *i.e.*, they cannot propagate M₁ (Table 2, Figure 3). Cells harboring the *upf1, upf2, upf3, upf4, ifs1* and *ifs2* alleles maintained the M₁ dsRNA (Table 2). The difference between the *mof4-1* allele and the *upf1Δ* and ifs2 alleles in maintaining M₁ suggests that the *mof4-1* allele is a specific mutation in the *UPF1* gene that alters both mRNA decay and the efficiency of -1 ribosomal frameshifting.

Several results demonstrate that the Mak⁻ phenotype is a consequence of the *mof4-1* allele rather than a secondary mutation within the cell. A single copy *UPF1* gene introduced into *mof4-1* cells on a centromere plasmid rescued the ability of *mof4-1* cells to maintain M₁, while the vector transformed cells had no affect (Table 2, compare #3 to #4). Furthermore, deleting the *UPF1* gene from the chromosome in cells harboring the *mof4-1* allele restored the killer phenotype (Table 2, #5). Tetrad analysis of cells harboring the *mof4-1* allele crossed with a *MOF*⁺ L-A⁺, M₂⁺ strains demonstrated a 2:2 segregation of killer⁺ and killer phenotype (Figure 3). Furthermore, RNA analysis of total nucleic acids from these spore clones show that the 1.8 kb M₁ dsRNA band is present in the MOF⁺ spore clones and is absent in the *mof4-1* spore clones (Figure 3).

*Cells harboring the mof4-1* allele are more sensitive to paromomycin. Strains harboring mutations that lower translational fidelity have been shown to be hypersensitive to the aminoglycoside antibiotic paromomycin, a drug that is thought to increase the frequency of misreading in yeast [Palmer *et al., Nature,* **277:**148-150 (1979); Singh *et al., Nature,* **277:**146-148 (1979)]. The paromomycin sensitivity of strains containing the *mof4-1* allele was determined. The *mof4-1* strain, the *mof4-1* strain harboring the *UPF1* gene on a plasmid, and a *mof4-1* strain in which the *UPF1* gene has been deleted (*mof4-1* (UPF::URA3)) containing or lacking the *mof4-1* allele on a centromere plasmid were grown and paromomycin sensitivity was monitored by comparing the zone of growth inhibition around a drug containing disc. The results demonstrate that strains harboring *mof4-1* allele were more sensitive to paromomycin than cells harboring either the wildtype *upf1* gene or a *upf1Δ* allele (Table 2, compare #2 to #1, #4 to either #3 or #5). Unlike the *mof4-1* strain, the *upf1Δ* strain was no more sensitive to paromomycin than the wildtype *UPF1*⁺ strain, which is consistent with results reported previously [Leeds *et al., Mol. Cell. Biol.,* **12:**2165-2177 (1992); Cui *et al.,* (1995) *supra*]. The sensitivity of those strains to paromomycin was a consequence of the *mof4-1* allele, since deleting *UPF1* from the chromosome of the *mof4-1* strain made it as resistant to paromomycin as the *mof4-1* strain harboring the wild type *UPF1* gene (Table 2, #3 and #5). In addition, a paromomycin resistant colony isolated from a parental *mof4-1* strain maintained M₁ and had wildtype -1 ribosomal frameshifting efficiency (data not shown). The co-reversion of these three phenotypes indicate that they are all linked to the *mof4-1* allele of the *UPF1* gene.

The effect of paromomycin on the expression of the *LacZ* gene in wildtype and *mof4-1* strains was also monitored. Cells were grown in liquid media in the presence of different concentrations of the drug and the β-galactosidase activities were determined, normalized to the number of cells utilized in the assay. The β-galactosidase activity in a *mof4-1* strain was measured and the results demonstrated that the LacZ expression steadily climbed with increasing concentrations of paromomycin (Table 3). The β-galactosidase activity in either a wildtype strain or a *mof4-1* strain harboring the wildtype *UPF1* gene was unaffected by the addition of paromomycin (Table 3). Taken together, these results indicate that paromomycin exacerbates the defect in a *mof4-1* strain and suggests that paromomycin can affect the efficiency of -1 ribosomal frameshifting in a *mof4-1* strain.

**Table 3. Paromomycin effect on LacZ gene expression in mof4-1 strains**

| paromomycin (µg/ml) | % -1 ribosomal | frameshifting* |
|---|---|---|
| | *mof4-1* + p*UPF1* | *mof4-1* |
| 0 | 2.0 | 9.4 |
| 5 | 2.2 | 9.2 |
| 10 | 3.0 | 9.4 |
| 25 | 2.9 | 13.5 |
| 50 | 2.0 | 14.7 |
| 100 | 2.6 | 15.3 |
| 250 | 2.2 | 17.8 |
| 500 | 2.2 | 22.2 |

| | | |
|---|---|---|
| * Cells were JD474-5A containing either pT125 (0-frame control) or pF8 (-1 ribosomal frameshift tester) [Dinman *et al.,* (1991) *supra*]. Paromomycin was added to cells inoculated at 01. OD₅₉₅/ml and grown at 30°C for 4 hours. The β-galactosidase activities were measured and % -1 ribosomal frameshifting was calculated by: (pF8/pT125) x 100%. The average β-galactosidase activities of cells with pT125 and cells with pT125+pUPF1 were 50.1 ± 7.5 and 48.9 ± respectively. | | |

*Identification of the mof4-1* mutation. The *UPF1* gene has been cloned and sequenced [Leeds *et al.,* (1992) *supra;* Altamura *et al., J. Mol. Biol.,* **224**:575-587 (1992)]. The deduced amino acid sequence of the *UPF1* gene indicates that it encodes a 109KD protein with zinc finger motifs near its amino terminus and harbors the appropriate motifs to be classified as a member of the ATP-binding RNA/DNA helicase superfamily group I [Altamura *et al.,* (1992) *supra;* Koonin, *TIBS,* **17**:495-497 (1992)]. We next wanted to identify the mutation(s) that caused *mof4-1* phenotype. Utilizing the appropriate primers, PCR products corresponding to either the 5' one-third or the 3' two-thirds of the *UPF1* gene from the *mof4-1* strain were isolated and hybrid genes between the wildtype *UPF1* and the *mof4-1* allele were prepared (Figure 4A). In addition, the complete *UPF1* gene from a *mof4-1* strain was also synthesized by PCR. These plasmids were transformed into *a upf1Δ* strain and the CYH2 precursor abundance was determined in these strains. The CYH2 precursor was abundant in cells containing a hybrid in which the 5' segment of the wildtype *UPF1* gene was replaced with the 5' fragment from the *mof4-1* allele (Figure 4 pmof4AE₁₋₂), or in cells containing plasmid pmof4BE₁₋₂, which encodes the complete *mof4-1* allele of the *UPF1* gene. The concentration of CYH2 precursor was low in cells harboring plasmid pmof4AB₁₋₂, which contains the hybrid *UPF1* gene in which the 3' two-thirds of the gene was replaced with the DNA fragment from the *mof4-1* allele (Figure 4 pmof4AB₁₋₂). The results indicate that the mutation in the *mof4-1* allele is located within the 5' one-third of the *UPF1* gene. Consistent with these findings, only hybrids that contained the 5' one-third portion of the *mof4-1* allele was sensitive to paromomycin (Table 2, #6 and #7).

The DNA sequence of the 5' region of the *mof4* allele (1150 nt; an EcoRI-Asp718 DNA fragment) was determined from both plasmids pmof4AE₁ and pmof4BE₁ (Figure 4). Comparison of this section with wildtype *UPF1* revealed that there was a single G → A mutation at nucleotide 586 in the cysteine-rich region, changing a cysteine codon to a tyrosine (Figure 4). Both *mof4-1* alleles from plasmids pmof4AE₂ and pmof4BE₂ also contained the same G → A mutation (data not shown). To confirm that the Cys → Tyr mutation resulted in the Mof4 phenotype, a 900 bp BstX1-Asp718 DNA fragment from the wildtype *UPF1* gene was replaced with an analogous DNA fragment from either plasmid pmof4AE₁ or pmof4BE₁ harboring the *mof4-1* mutation (Figure 4 pmof4XAE and pmof4XBE). Cells harboring the hybrid *UPF1* gene had the same characteristics as the *mof4-1* strain, having elevated CYH2 precursor abundance and were more sensitive to paromomycin (Table 2, Figure 4).

### Discussion

The results presented here indicate that the *mof2-1, mof4-1, mof5-1,* and *mof8-1* alleles, which were identified as mutations that increased the programmed -1 ribosomal frameshift efficiencies at the L-A frameshift site [Dinman and Wickner, (1994) *supra*], also increase the abundance of the nonsense-containing CYH2 precursor and mini-*PGK1* mRNA, suggesting that these mutations either partially or completely abrogate the activity of the nonsense-mediated mRNA decay pathway (Figure 2A). Strains containing the *mof4-1* allele had the greatest affect on the activity of the nonsense-mediated mRNA decay and was shown to be an allele of the *UPF1* gene (Figure 2). The *UPF1* gene has been sequenced and harbors zinc finger, NTP hydrolysis and helicase motifs [Altamura *et al.,* (1992) *supra*]. A *UPF1* gene disruption results in stabilization of nonsense-containing mRNAs and leads to a nonsense suppression phenotype [Leeds *et al., Genes & Dev.,* **5**:2303-2314 (1991); Cui *et al.,* (1995) *supra*].

Although strains containing the *mof4-1* and *upf1Δ* alleles both increase the abundance of nonsense-containing mRNAs, strains harboring these alleles have significantly different phenotypes. For example, the *mof4-1* strain is more sensitive to the aminoglycoside paromomycin than a *upf1Δ* strain (Table 2). Furthermore, unlike a *mof4-1* strain, *upf1*Δ strains can support the M₁ killer dsRNA virus (Table 2). The 39 nm L-A encoded viral particle has icosahedral symmetry and is composed of 59 Gag dimers and 1 dimer of Gag-Pol [Cheng *et al., J. Mol. Biol.,* **224**:255-258 (1994)]. The 1.9% of -1 ribosomal frameshifting efficiency determines the stoichiometry of Gag to Gag-Pol protein.
Changing the efficiency of -1 ribosomal frameshifting efficiency affects the ability of cells to propagate M¹[Dinman and Wickner, (1992) *supra*]. The loss of M₁ in *mof4-1* strains cannot be explained by stabilizing the frameshift-containing L-A mRNA. Overexpression of the L-A mRNA from a cDNA clone confers a Super Killer (Ski⁻) phenotype upon yeast cells [Wickner *et al., J. Virol.,* **65**:151-161 (1991); Masison *et al., Mol. Cell. Biol.,* **15**:2763-2771 (1995)], the opposite of the Mak⁻ phenotype. Our results suggest that the *mof4-1* allele of the *UPF1* gene specifically affects programmed -1 ribosomal frameshifting efficiency, changing the ratio of the Gag to Gag-Pol products synthesized. Interestingly, strains containing the *upf1Δ, upf1-2, upf2Δ, upf3-1* alleles, which all inactivate the nonsense-mediated mRNA decay pathway equivalent to the *mof4-1* allele, do not promote loss of M₁ (Table 2). This is consistent with the notion that simply stabilizing the L-A mRNA does not in itself promote loss of the M₁ RNA virus. Taken together, these results suggest that *mof4-1* is a unique allele of the *UPF1* gene that elevates the abundance of nonsense-containing mRNAs, increases the efficiency of directed -1 ribosomal frameshifting, and sensitizes these cells to paromomycin.

A single Cys → Tyr change at codon 62 in the *UPF1* gene accounts for the *mof4-1* allele of *UPF1* gene (Figure 4). This mutation is in the amino terminal cysteine rich region of the *UPF1* gene (Figure 4). A genetic analysis of the *UPF1* gene that investigated the role of the *upf*1p in mRNA turnover and nonsense suppression demonstrated that an amino terminal deletion that removed the cysteine rich region of the *UPF1* gene separated its decay activity from its function in translation termination. Cells harboring a *upf1* allele in which the zinc finger region was deleted were able to efficiently degrade nonsense-containing transcripts but inactivated its translation termination activity, allowing for suppression of nonsense alleles. Taken together, these observations further demonstrate that *upf*1p has activities involved in mRNA decay as well as modulating several aspects of the translation process. The *mof4-1* allele is unique because this lesion inactivates the nonsense-mediated mRNA decay activity and alters programmed translational frameshifting.

The results described here demonstrate that utilizing only frameshift reporter screens is insufficient for identifying ribosomal frameshifting mutants. The *upf1Δ, upf1-2, upf2-1, upf2Δ, upf3-1, ifs1, ifs2,* and *mof8* mutants score positive in reporter screens but do not affect the maintenance of the M₁ dsRNA virus (Table 2 and data not shown). Thus the MAK phenotype allows us to distinguish between mutations that affect ribosomal frameshifting efficiencies from those mutations that only affect mRNA turnover.

Viral packaging requires the appropriate stoichiometry of Gag to Gag-Pol proteins synthesized. This is often accomplished by ribosomal frameshifting or suppression of nonsense mutations. That the efficiency of -1 ribosomal frameshifting in *mof4-1* cells is elevated in response to increasing doses of paromomycin is important, since it demonstrates that a drug can modulate frameshift efficiencies, supporting the notion that ribosomal frameshifting may serve as a potential target for antiviral compounds. It is anticipated that the identification and characterization of gene products involved in these processes and of drugs that modulate this process will lead to therapeutics to combat viral diseases.

### EXAMPLE 2: Analysis of mof2-1 and sui1-1 at the genetic and molecular levels

*mof2-1* is of great interest in that, 1) it confers the greatest increase in -1 ribosomal frameshifting efficiency of all of the *mof* mutants, 2) it cannot propagate the M₁ satellite virus, 3) it is temperature sensitive, having a classic cell cycle dependant arrest phenotype, and 4) it also has a mutant nonsense-mediated mRNA decay (Upf) phenotype. A clone of the *SUI1* gene was able to complement the temperature sensitivity, frameshifting and Upf phenotypes of *mof2-1* mutants. Based on this result, this Example proposes to: A) analyze *mof2-1* and *sui1-1* at the genetic and molecular levels; B) determine whether the human homologue of the yeast *SUI1* gene, *hISOSUI1*, can complement the various mutant phenotypes of *mof2-1* cells, and; C) test the hypothesis that the Mof2 protein (Mof2p) is a general regulator of translational fidelity which increases rates of translational reinitiation and/or as a stimulatory factor upon nucleotide triphosphate (NTP) hydrolysis.

*Genetic and molecular analysis of mof2-1.* Figure 5A depicts a map of the original clone (p18) which was able to complement the *mof2-1* phenotype. Deletion analysis had revealed that a subclone of p18, harboring the *SUI1* gene was able to complement 1) the ts⁻ growth defect, 2) the nonsense-mediated mRNA defect, and 3) the Mof phenotype of *mof2-1* cells. The first set of experiments were designed to determine whether *MOF2* is allelic to *SUI1.* All of the spore clones generated from a genetic cross of *mof2-1* with *sui1-1* mutants were temperature sensitive for growth at 37°C, evidence that *mof2-1* and *sui1-1* belong to the same complementation group. In a second experiment, a *URA3* based yeast integrating vector was constructed using a yeast DNA fragment lying downstream of the *SUI1* gene (the H3 to *Sal* I fragment from p18). This was linearized with *Sph* I and integrated into the DNA from a *mof2-1*/wild-type diploid strain. After southern analysis to determine that the fragment had integrated into the correct location of one of the chromosomes, the diploid was sporulated and the resulting spore clones were analyzed. The Ura3⁺ phenotype always co-segregated with the Mof2-1 phenotype, indicating that DNA. sequence from p18 (which is physically linked with *SUI1*) was also physically linked to the *mof2-1* gene. Taken together, these data prove that *mof2-1* is an allele of *SUI1.* We were also able to demonstrate that a plasmid harboring the *SUI1* gene allowed *mof2-1* mutants to propagate the M₁ satellite virus.

The *mof2-1* allele of *SUI1* was cloned using polymerase chain reaction (PCR) techniques and we were able to sequence the entire gene. Sequence analysis revealed that the mof2-1 allele results from a single G-->A base mutation which changes amino acid residue 115 from a highly conserved Glycine to Arginine (Figure 5B). This represents a unique *sui1* allele: all of the other *sui1* alleles are clustered at highly conserved D81 and Q82 amino acid residues within the context LQGDQR (SEQ ID NO:12) [Yoon and Donahue, *Mol. Cell. Biol.,* **12**:248-260 (1992)]. This figure also depicts a comparative alignment of the *SUI1* homologues from humans (SEQ ID NO:1), *Aedes* sp (mosquitos) (SEQ ID NO:2), rice (SEQ ID NO:3), *S. cerevisiae* (SEQ ID NO:4), and *Methanococcus* sp (SEQ ID NO:5).

*The human homologue of the yeast SUI1 gene can complement the Mof- and nonsense-mediated mRNA mutant phenotypes of mof2-1 cells.* Several mammalian initiation factors have been shown to be able to complement the counterpart defects in yeast cells, and the human homologue of *SUI1* (*hSUI1ISO1*) has been cloned and sequenced. Sui1p and hSUI1ISO1p share 60% identity and 80% similarity (31). *hSUI1ISO1* is able to complement the temperature sensitivity, increased efficiency of -1 ribosomal frameshifting, nonsense-mediated mRNA decay, and M₁ maintenance phenotypes of *mof2-1* mutants. These experiments demonstrate the conservation of function between homologous human and yeast genes, serving as yet another example of the basic biological similarity of these two organisms. These observations are of great help with regard to our investigations into the linkage between translational initiation, translational elongation, and nonsense-mediated mRNA decay in mammalian systems, and also help in the in the process of identifying proteins which may serve as potential targets for the rational design of antiviral agents which affect -1 ribosomal frameshifting.

*Further genetic investigations using the different forms of Sui1.* Having shown that *mof2-1* is a unique allele of *SUI1* and that the human homologue can complement all of the *mof2-1* phenotypes, we conducted a series of experiments to examine the genetic links between ribosomal frameshifting, viral maintenance and translational misreading resulting in suppression of translation initiation mutations. To best address this, isogeneic yeast strains were constructed. A haploid cell harboring the wild-type *SUI1* gene on a low copy *URA3* vector was transformed with a *sui1*/yeast integrating vector, disrupting the chromosomal copy of the *SUI1* gene. After selection and confirmation of the disruption by southern analysis, low copy *TRP1* vectors harboring the wild-type *SUI1* gene, the *mof2-1* allele, the *sui1-1* allele, or *hSUI1ISO1* were introduced into this strain. Subsequently, the *URA3* based vector was cured from the cell using 5-flourootic acid (5-FOA), leaving only the *TRP1* vectors in the cells. Further genetic analysis examined comparative the Mof and Sui phenotypes of *mof2-1, sui1-1,* and *hISOSUI1* (Tables 4 and 5).

**Table 4. Characterization of frameshifting efficiencies in isogenic MOF2/SUI1 cells.**

| Strain | β-galactosidase* activity | | | Frameshifting efficiency %(5) | | Frameshifting (fold wild type) | | Maintenance (+) or loss (-) of M₁ dsRNA |
|---|---|---|---|---|---|---|---|---|
| | 0 | -1 | +1 | -1 | +1 | -1 | +1 | |
| Y218 (WT) | 43.48 | 0.91 | 1.39 | 2.1 | 3.2 | 1.0 | 1.0 | + |
| Y219 (*mof2-1*) | 45.46 | 4.86 | 1.64 | 10.7 | 3.6 | 5.1 | 1.2 | - |
| Y220 (*sui1-1*) | 44.70 | 2.41 | 1.38 | 5.4 | 3.1 | 2.6 | 1.0 | + |
| Y221 (hISOSOUI) | 32.38 | 0.65 | 0.97 | 2.0 | 3.0 | 1.0 | 1.0 | + |

| | | | | | | | | |
|---|---|---|---|---|---|---|---|---|
| * These experiments were performed in at least three different colonies from each strain. The unit of β-galactosidase activity was presented as activity/OD₆₀₀/hour and they varied no more than 15%. Maintenance or loss of M₁ dsRNA virus was determined by both M₂ killer assay and RNA electrophoresis on a agarose gel. | | | | | | | | |

**Table 5. His4^{UUG} suppression in SUI1/MOF2 strains**

| Strains (allele)* | FUS4^{AUG}-lacZ β-gal activity | his4^{UUG}-lacZ β-gal activity | UUG/AUG ratio(%) | UUG suppression (fold wildtype) |
|---|---|---|---|---|
| Y218(WT) | 8.6 | 0.22 | 2.5 | 1.0 |
| Y219(*mof2-1*) | 7.1 | 1.14 | 16.0 | 6.4 |
| Y220(*suil-1*) | 4.6 | 0.68 | 15.6 | 6.2 |
| Y221(hISOSUI1) | 6.1 | 0.19 | 3.0 | 1.2 |

| | | | | |
|---|---|---|---|---|
| * These experiments were done in the isogenic SUI1/MOF2 strains. The numbers were the average of measurements in three independent colonies. The β-galactosidase activity was presented as activity/OD₆₀₀/hour with variations no more than 15%. | | | | |

Table 4 shows the characterization of frameshifting efficiencies in isogenic *MOF2*/*SUI1* cells. The efficiency of -1 ribosomal frameshifting of *mof2-1* cells is approximately 5-fold greater than their wild-type counterparts. *sui1-1* mutants also increase the efficiency of -1 ribosomal frameshifting approximately 2.5 -fold above wild-type levels. However, this is not enough of an increase to promote the loss of the M₁ satellite virus, *i.e.,* only the *mof2-1* mutation confers the Mof phenotype on cells. The human homologue appears to be completely capable of substituting for the yeast gene, further supporting the notion of evolutionary conservation of Sui1p function. Interestingly, none of the forms of Sui1 tested had any affect on Ty*1* promoted +1 ribosomal frameshifting.

Table 5 reports the Sui phenotypes of our constructs. This analysis shows that *mof2-1* cells also have a complete Sui⁻ phenotype, promoting efficient suppression of the *his4*^{*UUG*} allele. Again, the human isogene is able to complement the yeast gene.

*Characterization of the GCN4 derepression phenotype of the sui1-1 and mof2-1 mutants.* The *GCN4* gene is repressed by a translational control mechanism in which short open reading frames upstream of the gene (uORFs) are translated in the presence of abundant pools of aminoacylated tRNAs. This causes translating ribosomes to dissociate from the mRNA prior to being able to re-initiate at the start of the GCN4 message. Under conditions of amino acid starvation, *GCN4* is derepressed *via* enhanced levels of re-initiation at the GCN4 initiation codon. The *sui2* (eIF-2α) and *SUI3* (eIF-2β) mutants abolish translational repression of *GCN4,* leading to constituitively derepressed *GCN4* expression and *HIS4* transcriptional activation independent of amino acid availability.

We tested whether the *mof2-1, sui1-1,* and *hSUI1ISO1* forms were also able to derepress *GCN4* by measuring β-gal activity expressed from a *GCN4-lacZ* fusion constructs. *GCN4-lacZ* enzyme activity derepresses about 10-fold in response to histidine starvation in wild-type cells (38). If the *suil-1* and *mof2-1* mutations had yielded increased *GCN4-lacZ* expression, it would have suggested that these mutations, like the *sui2* and *SUI3* mutations disrupt the translational control of the GCN4 mRNA. Figure 7 shows that neither *mof2-1* nor *sui1-1* derepress *GCN4* expression. This suggests that the apparent increases in -1 ribosomal frameshifting are not due to increased levels of ribosomal reinitiation or internal initiation in the out of frame lacZ reporter mRNA, further supporting the conclusion that the *mof2-1,* and to a lesser extent, the *sui1-1* mutants truly exert their effects at the level of -1 ribosomal frameshifting.

*Experiments directed toward the biochemical characterization of Sui1p.* Clones of *SUI1, mof2-1, sui1-1* and *hSUI1ISO1,* that contain the FLAG epitope tagged to their N-terminal ends, have been prepared in order to biochemically evaluate Sui1p. These clones are capable of supporting yeast cell growth in the absence of other forms of *SUI1,* indicating that the addition of the epitope tags do not interfere with Sui1p function. We have also been able to express these recombinant proteins using high copy vectors in *E*. *coli,* and are able to isolate large amounts of pure protein using immunoaffinity columns.

We have also developed an *in vitro* -1 ribosomal frameshifting assay using extracts of yeast cells. Preliminary experiments using the isogenic strains described above have demonstrated that the general pattern observed *in vivo* holds true for the *in vitro* system, *i.e.,* that the efficiency of -1 ribosomal frameshifting is approximately 5 fold greater in extracts of *mof2-1* cells, and 2 fold greater in extracts of *sui1-1* cells (data not shown).

*Investigations into the possible ribosome association of the Sui1p, Mof2-1p, Sui1-1p and hISOSUI1p.* The fact that *mof2-1* has effects on both -1 ribosomal frameshifting and nonsense-mediated mRNA decay suggests that Sui1p may be involved in translation processes beyond initiation. Western blots of polysome fractions can be probed for the Sui1p to determine if this protein is present in the elongating ribosomes. For example, in cells harboring FLAG-tagged Sui1 proteins, an anti-FLAG antibody can be used to visualize the Sui1p bands. Sui1p in the fractions corresponding to elongating ribosomes can then be compared with those gleaned from extracts of cells harboring FLAG-tagged *mof2-1* and *sui1-1* mutations. The polysome profiles may be performed under different conditions, *e.g.*, growth rate, temperature, drug treatment (cycloheximide, puromycin), and salt concentrations. An alternative approach is to gently lyse logarithmically growing cells and fractionate them using gel filtration techniques. An anti-*TCM1* antibody (ribosomal protein L3) can be used to identify the various ribosome fractions. Polysome fractions, which should correlate with ribosomes in the elongation phase, will be larger than the monosome fractions, and as such should elute earlier from the column. These fractions can be probed for the various FLAG-tagged Sui1p isoforms using the anti-FLAG antibody. Quantative western blotting techniques may be used in an effort to discern qualitative differences in binding of the different Sui1p isoforms.

*Examining the effects of purified wild-type, Mof2-1p, Sui1-1p and hSUI1ISOp on GTP hydrolysis with purified G-proteins known to be involved in elongation phase of translation.* Standard assays are used to determine the baseline rates of GTP hydrolysis of EF-1α and EF-2β [Kinzy and Woolford, *Genetics,* in press; Merrick, *Enzymol.,* **60**:108-123 (1979); Perentesis *et al., J. Biol. Chem.,* **267**:1190-1197 (1992)]. The different purified isoforms of our Sui1p's are added to these reactions to determine their effects on the rates of GTP hydrolysis. Sui1p enhanced GTP hydrolysis by one or both of these proteins would support a conclusion that Sui1p also acts during translational elongation.

*Examining the effects of purified wild-type, Mof2-1p, Sui1-1p and hSUI1ISOp on ATP hydrolysis using purified Upf1p, which is known to be involved in elongation phase of translation.* The observation of either stimulatory or inhibitory effects substantiates Sui1p's involvement in the peptidyl-transfer step of translational elongation (nonsense-suppression occurs at termination, which occurs at the peptidly-transfer step). In the event that Sui1p's effects on Upf1p mediated ATP hydrolysis are observed, the effects of the Sui1p isoforms can be examined as well. If Sui1p does affect Upf1p mediated ATP hydrolysis, any changes in stimulatory/inhibitory effects of Sui1p on the Upf1p mutants will be of particular interest.

*Gene dosage experiments.* These experiments are designed to determine whether overexpression of EF-1α, EF-2β, or Upf1p can suppress either the *mof2-1,* or the *sui1-1* mutations. *mof2-1* and *sui1-1* cells can be transformed with high copy plasmids harboring the wild-type *TEF2, EFT1,* or *UPF1* genes. The -1 ribosomal frameshifting efficiencies, ability to maintain M₁, nonsense-mediated decay phenotypes, and ability to suppress the *his4*^{*UUG*} allele can be evaluated.

### EXAMPLE 3: Identification Of Drugs Which Alter Ribosomal Frameshifting and Inhibit Viral Propagation

One critical step in viral propagation is assembly of the viral particle, which is partially dependent upon the availability of the correct relative amounts of viral proteins. Thus, although maintenance of translational reading frame is considered to be fundamental to the integrity of the translation process and ultimately, to cell growth and viability, many cases have been described in which ribosomes are directed to shift reading frame by viral signals in order to ensure the appropriate ratios of structural (Gag) to enzymatic (Gag-pol) proteins available for viral particle assembly. Such ribosomal frameshifting signals are for the most part seen in RNA viruses, *e.g.*, retroviruses and retrotransposable elements, coronaviruses, astroviruses, totiviruses, and some unsegmented (+) ssRNA viruses of plants. Ribosomal frameshifting has also been described in bacteriophage T7, and a number of bacterial transposons, as well as in a few bacterial cellular genes and in one mammalian chromosomal gene. Viral frameshifting events produce fusion proteins, in which the N- and C- terminal domains are encoded by two distinct, overlapping open reading frames. Ribosomal frameshifting in the -1 direction requires a heptameric sequence, X XXY YYZ (the 0-frame is indicated by spaces) called the "slippery site" [Jacks and Varmus, *Science* 230:1237 (1985)]. The simultaneous slippage of ribosome-bound A- and P-site tRNAs by one base in the 5' direction still leaves their non-wobble bases correctly paired in the new reading frame. Ribosome bound tRNAs must first un-pair from the 0-frame and then re-pair their non-wobble bases to the -1 frame. A second frameshift promoting element, usually an RNA pseudoknot, is located immediately 3' to the slippery site. The mRNA pseudoknot makes the ribosome pause over the slippery site while the ribosomal A- and P-sites are occupied by aminoacyl-tRNA (aa-tRNA) and peptidyl-tRNA species, respectively. It is thought that the RNA pseudoknot promotes ribosomal pausing over the slippery site, increasing the probability of 5' ribosomal movement [Somogyi et al., *Mo. Cell. Biol.* **13**:6931 (1993); Tu et al., *Proc. Natl. Acad. Sci. USA* **89**:8636 (1992)].

The effects of two peptidyl-transferase inhibitors, anisomycin and sparsomycin, on ribosomal frameshifting efficiencies and on the propagation of yeast dsRNA viruses were examined. These drugs specifically alter the efficiency of -1, but not of +1 ribosomal frameshifting both *in vivo* and *in vitro,* and promote loss of two viruses (L-A and L-BC) which utilize a -1 ribosomal frameshift in yeast cells cultured in sub-lethal doses of these drugs. Both of these drugs also change the efficiency of -1 ribosomal frameshifting in a rabbit reticulocyte system, suggesting that they may have applications for RNA viruses of higher eukaryotes which also utilize this translational regulatory mechanism. Our results offer a new set of compounds to the arsenal of antiviral agents, having a potentially broad range of applications in the clinical, veterinary and agricultural fields.

To examine the effects of these drugs on ribosomal frameshifting efficiencies *in vivo,* selective medium containing the indicated concentrations of anisomycin or sparsomycin was inoculated with equal amounts of logarithmically growing yeast cells harboring the 0-frame control (pTI25), L-A derived -1 ribosomal frameshift test (pF8) or Ty*1* derived +1 ribosomal frameshift test plasmids (pJD104) [Balasundaram et al., *Proc. Natl. Acad. Sci. USA,* **91**:172 (1994)], and incubated at 30°C for specific amounts of time, after which β-galactosidase (β-gal) activities were determined. Two important sets of information were gleaned from these experiments (Figure 8). First, as indicators of the effects of these drugs on translation in general, β-gal activities from cells containing pTI25 grown in different drug concentrations were monitored. This set of data shows that these drug concentrations do not decrease overall translation to less than 80% (anisomycin) (Figure 8A) or less than 50% (sparsomycin) (Figure 8B) of the no drug controls. The second data set, measurement of the efficiencies of -1 and + 1 ribosomal frameshifting was determined by calculating the ratios of β-gal produced from cells harboring pF8 or pJD104 divided by the β-gal activities from cells harboring pTI25 grown in equivalent concentrations of drugs. Here, the data show that, in general, increasing concentrations of sparsomycin tend to increase the efficiency of -1 ribosomal frameshifting (Figure 8C), whereas anisomycin has the opposite effect, *i.e.,* decreasing -1 ribosomal frameshifting efficiencies (Figure 8D). As predicted, neither drug had any affect on + 1 ribosomal frameshifting.

To determine whether or not changes in raw β-gal activity values were a consequence of either decreased (anisomycin) or increased (sparsomycin) β-gal half lives, the effects of these drugs were measured after a number of different periods of incubation. The results remained consistent, *i.e.,* longer incubation times did not result in greater changes in -1 ribosomal frameshifting efficiencies (data not shown). To remain consistent, all subsequent *in vivo* measurements of ribosomal frameshifting were assayed after 6hr incubations with these drugs. Apparent changes in ribosomal frameshifting efficiencies could also be due to decreased (anisomycin) or increased (sparsomycin) abundances of the frameshift reporter mRNAs. To address this, RNA was extracted from mid-log phase cells grown in different concentrations of anisomycin or sparsomycin, equal amounts of RNA were separated through a denaturing-agarose gel, transferred to nitrocellulose and probed with radiolabeled *lacZ* probe. The abundances of the lacZ mRNAs were equivalent at all concentrations of drugs tested, suggesting that drug induced changes in reporter mRNA abundance was not responsible for the apparent changes in -1 ribosomal frameshifting efficiencies (data not shown).

The model also predicts that host cells harboring mutations in gene products involved in the formation of the peptidyl transferase center should 1) have efficiencies of -1 ribosomal frameshifting different from wild-type cells, 2) should have viral propagation defects and, 3) neither anisomycin nor sparsomycin should have any additional effects upon -1 ribosomal frameshifting efficiencies. The *TCM1* (*MAK8*) gene encodes ribosomal protein L3 [Fried and Warner, *Proc. Natl. Acad. Sci. USA,* **78**:238 (1981); Fried and Warner, *Nucl. Acids Res.,* **10**:3133 (1982); Wickner et al., *Proc. Natl. Acad. Sci. USA****,* 79:**4706 (1982)], and *mak8* mutants provide an appropriate test for these criteria. *tcm1*/*mak8* mutants cannot maintain M₁, and they are resistant to anisomycin and a variety of other peptidyl transferase inhibitors [Jiminez et al., *Biochim. Biophys. Acta* **383**:427 (1975)]. The efficiency of -1 ribosomal frameshifting in *mak8-2* cells in the absence of drugs was determined to be approximately 5.2% (Figure 9A,B, no drug). This value is 2.9 fold greater than wild-type cells (1.8%), consistent with the notion that conditions affecting the peptidyl-transferase center should change the efficiency of -1 ribosomal frameshifting. As predicted, neither of these drugs affects the efficiency of -1 ribosomal frameshifting (Figure 9C, D).

Although increasing doses of either anisomycin or sparsomycin tend to inhibit translation in wild-type cells *in vivo,* they have opposite effects on the efficiency of -1 ribosomal frameshifting. To determine whether these drugs are acting at the same step in translation, a mixing experiment was assayed (Figure 10). These results show that, with regard to effects on -1 ribosomal frameshifting, anisomycin and sparsomycin cancel one another out. This is consistent with the fact that they both act at the peptidyl transferase step in translation, but does not distinguish between whether they compete for the same, overlapping, or separate binding sites.

By changing the efficiency of -1 ribosomal frameshifting, these drugs should change the ratio of Gag to Gag-pol proteins available for viral particle assembly, consequently interfering with viral propagation. To address this, wild-type cells were cultured in rich medium containing different concentrations of either anisomycin or sparsomycin. After 24, 48, 72, 96, or 120 hrs., aliquots of cells were streaked for single colonies onto rich medium, which were then replica plated onto indicator to score their killer phenotypes. Figure 11A,B shows that loss of the killer phenotype correlated with both increasing drug dosage and fold changes in -1 ribosomal frameshifting efficiencies. Although the killer phenotype is stably maintained in JD88 cells in the absence of drugs, growth in 3.8 µM anisomycin resulted in approximately 53% of total colony forming units losing the killer trait after only 48 or 72 hours (Figure 11A). More dramatically, growth in the presence of 2.6 µM sparsomycin resulted in 70% - 75% loss of the killer phenotype (Figure 11B).

To determine whether loss of the M₁ satellite virus was responsible for loss of the killer phenotype, a non-killer (K⁻) colony from each drug concentration in the 72 hr data set was picked at random and total nucleic acids (TNA) were extracted [Park et al., *Virology* **216**:451 (1996)]. Approximately equal amounts of TNA were separated through a 1% non-denaturing TAE-agarose gel and stained with ethidium bromide (Figure 12A). As expected, the 1.8 kb M₁ dsRNA band is not present in the K⁻ (drug treated) samples. Further, L-A appears to have also been cured by these drugs. To confirm this, the RNA was denatured in the gel, transferred to nitrocellulose and hybridized with [³²P]CTP labeled L-A and M₁ (+) strand RNA probes. Figure 12B confirms that these samples do not hybridize with either of the probes, confirming that loss of the killer phenotype was a consequence of loss of L-A, supporting the notion that drug induced changes in the efficiency of -1 ribosomal frameshifting interfered with the assembly and propagation of L-A viral particles.

Recently the cDNA sequence of L-BC (also known as La), a ubiquitous minor dsRNA virus of yeast has been determined [Park et al., *supra*]. L-BC also uses the classic -1 ribosomal frameshift mechanism in the production of its Gag-pol fusion. L-BC, however, has been shown to be much more difficult to cure from cells than L-A [Hopper et al., *J. Biol. Chem.* **252**:9010 (1977)]. Interestingly, after 72 hrs at 3.8 µM concentrations of anisomycin, the L-BC dsRNA band also appears to missing (Figure 12A). No L-BC specific probe is available however, and this could not be confirmed by northern blot. However, these data suggest that in addition to promoting the loss of L-A and M₁, anisomycin induced decreases in -1 ribosomal frameshifting also promotes the loss of L-BC.

The specific actions of antibiotics on the translational apparatus were classically assayed using *in vitro* systems. To test the effects of anisomycin and sparsomycin on *in vitro,* a yeast based *in vitro* translation system was used to monitor the efficiency of -1 ribosomal frameshifting using a luciferase based reporter system. The system comprised translation competent yeast extracts from an L-A-o, L-BC-o wild-type strain to which either a 0-frame control or a -1 ribosomal frameshift tester luciferase reporter mRNAs were added. Figure 13 shows that the trends observed *in vivo* were reproducible using the *in vitro* system, *i.e.,* increasing concentrations of anisomycin decreased the efficiency of -1 ribosomal frameshifting, while increasing concentrations of sparsomycin increased -1 ribosomal frameshifting efficiencies. Some interesting differences are observed between the *in vivo* and *in vitro* systems. First, the effective concentrations of these drugs are three orders of magnitude lower *in vitro,* most likely a reflection of drug uptake and metabolism in intact cells. Second, at these lower drug doses, both drugs initially stimulate luciferase production from the LUC0 reporter mRNA. However, even in the stimulatory range, the ribosomal frameshifting trends remain the same. Third, the baseline efficiency of -1 ribosomal frameshifting is significantly higher *in vitro* than *in vivo*. This could be indicative of differences in the stabilities of the reporter mRNAs in the two systems. Since the -1 ribosomal frameshift reporter mRNA has two in-frame termination codons within the first 200 bases of its 3.3 kb mRNA, it constitutes a nonsense-mRNA. This makes it a substrate for attack by the nonsense-mediated mRNA decay pathway. Indeed, cells harboring mutations in genes involved in this pathway stabilize this reporter mRNA and yield apparent increased efficiencies of -1 ribosomal frameshifting *in vivo.* The observed differences in the baseline efficiencies of -1 ribosomal frameshifting may be an indication that the nonsense-mediated mRNA decay pathway may be partially or wholly inactivated in the *in vitro* translation system. The *in vitro* system also differs in that it contains the RNase inhibitor RNAsin which may help to stabilize the LUC-1 reporter mRNA from general nucleolytic attack. These factors are most likely responsible for higher baseline efficiencies of -1 ribosomal frameshifting observed in this, and other reports using *in vitro* translation systems, and underscores the importance of using ribosomal frameshifting efficiencies determined *in vivo* as more reliable indicators of virally determined Gag to Gag-pol ratios.

Asinomycin and sparsomycin affect the efficiency of -1 ribosomal frameshifting and viral propagation in yeast: do these results translate to higher eukaryotes? As a first step, the LUC0 and LUC-1 mRNAs were used to measure the effects of these two drugs on translation and -1 ribosomal frameshifting in an *in vitro* rabbit reticulocyte translation system. Figure 14 shows that the same general trends seen in the yeast based systems are recapitulated in the reticulocyte system, *i.e.,* anisomycin inhibits, and sparsomycin stimulates -1 ribosomal frameshifting. Some notable differences were observed. First, the reticulocyte extracts were approximately 10 times more sensitive to anisomycin and 100 times more sensitive to sparsomycin than were the yeast extracts. In this range of concentrations, both drugs stimulated overall production of luciferase from the LUC0 mRNA, which is not inconsistent with the lower range drug concentration data from the yeast extract system. With regard to -1 ribosomal frameshifting, the reticulocyte system was much more sensitive to both drugs. The highest concentration of anisomycin (15.2 nM) decreased -1 ribosomal frameshifting efficiencies to only 3% of the no drug control, effectively shutting it down. The highest concentration of sparsomycin stimulated -1 ribosomal frameshifting 14-fold, or from approximately 6% in the no drug control to almost 84%, *i.e.,* almost as many ribosomes were induced to shift reading frame as remained in frame. As with the yeast *in vitro* translation system, initial efficiencies of -1 ribosomal frameshifting were higher than observed *in vivo,* again most likely due to stabilization of the LUC-1 mRNA in this system.

Antiviral activities of these antibiotics were also tested against HIV. Figure 15 shows that these drugs decrease HIV viral titers to approximately 30% of the no drug controls at concentrations of anisomycin that were 1500-fold lower than the minimum inhibitory concentration of anisomycin (anisomycin MIC is about 1.5 ug/ml), and 50 times less than the MIC of sparsomycin (sparsomycin MIC = 50 ng/ml). Thus, these two peptidyl transferase inhibitors possess the antiviral properties predicted by the model present in Figure 1 at concentrations that are well below toxic levels to the human host cells.

### EXAMPLE 4: Demonstration of Non-Redundant Phenotypes Of The Upf Proteins

The *TCM1* gene encodes the 60S ribosome subunit protein L3 which has been implicated as a component involved in peptidyl-transferase activity (Schulz and Nierhaus, 1982). *TCM1* is the same as *MAK8,* a gene required for the maintenance and propagation of the M₁ double-stranded RNA satellite virus. A genomic fragment of *TCM1*/*MAK8* (L3Δ). encoding N-terminal 100 amino acids of L3 was isolated as an antisuppressor of *upf2-1,* a mutant allele that stabilizes nonsense-containing mRNAs. This fragment can also act as an antisuppressor of *upf1-2,* but not *upf3-1*, two other alleles that are involved in the nonsense-mediated mRNA decay pathway. A full length clone of *TCM1*/*MAK8* did not have this antisuppressor activity. Expression of L3Δ has no effect on the stability of nonsense mRNAs in either wild-type or *upf* mutant cells. Expression of the L3Δ fragment conferred a Mof⁻ phenotype on wild-type cells, increasing the efficiency of -1, but not of +1 ribosomal frameshifting, and the efficiency of -1 ribosomal frameshifting is also elevated in a *mak8* strain. Polysome profiles from strains harboring the L3Δ fragment showed reduced levels of 60S subunits compared with wild-type cells, a characteristic feature of most *mak* mutants, and killer assays demonstrated that episomal expression of the L3Δ fragment confers a dominant negative Mak⁻ phenotype on wild-type and Ski⁻ strains. *upf1* and *upf2* mutants are normally able to maintain M₁ and expression of L3Δ increases rates of killer loss in these cells. *upf3* mutants have an weak Mak⁻ phenotype which becomes complete in the presence of L3Δ. These data implicate the involvement of the peptidyl-transferase center in the maintenance of frame in translation independent of the nonsense-mediated mRNA decay pathway

### Materials and Methods

Restriction enzymes were obtained from Boehringer Mannheim, New England Biolabs, and BRL. Radioactive nucleotides were obtained from either NEN or Amersham. Oligonucleotides used in these studies were purchased from the UMDNJ-RWJ DNA synthesis center.

*Isolation and characterization of the L3Δ clone.* Ycp50 (Ausubel et al., 1992) and YCplac33 (Gietz and Sugino, 1988) were used in these studies. The L3Δ clone was isolated from a YCp50 yeast genomic library (purchased from ATCC) that was prepared from a partial *Sau3A* digest as previously described (Cui et al., 1995). Briefly, the *upf2-1 his4-38 SUF1-1* strain PLY136 was transformed with this library and a total of 5000 Ura⁺ transformants were screened by replica-plating onto minimal media lacking uracil and histidine and grown at either 30°C or 37°C for 3 days. Colonies that grew at 30°C but not at 37°C were picked and retested. Nine strains harboring plasmids were isolated (YPF2-1 to YPF2-9). To confirm that loss of suppression was due to the plasmid, the transformed strains were cured with 5-Flouroorotic acid (5-FOA) (Rose et al. 1990) and tested for their ability to suppress growth at 37°C on selective media. The plasmids were then isolated from these strains and propagated in *E. coli.* YPF2-4 and YPF2-9 were found to be identical (named YcpA9), each containing the L3Δ gene fragment.

*Subcloning of the L3A gene fragment.* YCpA9 was analyzed by restriction endonulease mapping. Fragments of the DNA insert in YCpA9 were subcloned into the yeast centromere plasmid YCplac33: YCpESp (2.7-kb *EcoRI-SphI* DNA fragment), YCpESn (4.2-kb *EcoRI-SnaBI* DNA fragment), YCpBS (4.3-kb *BglII-SalI* DNA fragment), YCpNS (3.5-kb *NcoI-SalI* DNA fragment), YCpPS (0.9-kb *PvuII-SalI* DNA fragment). Plasmid YCpdP is a derivative of YCpA9 in which the *PvuII* DNA fragment was deleted.

*Tagging the L3Δ fragment with the FLAG-epitope, creation of a L3Δ*/*β-gal fusion protein, and subcloning.* Oligo 5'-ATAGGATCCTTAACCGGCCGGACAGTAATA-3' (SEQ ID NO:13) corresponding to the 5' of the *TCM1* gene and oligo 5'-ATAGGATCCTTGTCATCGTCGTCCTTGTAGTCTCTCAAACCTCTTGGGGTT-3' (SEQ ID NO:14) containing the FLAG-epitope sequence and sequence complementary to the 3' end of the L3Δ coding region were used for Polymerase chain reaction (PCR) mutagenesis using genomic DNA from wild-type cells as template. The PCR products were digested with *Bgl II and BamHI,* and cloned into the YCplac33 vector. The complete *TCM1*/*MAK8* gene was also cloned by polymerase chain reaction and subcloned into YCplac33 and YEplac195. pJD134 was constructed by cloning a blunt ended *Bgl II*/*Hind III* fragment containing the *PGK1* transcription terminator into a blunt ended *Not I* site of pRS315 (Sikorski and Hieter, 1989). The FLAG-tagged L3Δ *Bam HI* fragment was subcloned into pJD134 to create pJD138, a *CEN6 LEU2* vector containing the *PGK1* transcription termination sequence downstream of the C-terminal FLAG-tagged L3Δ fragment. pJD139 contains the L3Δ -FLAG- PGK1-transcriptional terminator subcloned into the 2µ *URA3* vector pRS426 (Christianson et al., 1992).

*Cloning of UPF3.* The strategy used to clone the *UPF3* gene was the same that was used to clone *UPF2* (REF) and the L3Δ fragment. The *upf3-1* strain PLY139 was transformed with a Ycp50 library (Rose et al.,) and a total of 2 x 10⁴ Ura⁺ transformants were screened by replica-plating onto minimal media lacking uracil and histidine and grown at either 30° or 37° for 3 days. Colonies that only grew at 30° were chosen for further analysis and one plasmid, YCpB1, was confirmed to harbor the UPF3 gene by standard genetic methods, including the construction of gene knockout alleles. Subsequent subcloning revealed that a 2.1 kb *Asp718-Bgl II* fragment was sufficient to complement *upf3* mutations, and sequence analysis of this clone showed that it was identical to the UPF3 sequence previously reported (REF).

*Preparation of polysome profiles.* Polysome extracts were made according to Baim et al. (1985), but modified as follows. Cycloheximide was added to 200ml cultures (OD₆₀₀=0.7) of cells harboring either pJD139 or pRS426 to a final concentration of 100 µg/ml, and mixed with 100ml ice cold media containing 100 µg/ml cycloheximide. The cells were immediately collected by centrifugation, and washed twice with extract buffer (10mM Tris, pH 7.4, 100mM NaCl, 5mM MgCl₂, 100 µg/ml cycloheximide, 200_g/ml Heparin). The cell pellets were resuspended in an equal amount (w/v) of extract buffer. Approximately 0.25 ml of acid washed glass beads (Thomas Scientific Co.) were added and the cells were lysed by vortexing the suspensions eight times for 15 seconds, with a 30 second period of cooling on ice after each agitation. 1 ml of the extract buffer was added to the lysate and the lysate was centrifuged once at 5,000g for 5 minutes and then once at 10,000g for 10 min. 30 OD₂₆₀ units of the supernatant were applied to a 12 ml linear 7%-47% sucrose gradient containing 50 mM Tris-acetate (pH 7.4), 50 mM NH₄Cl, 12 mM MgCl₂, and 2mM 2-Mercaptoethanol. The sucrose gradient was centrifuged at 39,000 rpm for 150 minutes in a SW41 rotor (Backman), and polysome profiles were collected using a Density Gradient Fractionator, Model 640 (ISCO) and recorded by a VA-6 UV/VIS Detector (ISCO).

*Killer Assays, frameshifting assays and extraction and analysis of total nucleic acids.* Yeast strains harboring the L-A and M₁ viruses were transformed with the L3Δ fragment on either low or high copy plasmids, or with vector alone. The killer assay was carried out as previously described (See Example 1) by replica plating colonies onto 4.7MB plates newly seeded with a lawn of 5x47 killer indicator cells (0.5 ml of a suspension at 1 unit of optical density at 550 nm per ml per plate). After 2 days at 20°C, killer activity was observed as a clear zone around the killer colonies. To quantitate loss of killer activity, colonies that had been identified as killer⁺ were re-streaked for single colonies and the percentage of killer⁻ colonies were determined.

The efficiencies of -1 and + 1 ribosomal frameshifting were determined as previously described using 0-frame control, -1 and +1 ribosomal frameshift test plasmids. β-galactosidase assays using the L3Δ-lacZ fusion reporter used this method as well.

Total nucleic acids (TNA) were extracted from cells as previously described (Dinman and Wickner, 1992, 1994). Equal amounts of TNA were separated through 1.0% agarose gels, and visualized with ethidium bromide. TNA was denatured in the gels at 45°C for 30 min in 50% formamide, 9.25% formaldehyde, 1x TAE, the gels were washed with water and nucleic acids were transferred to nitrocellulose.

*Preparation of radioactive probes.* DNA probes were labeled to high specific activity with [α-³²P]dCTP by random hexonucleotide primer extention. A 0.6-kb *EcoRI-HindIII* fragment from the *CYH2* gene was used as a probe to monitor the abundance of the CYH2 precursor and CYH2 mRNA. A 1.2-kb *BglII-SalI* fragment from the *HIS4* gene was used as a probe to monitor the abundance of the his4-38 mRNA. A 3.1 kb *Sma I*/*Hind III* fragment from pTI25 was used as a probe to monitor the abundance of the lacZ mRNA.

L-A and M₁ (+) strand RNA probes were made as previously described (Dinman and Wickner, 1994) using [α-³²P]CTP labeled T3 RNA polymerase runoff transcripts from Eco RV digested pLM1 (to hybridize with L-A (-) strand) and Pst I digested p596 (M₁ (-) strand). Membranes were pre-hybridized for 5 hrs at 55°C in 50% formamide, 5X SSC, 50 mM NaHPO₄, pH 6.8, 0.1% SDS, 1 mM EDTA, 0.05% each of BSA, Ficoll, and polyvinylpyrrolidone, and hybridized with 10⁷ cpm of each probe in the same buffer at 55°C overnight. Membranes were washed in five changes of 0.1X SSC, 0.1% SDS at 65°C for 20 min and exposed for autoradiography.

### Results

Previous results have demonstrated that mutation or deletion of either the *UPF1, UPF2, UPF3* genes result in stabilization of nonsense-containing mRNAs. Combinations of mutations or deletions of the *UPF* genes do not result in further stabilization of nonsense-containing mRNAs, indicating that these proteins may function as complex. Consistent with this notion, recent results have demonstrated that Upf2p interacts with Upf1p and Upf3p. Two sets of results will be presented that monitor ribosomal programmed frameshifting and frameshift suppression that indicate mutation or deletion of the *UPF3* gene demonstrate unique phenotypes that are not observed in strains that harbor mutations or deletions of the *UPF2* or *UPF3* genes.

*Identification of an antisuppressor of his4-38 in upf2-1 and upf1-2, but not in upf3-1 mutant cells.* Deletion or mutation of the *UPF2* gene results in stabilization of nonsense-containing mRNAs, nonsense suppression and, in combination with Two phenotypes The wild-type *UPF2* gene was isolated by screening a strain harboring *upf2-1 his4-38 SUF1-1* strain with a genomic library, and identifying cells that could no longer grow at 37°C on medium lacking histidine. At that time we isolated another clone (YCpA9) which also prevented the growth of the *upf2-1* mutant strain at 37°C on medium lacking histidine. Since this did not encode *UPF2,* we tested the ability of this clone to act as an antisuppressor of *upf1-2* and *upf3-1* in strains harboring the *his4-38 SUF1-1* alleles. YCpA9 was able to act as an antisuppressor of *upf1-2* and of *upf2-1,* but not of *upf3-1.*

*The N-terminal 100 amino acids of the TCM1*/*MAK8 gene product are responsible for the upf1-1 and upf2-1 antisuppressor phenotype.* YCpA9 contains a 6.4kb yeast genomic DNA insert. Several Ycplac33 based low copy plasmid subclones of this were constructed and were transformed into the *upf2* strain PLY36 in order to localize the functional fragment. A 600nt fragment at one end was sufficient to abrogate the allosuppression phenotype of both *upf2-1* and *upf1-2.* Sequence analysis showed that this fragment contains the complete 5' untranslated region and the first 300nt (100 amino acids), *i.e.,* the N-terminal 1/4 of the *TCM1*/*MAK8* gene which encodes the 60S ribosomal subunit protein L3, and which is thought to be involved in the formation of the peptidyltransferase center. We named this fragment L3Δ. We also tested the effects of episomal expression of the complete *TCM1*/*MAK8* gene and found that this conferred the same growth phenotypes as strains transformed with vector alone, i.e. no antisuppression of *upf1-2* or of *upf2-1.* Thus, expression of the L3Δ fragment is responsible for the antisuppression of *upf1-2* and *upf2-1.*

*Episomal expression of the L3Δ fragment affects cellular growth rates.* We noted that cells transformed with the L3Δ bearing plasmids grew slower than control cells. To quantitate the affect of this clone on cell growth, the doubling times of cells containing low or high copy plasmids harboring the L3Δ fragment or vector alone were determined. Doubling times of wild-type cells expressing L3Δ on either high- or low-copy plasmids were approximately 2-fold greater than cells harboring vector alone (8 hrs. versus 4 hours at 30°). The growth rates of cells harboring the plasmid borne full length *TCM1*/*MAK8* gene had no effect on cellular growth rates (data not shown).

*Antisuppression of upf1-2 and upf2-1 by L3Δ is independent of the status of the nonsense-mediated mRNA decay pathway.* The different effects of the L3Δ fragment in the *upf1*⁻*, upf2*⁻*,* and *upf3*⁻ strains might be due to changes in either rates of degradation of the *his4-38* nonsense mRNA, or to effects at the translational level. In order to distinguish between these two possibilities, steady state his4-38 mRNA abundance in these and wild-type strains were determined by Northern blot analysis. Total RNAs were isolated, separated through a formaldehyde agarose gel, transferred to a nylon membrane, and the mRNA was probed using a *HIS4* fragment. As controls, we also probed for the *CYH2* precursor and *CYH2* mRNAs using a radiolabelled *CYH2* fragment. In all of the *upf* strains transformed with either the L3Δ fragment or a control vector, the *his4-38* mRNA abundance remained high. The *his4-38* mRNA level in wild-type strains (*upf* strains transformed with the *UPF*⁺ plasmid) were low. Thus the growth defect of the strain harboring an episomally expressed copy of the L3Δ fragment in *upf1*⁻ and *upf2*⁻ strains, but not in *upf3*^{*-*} strain, was not due to a decrease in the level of *his4-38* mRNA decay.

*The L3 fragment encoded by L3Δ decreases free ribosomal 60S subunits.* Since L3A has the complete 5' leader sequence and about 300 nucleotides of the N-terminal coding region, this genomic fragment should be transcribed and translated. In an attempt to determine whether this small fragment was expressed and whether it was assembled into ribosomes, a FLAG-epitope was inserted into the 3'-end of the L3Δ fragment. The epitope-tagged L3 fragment had the same phenotype as the L3 fragment as judged by the killer data (see below) and the anti-suppression of the *his4-38* in the *upf2-1* mutant strain (data not shown).

Polysome profiles were obtained from a wild-type strain transformed with the tagged L3Δ on a high copy plasmid (pJD 139), or a vector control (pRS426). Examination of the polysome profiles showed that episomal expression of the L3Δ fragment resulted in a decrease in the peak height of free 60S ribosomal subunits. Fractions were collected along the gradient and equal amounts protein from each fraction were separated by 10%-20% gradient SDS-PAGE. Although we attempted to monitor the amount of tagged L3 fragment by Western blot analysis, we could not detect it in any of the polysome fractions, nor in whole cell lysates. We do not know whether this is a consequence of poor translation of the fragment, inaccessibility of the FLAG epitope to the antibody, or post-translational degradation of the FLAG epitope. We also attempted to create an L3Δ/β-galactosidase fusion protein with the aim of assaying β-galactosidase throughout polysome gradients. Although sequence analysis confirmed that we had constructed the correct clones, we found these constructs to be unstably maintained in yeast cells (data not shown). Thus, we cannot definitively state that the L3Δ protein is actually expressed, although the multiple phenotypes that are observed in cells transformed with these plasmids are strong evidence of this. Further, our data do not allow us to determine whether the L3Δ fragment is p[hysically associated with ribosomes.

*Expression of the L3Δ fragment causes loss of killer activity and M*_{*1*} *dsRNA virus.* The M₁ satellite virus of L-A requires the products of at least 30 chromosomal *MAK* genes for its maintenance and replication, including *TCM1*/*MAK8.* Previous studies have shown that in most of the *mak*⁻ mutants, the level of 60S subunits were decreased compared with the wild-type cells. Expression of L3Δ also reduced 60S ribosome subunit levels in wild-type cells. To determine if episomal expression of the L3A fragment can recapitulate the Mak8⁻ phenotype, high and low copy plasmids harboring the L3Δ gene fragment were transformed into wild-type killer⁺ cells, and loss of killer phenotype was assayed by replica-plating the transformants onto killer indicator plates. While cells transformed with vector lost only a small fraction of killer activity, the cells transformed with either the original or with the FLAG-tagged L3Δ gene fragment had significant rates of loss of the killer phenotype. An assay for the loss of killer shows that cells that have lost killer activity have also lost the M₁ dsRNA. It is notable that the expression of L3Δ promoted loss of killer (and M₁) even when it was supported by the [B] isotype of L-A, which is capable of bypassing the requirement of cells for most of the *MAK* genes, including *MAK8.*

We also examined the effects of L3Δ expression with regard to killer loss in *upf* and *ski* mutants, and found that this also exacerbates the loss of killer in these contexts. Interestingly, *upf3* deletion mutants (*upf3Δ*) have intrinsically high rates of killer loss, which in the presence of L3Δ is reduced to nothing. The *ski* mutants normally have the opposite phenotype of *mak* mutants, *i.e.,* they increase the copy number of the L-A and M viruses, and *ski* mutants are generally dominant to *mak* mutants in that *mak*/*ski* double mutants are K⁻ (Toh-E and Wickner, 1980). This is particularly true of *ski*/*mak8* double mutants. However, when transformed with a high copy vector harboring the L3Δ fragment (pJD139), even the *ski* mutants displayed high rates of killer loss due to loss of M₁. Thus, expression of the L3Δ fragment is episatic to the *ski* mutants.

*Expression of L3Δ confers a Mof phenotype.* The ratio of available L-A encoded Gag/Gag-pol fusion proteins as determined by the efficiency of -1 ribosomal frameshifting is critical for maintenance of the M₁, and mutations which affect the efficiency of ribosomal frameshifting change the ratio of the Gag/Gag-pol proteins, promoting loss of M₁. To determine whether L3Δ expression affected this process, we measured the efficiency of ribosomal frameshifting in several wild-type strains harboring the L3Δ fragment L-A derived -1 or Ty1 derived +1 ribosomal frameshift reporters. Episomal expression of the L3Δ fragment increases the efficiency of -1, but not of programmed +1 ribosomal frameshifting in wild type cells. The 2.3 fold increase in -1 ribosomal frameshifting efficiency is at the upper limit of frameshifting efficiency tolerated for the minimal maintenance of M₁. The intrinsic efficiency of-1 ribosomal frameshifting is 5.3% in *mak8-2* mutants, i.e. 2.9 fold greater than in wild-type cells. Thus, the *mak8-2* mutation is also a *mof* mutant.

We also investigated the effects of expression of L3Δ in the *upf* mutants. A number of interesting pieces of information were found. First, in the *upf1Δ* and *upf2Δ* strains, the intrinsic efficiency of -1 ribosomal frameshifting appears to be approximately 2 fold greater than wild-type cells. This is due to the fact that the -1 frameshift reporter is a nonsense mRNA, which is stabilized in these cells allowing for greater overall production of the β-gal reporter protein as opposed to increased efficiencies of -1 ribosomal frameshifting. This is consistent with data presented above (Example 1). Further, expression of L3Δ increases the efficiency of -1 ribosomal frameshifting in these cells to the same extent as in wild-type cells, *i.e.,* approximately 3 fold. The apparent efficiency of -1 ribosomal frameshifting in *upf3Δ* cells is intrinsically high (7%) and the additional expression of L3Δ only increases this to 9.4%. This explains why *upf3*x cells show high intrinsic rates of killer loss: they are also *mof* mutants in that they increase the efficiency of -1 ribosomal frameshifting independent of their ability to stabilize nonsense mRNAs.

### Discussion

*The L3Δ fragment acts as an antisuppressor of the his4-38 frameshift allele of the in the upf1-2 and upf2-1 mutant cells.* We identified the L3Δ fragment as an anti-suppressor of *upf2-1* by the loss of the ability of *upf2-1 his4-38 SUF1-1* cells transformed with a plasmid containing this gene fragment to grow at 37°C in media lacking histidine. That a full length *TCM1*/*MAK8* clone did not have this activity suggests that expression of the N-terminal 100 amino acids of this protein is responsible for the observed phenotypes.

Antisuppression of *upf1-2* and *upf2-1* by L3Δ can result from decreasing the overall amount of functional His4 protein translated from the HIS4-38 mRNA at 37°C. This could be due to 1) abrogation of the ability of the *SUF1-1* encoded glycine tRNA to suppress frameshifting at 37°C, 2) the re-activation of the nonsense-mediated mRNA decay pathway at the non-permissive temperature, or 3) a translational defect which results in the inability of the *upf1-2* and *upf2-1* mutants to act as allosuppressors with *SUF1-1* glycine tRNA at 37°C. That expression of the L3Δ fragment in combination with the *upf3-1* mutation does not abrogate *SUF1-1* suppression at 37°C argues against a defect in *SUF1-1* encoded glycine tRNA function at this temperature. Further, since the His4-38 mRNA and the Cyh2 precursor mRNA abundances were equal in cells with or without the L3Δ gene fragment directly argues against re-activation of the nonsense-mediated mRNA decay pathway. Thus it is likely that the L3Δ fragment exerts its anti-suppressor activity at the level of translation by decreasing the translation of functional His4 product from the His4-38 mRNA.

*TCM1*/*MAK8* encodes ribosomal protein L3, which is involved in peptidyl- transferase center formation. Transcription of *TCM1* is under the control of a single upstream activation sequence recognized by a multi-functional transcription factor (Dorsman et al. 1989; Hamil et al. 1988), and the L3Δ fragment contains all of the upstream sequence required for its transcription. Moreland et al. (1985) examined the nuclear localization signal in the *TCM1* gene by making various deletions of the 3'-coding region fused in-frame with *lacZ* gene, and detected the location of the β-galactosidase by immunofluorescence microscopy. Their results suggested that expression of the first 21 amino acids of L3 was sufficient to direct the fusion protein into the nucleus. Thus it is quite possible that the L3 fragment produced by L3Δ is transported into the nucleus and subsequently into the nucleolus for assembly into the ribosome. Like other ribosomal proteins, the expression of the L3 protein is regulated, and the level of L3 protein remains the same even when the cells have extra copies of the *TCM1* gene (Pearson et al. 1982; Beus et al. 1994). The reduction of growth rates of cells expressing the L3Δ fragment, and the decrease is peak heights of 60S ribosomal subunits supports the notion that expression of this gene fragment affects translation. Two non-mutually exclusive models can be proposed to account for our observations. 1) That decreased levels of 60S subunits could be due to incorporation of the L3 protein fragment into ribosomes, directly affecting the process of peptide transfer, or alternatively, 2) that the observed effects could be due to indirect effects of the expression of this peptide fragment on ribosome biogenesis. If the L3Δ fragment is translated, but is not assembled into ribosomes, the peptide fragment may be downregulating the expression of wild-type *TCM1* gene. This would account for the reduction of 60S subunit peak heights in the polysome profiles from strains harboring the L3Δ fragment. Unfortunately, our data do not allow us to distinguish between these possibilities.

*The L3Δ fragment recapitulates a mak8 mutation.* That cells expressing the L3Δ fragment lost the M₁ dsRNA indicates that loss of killer activity was not due to defects in the translation, processing or export of the killer toxin. Further, since reduction in 60S ribosome subunit levels is a phenomenon characteristic of most of the *mak* mutants (Ohtake and Wickner 1995) the results presented here clearly that extrachromosomal expression of the L3Δ fragment confers a dominant negative Mak⁻ pehnotype on wild-type cells. The observation that expression of the L3Δ fragment results in a 2.3 fold increase in the efficiency of -1 ribosomal frameshifting is interesting in that this is just at the borderline where loss of M₁ is observed, and demonstrates that expression of this fragment confers a dominant Mof phenotype as well. Two other pieces of data support this notion. First, expression of this clone leads to the exclusion of M₁ from cells harboring the "Bypass" [B] isotype of L-A (strains JD88 and JD111 are L-AHNB M₁). Whereas most *mak* mutants, including *mak8,* can be bypassed by [B] (*i.e.,* L-AHNB can support M₁ and killer in these *mak* mutants), those *mof* mutants which cannot support killer lose M₁ regardless of the L-A isotype. That the efficiency of -1 ribosomal frameshifting is significantly elevated in *mak8-2* mutants suggests that mutations in ribosomal protein L3, and hence the peptidyl transferase center, can affect translational maintenance of reading frame as well.

An important finding is that expression of the L3Δ fragment resulted in an increase in the efficiency of L-A directed -1 ribosomal frameshifting, but did not affect Ty*1* promoted + 1 ribosomal frameshifting.

### EXAMPLE 5: mof Mutants Affect Peptidyl Transferase Center Activity

Epistasis experiments have shown that certain *mof* mutants affect peptidyl transferase center activity. Two antibiotics, trichodermin and anisomycin, both peptidyl transferase inhibitors, have been shown to be ineffective in *tcm1* mutants (which were first identified by their resistance to trichodermin). Neither of these drugs has any effect on -1 ribosomal frameshifting in *mak8-2* mutants, and their efficiencies of -1 ribosomal frameshifting are also elevated, making these cells *mof* mutants as well. Other *mof* mutants show some rather interesting trends. Like *mof8-2,* neither of these drugs affect -1 ribosomal frameshifting efficiencies in *mof1-1* and *mof2-1* mutants, suggesting that defects in the peptidyl-transfer reaction are either responsible for, or contributing factors towards, the Mof phenotypes of these cells. Interestingly, anisomycin does affect -1 frameshifting efficiencies in *mof6-1* cells, but sparsomycin has no such effect (Figures 16A. B). Conversely, sparsomycin changes the efficiency of -1 ribosomal frameshifting in *mof9-1* cells, whereas there is no effect with anisomycin with these cells (Figures 16A, B). These data suggest that the *mof6-1* and *mof9-1* mutations can be used as probes in the dissection of the two different sites of action of these drugs on the peptidyl transferase reaction.

### EXAMPLE 6: Anisomycin and Sparsomycin Suppress Nonsense Mutations

UPF1 + and UPF1- strains were treated with 5 ug/ml sparsomycin, anisomycin, and paromomycin, and tested for nonsense suppression. As shown in Figure 17, these drugs were able to suppress nonsense mutations.

## Claims

1. Use of a compound selected from anisomycin, sparsomycin or paromomycin in the manufacture of a medicament for the treatment of viral infections caused by viruses using -1 ribosomal frameshifting.

2. The use of claim 1, wherein the medicament is for treating an HIV infection.

3. A polypeptide corresponding to the N-terminal 100 amino acids of ribosomal binding protein L3.

4. A pharmaceutical composition for increasing the efficiency of programmed -1 ribosomal frameshifting, comprising the polypeptide of claim 3 and a pharmaceutically acceptable carrier.

5. The polypeptide of claim 3 or the pharmaceutical composition of claim 4 for treating a viral infection caused by a virus using -1 ribosomal frameshifting.

6. A nucleic acid encoding the polypeptide of claim 3.

7. An expression vector comprising a nucleic acid of claim 6 operatively associated with an expression control sequence.

8. The expression vector of claim 7 for use in modulating ribosomal frameshifting or for use in treating a viral infection.

## Patentansprüche

1. Verwendung einer Verbindung, die aus Anisomycin, Sparsomycin oder Paromomycin ausgewählt ist, bei der Herstellung eines Arzneimittels zur Behandlung von Virusinfektionen, die von Viren verursacht wurden, unter Anwendung von ribosomalem -1-Leserastersprung.

2. Verwendung nach Anspruch 1, wobei das Arzneimittel zum Behandeln einer HIV-Infektion ist.

3. Polypeptid, das den N-terminalen 100 Aminosäuren von ribosomalem Bindungsprotein L3 entspricht.

4. Pharmazeutische Zusammensetzung zum Steigern der Effizienz von programmiertem ribosomalem -1-Leserastersprung, die das Polypeptid nach Anspruch 3 und einen pharmazeutisch unbedenklichen Träger umfasst.

5. Polypeptid nach Anspruch 3 oder pharmazeutische Zusammensetzung nach Anspruch 4 zum Behandeln einer Virusinfektion, die von einem Virus verursacht wurde, unter Anwendung von ribosomalem -1-Leserastersprung.

6. Nukleinsäure, die das Polypeptid nach Anspruch 3 kodiert.

7. Expressionsvektor, der eine Nukleinsäure nach Anspruch 6 umfasst, die mit einer Expressionskontrollsequenz operativ verbunden ist.

8. Expressionsvektor nach Anspruch 7 zur Verwendung beim Modulieren von ribosomalem Leserastersprung oder zur Verwendung beim Behandeln einer Virusinfektion.

## Revendications

1. Utilisation d'un composé sélectionné parmi l'anisomycine, la sparsomycine ou la paromomycine dans la fabrication d'un médicament pour le traitement d'infections virales provoquées par des virus utilisant le déphasage ribosomique -1.

2. Utilisation selon la revendication 1, dans laquelle le médicament est pour le traitement d'une infection au VIH.

3. Polypeptide correspondant aux 100 acides aminés N-terminaux de la protéine de liaison ribosomique L3.

4. Composition pharmaceutique pour l'augmentation de l'efficacité du déphasage ribosomique -1 programmé, comprenant le polypeptide selon la revendication 3 et un support pharmaceutiquement acceptable.

5. Polypeptide selon la revendication 3 ou composition pharmaceutique selon la revendication 4 pour le traitement d'une infection virale provoquée par un virus utilisant le déphasage ribosomique -1.

6. Acide nucléique codant pour le polypeptide selon la revendication 3.

7. Vecteur d'expression comprenant un acide nucléique selon la revendication 6 associé de manière opérationnelle à une séquence de contrôle d'expression.

8. Vecteur d'expression selon la revendication 7 destiné à l'utilisation dans la modulation du déphasage ribosomique ou à l'utilisation dans le traitement d'une infection virale.
